Europäisches Patentamt

European Patent Office

Office européen des brevets

(11) Publication number: **0 109 150 B1**

## EUROPEAN PATENT SPECIFICATION

(45) Date of publication of patent specification: **12.08.92**  (51) Int. Cl.[5]: **C12N 15/69**

(21) Application number: **83305438.0**

(22) Date of filing: **16.09.83**

(54) **Plasmids with conditional uncontrolled replication behaviour.**

(30) Priority: **16.09.82 DK 4151/82**
**24.09.82 DK 4270/82**

(43) Date of publication of application:
**23.05.84 Bulletin 84/21**

(45) Publication of the grant of the patent:
**12.08.92 Bulletin 92/33**

(84) Designated Contracting States:
**AT BE CH DE FR GB IT LI LU NL SE**

(56) References cited:
**EP-A- 60 045**
**EP-A- 0 003 062**
**EP-A- 0 121 386**

**GENE, vol. 16, 1981, pages 275-286,
Elsevier/North-Holland Biomedical Press; J.J.
SNINSKY et al.: "Construction and character-
ization of a novel two-plasmid system for
accomplishing temperature-regulated, am-
plified expression of cloned adventitious
genes in Escherichia coli"**

(73) Proprietor: **BENZON PHARMA A/S
Helseholmen 1
DK-2650 Hvidovre(DK)**

(72) Inventor: **Molin, Soren
Mothsvej 70
DK-2840 Holte(DK)**
Inventor: **Light, Janice Ann
11 Gravett Close
Henley-on-Thames Oxon(GB)**
Inventor: **Larsen, Jens Erik Love
Odensevej 9 Jordlose
DK-5683 Harby(DK)**

(74) Representative: **Vingtoft, Knud Erik et al
Plougmann & Vingtoft Sankt Annae Plads 11
P.O. Box 3007
DK-1021 Copenhagen K(DK)**

Rank Xerox (UK) Business Services

CHEMICAL ABSTRACTS, vol. 96, no. 5, March 1982, page 133, no. 63648v, Columbus, Ohio, US; M. BITTNER et al.: "Versatile cloning vectors derived from the runaway-replication plasmid pKN402" & GENE, 1981, 15(4), 319-29

Stougaard et al. (1982) EMBO Journal, vol. 1, n 3, pages 323-328

Light, J. and Molin, S. (1982) Replication of prokaryotic DNA, EMBO Workshop "De Eem-hof", pages 137-139

Koepsel, R.R. and Saleem, A.K. (1987) Nucleic Acids Research, vol. 15, n 10, pages 4085-4097

## Description

The present invention relates to novel plasmids useful as cloning and production vectors in the field of recombinant DNA technology, the replication behaviour of which is uncontrolled under conditions favourable therefor, and to a method for preparing such plasmids.

TECHNICAL BACKGROUND

In published European Patent Application No. 82300865.1 are disclosed plasmids which are selected mutants in which an altered repressor gene leads to a high copy number replication. These plasmids are modified by inserting a transcriptional stop signal after an inserted heterogeneous gene so that the read-through expression of the heterologous DNA will not continue into the replication region.

Plasmids with conditional uncontrolled replication behaviour (so-called runaway plasmids) are known from, e.g., published European Patent Application No. 78101877.5. The replication of these plasmids is temperature-dependent in that the plasmids show a controlled, constant, low copy number when host bacteria carrying the plasmids are grown at one temperature, e.g. a temperature about 30°C, and an uncontrolled copy number when the host bacteria are grown at a different temperature, e.g. a temperature above about 36°C.

The plasmids disclosed in the above-mentioned patent application were isolated by chemical mutagenisation of plasmid R1, a wild-type plasmid which is known to replicate autonomously in *Escherichia coli*. The mutagenisation was performed in two stages, the first stage comprising the isolation of a plasmid-carrying bacterial clone in which the plasmid copy number was about 1-2 at 30°C and 4-5 times higher at 40°C, and the second stage comprising the isolation of a bacterial clone in which the plasmid showed an uncontrolled copy number at the higher temperature. The plasmids showing runaway behaviour were thus double mutants of the parent plasmid.

The above-mentioned patent application also discloses miniplasmids which are useful as cloning vectors because they are relatively easily transformed to host bacteria due to their small size. These miniplasmids have retained the genes responsible for the temperature-dependent runaway replication behaviour.

As cloning and production vectors the plasmids disclosed in the above-mentioned patent application may be used to obtain gene products of inserted fragments of foreign DNA. The temperature-dependent replication of the plasmids may be utilized to produce amplified amounts of gene products from DNA fragments of, for instance, eucaryotic origin. Such amplification of gene product has not been available with conventional plasmid DNA amplification techniques which work by, for instance, the addition of chloramphenicol to the culture medium, as the presence of chloramphenicol causes the protein, synthesis to stop.

The plasmids disclosed in the above-mentioned patent application may advantageously be used as cloning and production vectors in cases where an inserted foreign, gene codes for a product which is detrimental to the bacterium to which the plasmids are transformed as, at low temperatures, the plasmids have a low copy number and, consequently, little gene expression. This means that during the multiplication stage of the culture which is conducted at low temperatures the bacteria are not likely to be impaired.

DISCLOSURE OF THE INVENTION

The present invention relates to novel plasmids which carry at least one regulatable promoter inserted in such a way that transcription therefrom regulates plasmid replication In particular, the invention relates to plasmids in which the regulatable promoter causes substantially increased replication when host microorganisms containing the plasmids are cultivated under conditions securing increased transcription from the promoter. The invention further relates to to a method for preparing such plasmids. The plasmids are useful as cloning and production vectors.

It is known to insert a regulatable promoter in order to control the expression of a structural gene inserted in the plasmid and coding for a desired gene product. It is, however, believed to be novel to insert a regulatable promoter in plasmids by means of which it is possible to regulate the number of plasmid copies formed.

A first aspect of the invention relates to a plasmid useful as a cloning vector having a size in the range of about 2.5-15.0 x $10^6$ daltons which plasmid carries:

(a) an inserted regulatable promoter at a position and in an orientation permitting transcription from said promoter into the replication region;

(b) said replication region comprising a gene coding for a gene product that is positively needed for

3

replication, increased transcription from the regulatable promoter leading to an increased level of the gene product which is positively needed for replication;

(c) said increased level resulting in a plasmid replication rate which is higher than the host cell growth rate leading to an increased plasmid copy number which, usually after 4-5 cell doublings, will have increased by a factor of 25-1000."

In the present specification and claims the term "regulatable promoter" refers to a promoter the activity of which, with respect to the rate of initiation of transcription, may be regulated by means of adjusting the conditions under which host microorganisms containing the promoter-carrying plasmids are grown. Such a regulatable promoter may, for instance, be a foreign promoter, i.e. a promotor not naturally related to the plasmid in which it is inserted. The promoter may be inherently regulatable due to a particular DNA structure within the region of the promoter. An example of such a promoter is the one found in the known runaway plasmids (cf. the description below). Alternatively, the promoter may be controllable by means of a regulating factor which may either exert positive control, i.e. the plasmid does not attain uncontrolled replication unless the promoter is positively induced, e.g. by adding an inducing substance to the medium in which the host cells are grown, or the regulating factor may exert negative control. The latter type of controlling factor is also known as a repressor the activity of which may also be regulatable by means of adjusting the growth conditions of the host cells. The repressor gene may be located on the plasmid itself together with the promoter, on a separate plasmid in the same host microorganism or on the chromosome of the host microorganism. The repressor gene codes for a product which inhibits transcription from the inserted promoter so that replication is kept at a low, constant level. When, for some reason, the repressor becomes inactivated, no such control exists, and transcription increases.

Unless otherwise specified, the term "promoter", as used in the present specification and claims, is usually meant to include both a promoter whose activity inherently responds to changes in the growth conditions of the host cells and a promoter which is controlled by a regulating factor. The expression "a plasmid which carries on inserted regulatable promoter" is also intended to apply to plasmids of the invention in which the promoter and the replicon regulated by the promoter have been inserted on the same DNA fragment, the promoter and the replicon being ultimately derived from different sources; this means that at one stage during the construction of the desired final plasmid, the promoter has been inserted into the plasmid from which the replicon is derived. The term "increased plasmid copy number" is understood to refer to the fact that when the conditions for growing the host microorganisms are altered to secure increased transcription from the promoter so that the plasmid loses its replication control, the plasmid copy number increases exponentially with a generation time of about 40-50%, or less, of that of the microbial population. The increase continues until the host cell ceases to grow, usually after 4-5 cell doublings, or less, at which point the content of plasmid DNA in the cell is about 40-70% of the total amount of DNA, i.e. the plasmid copy number increases by a factor of about 25-1000 or even more. In other words, the plasmid copy number does not reach a steady state. This type of replication behaviour is also termed "runaway" behaviour.

The inventors' recently acquired knowledge of the replication control principle of R1-type plasmids is utilized in the construction of plasmids according to the invention (for a further explanation of this principle, see DETAILED DESCRIPTION OF THE INVENTION). When, in accordance with the principle of the invention, a regulatable promoter is inserted in the plasmid, increased transcription from the promoter leading to an increased plasmid copy number may be caused by, e.g. derepression of the promoter. This means that a repressor gene inserted together with the promoter and coding for a gene product which controls the promoter under certain conditions, becomes inactivated under different conditions so that transcription from the promoter is no longer inhibited, ultimately resulting in an uncontrolled plasmid copy number.

This is an important improvement compared to the known runaway plasmids which may, under varying conditions, exhibit differences in their replication properties. There are no indications that this is the case with the plasmids of the invention as the foreign promoter inserted will normally be one which is so strong (i.e. the frequency of initiation of transcription is high) that such changing conditions are without effect.

One of the conditions of cultivation by means of which it is possible to regulate the activity of the regulatable promoter is the temperature at which the host microorganisms are grown. The activity of the promoter may be temperature-dependent in itself, i.e. reacting to temperature changes during cultivation, or the regulating factor may be temperature-sensitive, e.g. a temperature-sensitive repressor.

The regulatable promoter may be a promoter from the bacteriophage $\lambda$ which may be inserted as part of a DNA fragment which additionally carries the gene for a temperature-sensitive $\lambda$ cl repressor controlling transcription from the promoter. The $\lambda$ promoter may either be $\lambda P_R$ or $\lambda P_L$, especially $\lambda P_R$. However, other promoter systems inserted in the plasmids in accordance with the principles of the invention and influenced by other external factors than temperature may also be employed, such as promoters which are inducible with chemicals or regulatable by means of metabolites, such as *lac*, *trp* and *deo* promoters.

A favoured embodiment of the plasmid according to the invention is one in which the promoter regulatory system results in temperature-dependent replication behaviour of the plasmid. In particular, the temperature at which the plasmid shows a substantially increased plasmid copy number is a temperature higher than the temperature at which the copy number is low. Plasmids according to the invention carrying a regulatable promoter the transcription pattern of which is temperature-dependent, for instance due to the presence of a temperature-sensitive repressor, show a constant, low copy number at one temperature, such as a temperature of about 30°C, because at this temperature the promoter is repressed and transcription from it is consequently kept at a low level, and a substantially increased copy number at a higher temperature, such as a temperature in the range of about 36-42°C, as the regulatory system becomes inactivated in this temperature range causing derepression of the promoter and, consequently, increased transcription from the promoter. It is preferred that runaway replication occurs at temperatures above about 39°C.

In the following description, reference will be made only to plasmids the replication of which is controlled by a regulatory system which is temperature-sensitive, especially by a temperature-sensitive repressor, although it will be understood that other types of conditions by means of which plasmid replication may be regulated may also be utilized in an analogous manner, as explained above.

One type of plasmid according to the invention is a plasmid in which a regulatable promoter has been inserted in place of part of one native replication regulatory gene of the plasmid. For the purposes of the present specification and claims, such a plasmid is designated a type A plasmid. When the plasmid is a derivative of plasmid R1, the regulator gene deleted from the plasmid is the *copB* gene, coding for one of the native replication inhibitors of the plasmid. The deletion of the *copB* gene results in a slight, stable increase in plasmid copy number. Thus, the plasmid has a copy number of about 20-40, preferably 20-30 and particularly 20-25 copies per cell at about 30°C. However, when the temperature is raised to about 40°C the repressor function of the regulatable promoter is inactivated and replication becomes uncontrolled resulting in a plasmid copy number in the range of at least about 500-1000 copies per cell, dependent, inter alia, on the size of any foreign DNA fragment inserted in the plasmid (the larger the DNA fragment, the lower the number of plasmid copies formed), the effect of gene products of such foreign DNA and/or the microbial strain to which the plasmid is transformed. In some cases, however, the plasmid may form up to several thousand copies per cell at the higher temperature. At any rate, the content of plasmid DNA at the high temperature relative to the total DNA content is about 40-75%, usually 50-60%.

Another type of plasmid according to the invention is a plasmid with a copy number of about 3-5 copies per cell at one temperature, such as a temperature of about 30°C, and an uncontrolled copy number in the range of about at least 500-1000 copies per cell, dependent on the factors outlined above, and in some cases up to several thousand copies per cell, at a higher temperature, such as a temperature of about 42°C. Such a plasmid may be obtained by inserting the regulatable promoter upstream of the native replication control gene(s) of the plasmid. For the purposes of the present specification and claims, such a plasmid is designated a type B plasmid. In the case of plasmid R1 derivatives, the regulatable promoter has been inserted upstream of both the *copB* and *copA* genes, so that both these native replication inhibitors have been retained which causes the plasmids to show the above-mentioned copy number pattern. However, if the plasmid is one that lacks the *par* region (the presence on the plasmid of the region responsible for partitioning has the effect that the plasmid is stably inherited; this effect is ascribable to one or more genes in the *par* region), the plasmid will be lost with a frequency of about 1% per generation at the low temperature because of the low copy number at the low temperature. Consequently, it may be advantageous to insert the *par* region into such a plasmid to ensure that the runaway replication of the plasmid will subsequently take place in all cells of the population.

The type B plasmid has the added advantage of a large copy number range. As the copy number at the low temperature is very low it is possible to insert foreign genes into the plasmids the products of which are partially toxic (lowering the growth rate) or even lethal to the microorganism to which the plasmid has been transformed. This is sometimes the case with products from eucaryotic genes, which are usually those of the highest interest to, e.g., the medical industry. Because of the low replication rate at the low temperature foreign genes are either not expressed at all or only in such small amounts that the cells are not damaged thereby and may be grown at the low temperature under normal conditions. This greatly enhances or facilitates the process of producing certain polypeptides which has hitherto not been possible at all, or only with great difficulty.

A third type of plasmid according to the invention is a plasmid with a copy number in the range of about 0.5-1 copy per cell (the figure 0.5 is understood to mean that the frequency of replication is less than one per cell cycle) at one temperature, such as a temperature about 30°C, and an increased copy number in the range of about at least 500-1000 copies per cell, dependent on the factors outlined above, and in some cases up to several thousand copies per cell, at a higher temperature, such as a temperature of about 42°C. Such a plasmid may be a plasmid carrying the regulatable promoter in the replication region from which part of one native replication regulatory gene has been deleted which has then been inserted outside the replication region, i.e. at a site where it is not naturally located. For the purpose of the present specification and claims, this plasmid is designated a type C plasmid. In the case of plasmid R1 derivatives from which the *copB* gene has been deleted, the *copB* gene is reinserted, not at its original site, but at the *Eco*RI site outside the replication region. Even so, the *copB* inhibitor protein influences the replication of the plasmid but in such a way that the plasmid shows the above-mentioned copy number pattern. However, if the plasmid is one that lacks the *par* region, the plasmid is lost with a frequency of about 5% per generation at the low temperature because of this extremely low copy number at the low temperature. Consequently, it would be even more advantageous with this plasmid than is the case with the type B plasmids to insert the *par* region into the plasmid to ensure that the runaway replication of the plasmid will subsequently take place in all cells of the population. The replication behaviour at the low and high temperatures, respectively, and hence the advantages of using this plasmid, is otherwise similar to that of the type B plasmids.

A second aspect of the invention pertains to a plasmid in which, in addition to the first regulatable promoter regulating transcription from the replication region, a second promoter has been inserted in a manner permitting the insertion of a structural gene the expression of which is controlled from the second promoter. This second promoter may be controllable, i.e. it may be possible to regulate its function by adjusting the conditions under which the host cell is grown by means similar to those of regulating the first regulatable promoter. A number of promoters are at present contemplated to be useful for this purpose, such as a *lac* promoter, *trp* promoter, *deo* promoter, *recA* promoter, $\lambda P_L$ promoter, etc. The $\lambda P_L$ promoter may prove to be especially advantageous as a second promoter controlling the expression of the structural gene as it is also regulated by the temperature-sensitive cI repressor, and therefore, amplification conditions are the same so that the cultivation of the host cells is simplified, and product amplification occurs simultaneously with the amplification of plasmid DNA. It should be noted that the second promoter may also be identical to the first regulatable promoter.

The second promoter may be inserted into type A, type B and type C plasmids. Such a plasmid has a constant, low copy number and very little or no gene expression at about 30°C, and a substantially increased plasmid copy number, and gene expression, at a temperature in the range of about 36-42°C. Preferably, the plasmid has an increased plasmid copy number and a very high gene expression at temperatures above 39°C.

The plasmids of the invention may be used as cloning and production vectors, i.e. plasmids which may be used in the field of recombinant DNA technology for the purpose of obtaining a wide variety of products for technical or medical purposes, particularly polypeptides and proteins or fragments thereof, enzymes and the non-proteinaceous products of reactions of enzymes with a compound in the nutrient medium, low molecular weight products such as hormones, and nucleic acids; products of eucaryotic, especially mammalian, genes are of particular interest. To be useful as a cloning and production vector it is advantageous, though not essential, for the plasmid to have, for at least one restriction endonuclease, a unique site cleavable by this endonuclease. The site should be one which, upon insertion of a fragment of foreign DNA, permits the resulting recombinant plasmid to replicate autonomously and which permits the regulatable promoter inserted in the plasmid to regulate the transcription of the replication region leading, when transcription from this promoter is increased, to a substantially increased plasmid copy number. Restriction sites located within the replication region are thus not permissible as cloning sites, as this would cause the plasmid to lose its replication properties.

The plasmid of the invention is constructed by a method which comprises inserting a regulatable promoter into a plasmid in such a way that transcription from this promoter regulates replication and identifying the plasmid so treated by screening for those plasmids which show a substantially increased plasmid copy number when transcription from the promoter is increased. When the regulatable promoter is $\lambda P_R$, it may be inserted by mixing plasmid and phage DNA, restricting with an appropriate endonuclease, heat inactivating and ligating the mixture, and transforming the ligation mixture to a microorganism.

The construction of such plasmids may be performed by inserting the promoter at random in different sites on the plasmid. By screening for plasmids with uncontrolled replication behaviour under conditions securing increased transcription from the regulatable promoter, the correct insertion is identified. A simple screening for such plasmids comprises analysing the growth properties of the plasmid-carrying cells at the various conditions. The amount of plasmid DNA in the host cells may be measured in different ways, notably by means of agarose gel electrophoresis in a manner known *per se*. This screening method is easily and speedily performed.

In a specific embodiment of the invention involving the construction of type A plasmids, the construction method comprises deleting part of one native regulatory gene and its promoter, in the case of the *copB* gene from plasmid R1 by cleaving with the restriction endonuclease *Bgl*II, and inserting a DNA fragment, such as a *Bgl*II fragment, including the regulatable promoter at this site.

In another embodiment of the invention involving the construction of type B plasmids, the construction method comprises inserting the regulatable promoter into the plasmid upstream of the native replication control system, in the case of plasmid R1 derivatives at the *Bgl*II site upstream of the *copB* gene by means of partial restriction with *Bgl*II, ligation and transformation to the microorganism in question, such as *E. coli*. Alternatively, the plasmid may be constructed by using the type A plasmid with the partially deleted native replication regulatory gene as a starting material and reinserting the native regulatory gene at its original site, in the case of R1-type plasmids by reinserting the *copB* gene at the *Bgl*II site.

A third embodiment of the invention, involving the construction of type C plasmids, comprises, by using the type A plasmid as a starting material, inserting one native regulatory gene in a region of the plasmid where it is not located in the wild-type parent, in the case of R1-type plasmids by inserting the *copB* gene as an *Eco*RI fragment at the *Eco*RI site outside the replication region of the plasmid by means of restriction with *Eco*RI, ligation and transformation.

A fourth embodiment of the invention comprises inserting, in addition to inserting the regulatable promoter, a second promoter at an appropriate restriction site permitting the insertion of a structural gene the expression of which is regulatable from the second promoter. As mentioned above, this site must not be located within the replication region of the plasmid.

The plasmids according to the invention are miniplasmids, i.e. they are appreciably smaller than their wild-type parent plasmids. Generally, they have a size in the range of about $2.5\text{-}15.0 \times 10^6$ daltons, often $2.5\text{-}10.0 \times 10^6$ daltons, usually $4.0\text{-}12.5 \times 10^6$ daltons, especially $4.0\text{-}5.0 \times 10^6$ daltons.

The uncontrolled replication behaviour of the known runaway plasmids appears to depend on the specific bacterial strain to which it is transformed, the culture medium in which the bacteria are grown and the DNA inserted. This dependence is probably due to the fact that the runaway replication behaviour of the known plasmids is, in part, caused by the substitution of a single nucleotide in the *copB* promoter causing a slight increase in the transcription rate which may be affected by environmental changes as regards the bacterial species and strain involved and/or the culture medium as well as by foreign DNA inserted in the plasmids. The plasmids according to the invention do not seem to be similarly limited as the strong foreign promoter when activated/derepressed increases the transcription rate to such an extent that such changes are without significance. Consequently, these plasmids may be transformed to a wide variety of strains and they are also more reliable to handle than the known runaway plasmids.

The present invention also extends to plasmids derived from other wild-type plasmids than R1 and/or found in other plasmid-harbouring microorganisms than *E. coli*. It may also be envisaged to construct a plasmid of the above-described type which can be maintained in microorganisms not naturally harbouring plasmids. The replication system of several different microbial plasmids has been shown to be similar in many ways. For instance, all known plasmids require transcription if replication is to take place. By utilizing the principle of the invention it is possible to convey the uncontrolled replication phenotype to other plasmids so that plasmids already in use as cloning vectors and known to function well in a desired microorganism may acquire runaway behaviour. Thus, by inserting a regulatable promoter, e.g. $\lambda P_R$, at a proper position in such plasmids in such a way that transcription from this promoter regulates replication, uncontrolled replication will occur under conditions securing increased transcription from the promoter.

DETAILED DESCRIPTION OF THE INVENTION

*The Replication Control System of R1-type Plasmids*

The Basic Replicon

Until recently, the replication control system of R1-type plasmids was not known. It has now been found that the genetic information needed for plasmid R1 replication and control of replication is carried in a 2500 base pairs long region consisting of three *Pst*I fragments located within the resistance transfer factor (Molin et al., J. Bact. *138*, 1979, 70-79).

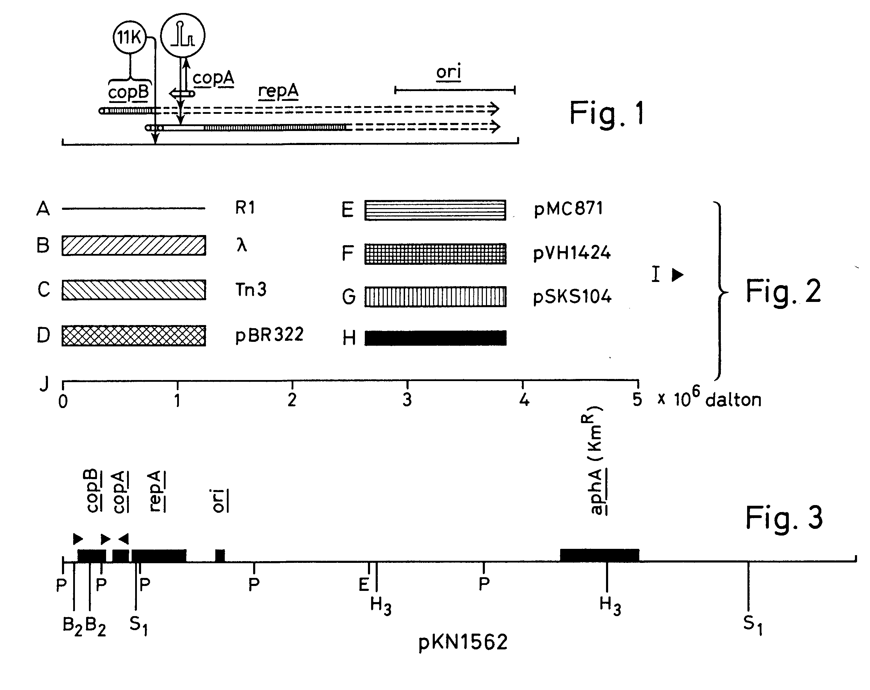

This region is defined as the *basic replicon* and contains four important elements, three genes (*copA*, *copB* and *repA*) and the replication origin (Molin et al., Microbiology, 1981, 408-11). A physical, genetic and functional map of the basic replicon is shown in Fig. 1. The gene *repA* codes for a function that is positively needed for replication. The gene product (according to the DNA sequence) is a protein of 278 amino acids and a molecular weight of about 33.000 daltons (Rosen et al., Mol. Gen. Genet. *179*, 1980, 527-37). This protein has not been unambiguously identified, but the capacity to form protein from the *repA* gene has been clearly demonstrated by the use of translational gene fusions with the *lacZ* gene (Light & Molin, Mol. Gen. Genet. *184*, 1981, 56-61). The biochemical function of the RepA protein has not yet been fully explored, but the RepA protein is believed to be a replication initiation factor.

The gene *copA* codes for a small (80-90 nucleotides long) unstable RNA molecule with a high degree of secondary structure (i.e. it forms hairpin loops) (Stougaard et al., Proc. Natl. Acad. Sci. USA 78, 1981, 6008-6012). The CopA-RNA is an inhibitor of replication; mutations affecting the nucleotide sequence of the CopA-RNA may lead to an increased copy number (*cop* mutants). DNA-sequence analysis of *copA* mutants shows that all mutations are strikingly clustered in a loop area within 5 bases. These mutations result in a drastically reduced or total loss of inhibitor activity against the wild-type plasmid. Therefore, it may be concluded that the specificity of the inhibitor resides in the loop. It is also striking that in all *copA* mutants whose nucleotide sequence is known, a cytosine in the CopA-RNA has been replaced by a uracil (or, in one case, by an adenine). This strongly suggests that the CopA-RNA acts by nucleic acid/nucleic acid interaction. This conclusion is further strengthened by the fact that even those *copA* mutants that have lost all CopA activity against the wild-type plasmid retain some CopA activity against themselves. Moreover, the wild-type CopA-RNA most often has reduced inhibitory effect against *copA* mutant plasmids. Hence, a *copA* mutant is not only changed in the inhibitor, but also in the target, *copT*; a single base substitution thus leads to what phenotypically is a double mutant.

The *copB* gene codes for a basic protein of 86 amino acids with a molecular weight of 11.000 daltons. This protein is formed in substantial amounts. Deletion of the *copB* gene with its promoter leads to an eightfold increase in copy number; therefore, it is concluded that the *copB* protein acts as a replication inhibitor, i.e. as a negatively acting control element in the regulation of replication of plasmid R1 (Molin et al., Mol. Gen. Genet. *181*, 1981, 123-30).

A double mutant (*copAcopB*) is unconditionally lethal to the host microorganism due to so-called runaway replication. Hence, the two control gene products act synergistically.

Transcriptional Activity in the Basic Replicon

There are three transcription initiation sites within the basic replicon, the *copB* promoter, the *repA* promoter (Light & Molin, Mol. Gen. Genet. *184*, 1981, 56-61) and the promoter for the *copA* gene (Stougaard et al., op. cit.). The latter transcribes away from the origin region, whereas the two former transcribe towards the origin region. Consequently, both strands are transcribed in the *copA* region. The CopB transcript continues past the *copB* gene, and this transcript and the transcript that starts at the *repA* promoter both transcribe the *repA* gene.

Deletions of the *copB* gene constructed by restriction with *BgI* result in 8-10 fold increased copy number of plasmid R1. This is surprising since the *copB* gene promoter and the proximal part of the *copB* structural gene are deleted resulting in 1) loss of *copB* activity and derepression of the *repA* promoter, and 2) loss of the contribution of the *copB* promoter to total *repA* expression so that the derepressed *repA* promoter effectively takes over total *repA* transcription from the *copB* promoter. The total effect should only be a twofold increase in the rate of transcription of the *repA* gene as the *repA* promoter has a strength that is two-fold increased above that of the *copB* promoter.

This apparent paradox is explained by the activity of the *copA* gene. The CopA-RNA is terminated in a normal termination structure with a stem ending in a series of U's. CopA-RNA control of the formation of RepA protein depends on such proper termination. If transcription of the opposite strand is strong (convergent transcription), the efficiency of termination is decreased. Increased transcription from the *repA* promoter results in an extension of the CopA transcript to about 150-200 nucleotides. The extended CopA transcript does not function as a replication inhibitor, i.e., it is substantially inactivated, leading to an increased amount of RepA protein and eventually to an increased plasmid copy number (Stougaard et al., the EMBO Journal *1*, 1982, 323-328).

Control of Replication

The products from two genes, *copA* and *copB*, negatively control replication of plasmid R1, i.e. they function as replication inhibitors. Through construction and analysis of *repA-lac* fusions it could be demonstrated that the two *cop* gene products act by negatively controlling the expression of the *repA* gene (Light & Molin, Mol. Gen. Genet. *184*, 1981, 56-61). Therefore, plasmid R1 replication seems to be controlled by the regulation of the formation of a protein which is positively needed for, presumably, the initiation of replication of the plasmid.

By means of *lac* gene fusions it was also demonstrated that *copA* and *copB* products have separate targets. The target for the *CopB* protein is the *repA* promoter, where it acts by inhibiting initiation of transcription. However, the CopB protein is unable to interfere with transcription initiated upstream of the *repA* promoter. The CopA-RNA does not affect transcription but acts at a post-transcriptional level, i.e. by inhibiting translation of the RepA-mRNA.

## Replication of the Runaway Mutants

The replication system of the plasmids disclosed in published European Patent Application No. 78.101877.5 is substantially the same as described above with respect to the wild-type R1 plasmids. Recent research by the present inventors has now partially established the cause for the existence of temperature-dependent plasmid mutants with runaway replication behaviour. As explained above, increased transcription towards the *repA* gene may lead to inactivation of the CopA-RNA molecule. In the runaway mutant plasmids disclosed in the above-mentioned patent application, the *copB* promoter is mutated by the substitution of a single nucleotide in a sequence, as shown:

a) Wild-type plasmid: 5'-......TTCTCAAGTCGCT...-3'
b) Runaway mutant: 5'-......TTCTCAAGT*T*GCT...-3'

The italics denote the nucleotide substitution. The region depicted is the RNA polymerase binding site.

This mutation was shown to cause increased transcription from the *copB* promoter, in particular at higher temperatures.

## Replication of Plasmids Carrying a Regulatable Promoter

In the construction of the plasmids of the present invention, the inventors' recently acquired knowledge of the replication system of the wild-type R1 plasmids has been utilized. The replication of the plasmids of the present invention is not, however, made to rely on mutations in the plasmid as is the case with the runaway plasmids disclosed in published European Patent Application No. 78.101877.5. Instead, a foreign promoter has been inserted so that transcription of the replication region is at least partly controlled from this promoter. The promoter may be regulatable by adjustments in the conditions under which the host microorganisms are grown, and the regulation of promoter activity may be effected by various means, such as by altering the cultivation temperature or the composition of the growth medium.

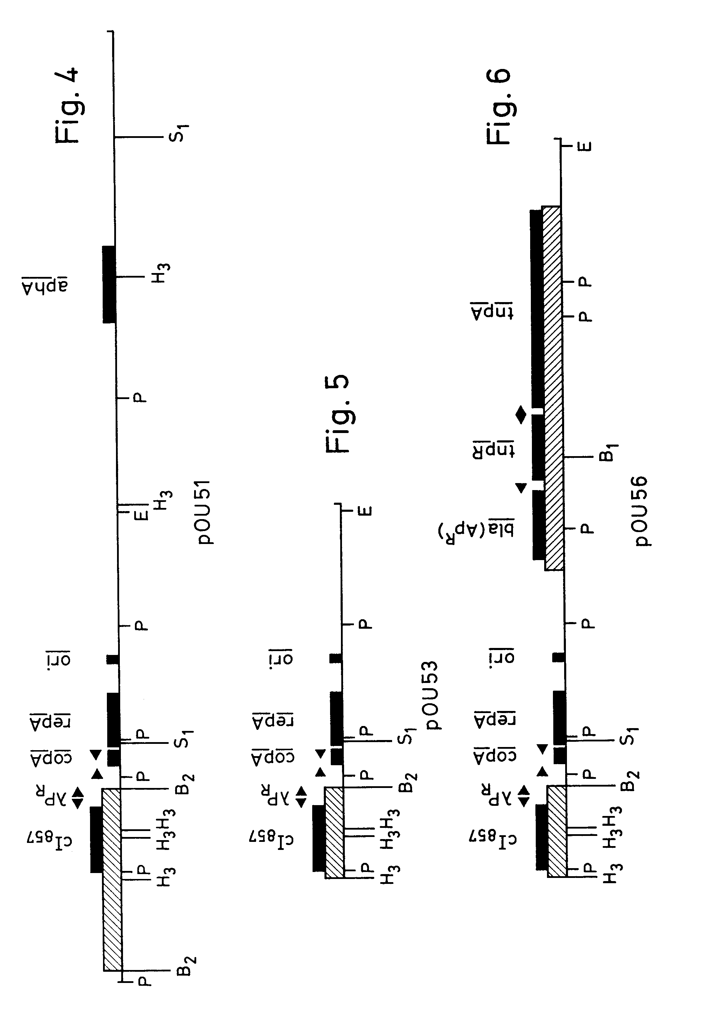

When the regulatable promoter is one selected from a group of temperature-dependent promoters, it is preferably a promoter derived from bacteriophage λ, such as λ$P_R$ or λ$P_L$, in particular λ$P_R$. In phage λ, the $P_R$ promoter is controlled by the cI repressor which, in accordance with the principles of the invention, is derived from phage λ together with λ$P_R$ and inserted in a plasmid in the form of a *Bgl*II fragment (*vide* the appended plasmid maps, Figs. 4-11). The wild-type cI repressor is not in itself temperature-sensitive, so in order to make the replication system regulatable by means of adjustments in the cultivation temperature, the specific cI repressor employed (cf. the following examples) is temperature-sensitive, coded by the mutant allele cI$_{857}$ (Sussman & Jacob, Compt. Rend. Acad. Sci. *254*, 1962, 1517). The repressor is active, i.e. it represses the λ$P_R$ promoter, at low temperatures such as a temperature of about 30°C, whereas, at higher temperatures such as temperatures above about 39°C, it is denatured causing an inactivation of the repressor function and a derepression of λ$P_R$. As the inserted promoter is very strong, this results in drastically increased transcription towards *repA* which, in turn, leads to increased plasmid replication.

Alternatively, the regulation of the promoter may be effected by chemical means, i.e. by adjusting the composition of the medium in which the host microorganisms are grown, either by adding a chemical inducing the promoter, for instance by causing inactivation of the repressor controlling promoter activity and consequently derepression of the promoter or by securing the formation of a promoter-inducing metabolite leading to activation of the promoter. A specific example of a chemically inducible promoter is the *deo* promoter from *E. coli* which is subject to negative control by two chromosomal repressor proteins, the products of the *deoR* and *cytR* genes. The *deo* promoter may be derepressed by adding cytidine to the growth medium, as cytidine acts by affinity to the repressor causing a conformational change thereof so that it ceases to function as a repressor. Additionally, positive control may be exerted on the expression from the *deo* promoter through the effect of catabolite repression. Activation of the *crp* (catabolite repressor protein) protein by cyclic AMP (cAMP) results in stimulation of the *deo* promoter activity; high cellular levels of cAMP are known to be correlated with low levels of glucose in, or the absence of glucose from, the growth medium, which may be obtained by adding only a limited amount of glucose to the medium, e.g. an amount of about 0.01%, together with another carbon source such as glycerol or succinate. When the glucose is consumed by the cell, the resulting increase in the intracellular level of cAMP will activate the *deo* promoter leading to an increased replication rate of the plasmid carrying the promoter. Thus, if the *deo* promoter is inserted upstream of the *copB* gene in a plasmid R1 derivative, induction of the promoter leads to increased replication. The increased replication behaviour may be reversed by adding glucose to the growth medium so that the copy number of the plasmid slowly decreases to pre-induction level. Another example of a chemically regulatable promoter is the *lac* promoter which is induced by the presence of lactose in the nutrient medium leading to increased replication. In this case, too, the process may be reversed by dosing the amount of lactose added to the medium so that it is eventually consumed by the cells.

Insertion in the proper position and orientation of a DNA fragment carrying a regulatable promoter in place of part of one native replication regulatory gene, e.g. in place of the the *Bgl*II fragment covering part of the *copB* gene of plasmid R1, results in a plasmid derivative the replication rate of which is at least in part controlled by the inserted promoter (a type A plasmid).

Insertion in the proper orientation of a DNA fragment carrying a regulatable promoter immediately upstream of the native replication regulatory gene, in the case of plasmid R1 derivatives the *copB* gene, results in a plasmid with slightly different replication properties (a type B plasmid).

As mentioned above, type B plasmids which lack the *par* region are lost with a frequency of about 1% per generation. Without selection for the presence of the plasmids in a culture, the plasmid is eventually lost from cells grown at about 30°C. In order to stabilise the plasmid, it is possible to insert a DNA fragment carrying a *par* region from, for instance, a wild-type R1 plasmid in a type B plasmid which leads to complete stability (no loss) of the plasmid from cells grown under similar conditions. Plasmid stability may be determined by initially growing cells containing type B plasmids in which *lac* genes as well as the *par* region has been inserted and, as a control, cells containing type B plasmids carrying the *lac* genes, but not the *par* region, selectively on plates containing an antibiotic (to which the plasmids mediate resistance) to ensure the presence of both kinds of plasmid in the starting colonies. Samples of both colonies are then streaked on plates not containing any antibiotic so that the cells are left to grow without selection for a number of generations (cell doublings) such as, for instance, 25 generations. Finally, cells from both the resulting colonies are streaked onto McConkey lactose indicator plates where the cells originally containing plasmids carrying the *par* region form red colonies which shows that the plasmids have been retained, whereas the control cells originally containing plasmids without the *par* region form both red and colourless colonies which means that during the selection-free period the plasmids have been lost from some of the cells. Thus, it is demonstrated that plasmids in which the *par* region has been inserted have become completely stable (cf. Example 11). This stabilising effect is especially important if an inserted DNA fragment causes a reduction of the growth rate of the plasmid-carrying cells compared to plasmid-free cells.

Finally, insertion of a DNA fragment carrying the native replication regulatory gene, such as the *copB* gene of plasmid R1, into a site outside the replication region of a type A plasmid results in a plasmid with particular replication properties (a type C plasmid).

The desirability of plasmid stability described above with respect to type B plasmids is even more pronounced where type C plasmids are concerned, since these, as mentioned above, have an even lower copy number at the low temperature and are therefore lost with a higher frequency than type B plasmids if they lack the *par* region. The type C plasmids may be similarly stabilised by inserting the *par* region in the plasmids in the case of plasmids where it is not already present.

According to a particular aspect of the invention, the plasmid carries, in addition to the first regulatable promoter, a second promoter located outside the replication region which promoter regulates the expression of an inserted foreign structural gene which, for instance, codes for a desired polypeptide This additional promoter may, for instance, be the cI promoter located on the fragment additionally carrying the $\lambda P_R$ promoter and the cI gene, which cI promoter is controlled by the cI gene product and transcribes in the opposite direction of $\lambda P_R$. Downstream of the cI promoter it is possible to insert a structural gene the expression of which is regulatable by this promoter. As an example, *lac* genes (known as the *lac* operon) lacking the *lac* promoter have been inserted downstream of the cI gene and the gene product amplification obtained suggested that transcription of the *lac* genes was, at least in part, controlled by the cI promoter (cf. Example 8). It was thus demonstrated that it is possible to see the plasmids of the invention as cloning vectors for the insertion of a foreign gene. The cI promoter has the advantage of being regulatable by the cI repressor, too, so that amplification of the gene product takes place simultaneously with amplification of plasmid DNA.

The second promoter may also be a $\lambda$ promoter, such as $\lambda P_L$, or it may be a *recA* promoter. When the

second promoter is $\lambda P_L$, it may be inserted in the plasmid as a *Bam*HI - *Bgl*II fragment from phage $\lambda$, thus producing a convenient cloning site (*Bam*HI) for the insertion of DNA fragments generated by several restriction enzymes, such as *Bam*HI, *Bgl*II, *Sau*3A and *Bcl*I. $\lambda P_L$ may also be inserted on an appropriate DNA fragment from a plasmid carrying the promoter. $\lambda P_L$ is advantageous as a second promoter because it is also controlled by the temperature-sensitive cI repressor so that amplification conditions are the same.

If, on the other hand, the promoter is *recA*, it may be inserted in the plasmid of the invention as a *Bam*HI fragment from, e.g., pBEU14 (Uhlin and Clark, J. Bact. *148*, 1981, 386-90). This promoter functions in different ways depending on the microbial strain to which the *recA* promoter-carrying plasmid has been transformed. In a *recA* strain, *recA* is controlled by the *lexA* repressor, which is located on the chromosome of the microorganism. At, e.g., a temperature-induction of uncontrolled replication the copy number of the plasmid and hence the number of *recA* promoters gradually increases. As the *lexA* repressor is only produced in small amounts and functions by binding tightly to the *recA* promoter region, the available amount of *lexA* repressor will gradually be titrated when the copy number increases. Thus, there is a gradual derepression of *recA* and when the plasmid copy number becomes higher, the transcription from *recA* will correspondingly increase.

### The Plasmids of the Invention as Cloning and Production Vectors

In their capacity as cloning and production vectors, the plasmids of the invention have a number of characteristic properties:

1) The plasmids are small with a molecular weight of about $4.0\text{-}12.5 \times 10^6$ daltons. The small size facilitates handling and improves transformation efficiency.

2) The plasmids have a number of unique sites for restriction endonucleases. The term "unique" is understood to mean that, for a specific restriction endonuclease, the plasmid has one and only one site cleavable by the enzyme.

As already mentioned, restriction sites located within the replication region are not permissible as cloning sites. Thus, in the plasmids described in the following examples, the restriction sites *Sal*I and *Pst*I are not suitable as cloning sites as they are located in the replicon of the plasmid, whereas the restriction sites *Eco*RI and *Bam*HI are not located within this critical region and thus form useful sites for the introduction of foreign DNA. If a suitable restriction site is not to be found on the plasmid itself, it may be constructed by inserting a small DNA fragment (known as a linker) containing such a site into the plasmid as described in Example 4.

In order to ensure the expression of the inserted structural gene, however, it is also necessary to have an appropriate translational start signal (ribosomal binding site) located upstream of the structural gene itself but downstream of the second promoter.

One way of securing the presence of the ribosomal binding site is to insert a variety of of DNA fragments (e.g. as linkers), which may be prepared synthetically, each carrying the translation start signal for a different structural gene. Alternatively, a DNA fragment carrying a gene which is already known to be expressed in microorganisms may be inserted in the runaway cloning vectors *in toto*, thus ensuring the presence of the correct translation start signal.

3) Although, as mentioned above, it is an advantage to have as small a cloning vector as possible, it is, for many purposes, practical that the cloning vector carries genes for the expression of a so-called marker useful for the identification and/or selection of cells carrying the plasmid. The most useful marker is antibiotic resistance, for example ampicillin resistance, as this permits, after a treatment for transforming a recombinant plasmid to a microbial host, an easy counter-selection of microorganisms which have not received the recombinant plasmid. Another marker which is present in all the plasmids described in the following examples is $\lambda$ immunity which is encoded by the cI gene. A further useful property in plasmids of the invention may be the presence of a gene mediating a selectable phenotype within which a unique restriction site is located. Insertion of a DNA fragment at this site results in insertional inactivation of the gene. An example of this is described in Example 9 with respect to the gene mediat the Lac$^+$ phenotype and Example 12 with respect to the gene encoding chloramphenicol resistance. When it is desired to introduce a marker, for instance antibiotic resistance, into a plasmid of the invention which is to be used as a cloning vector, this may be done by transposition or by inserting a foreign DNA fragment in a manner known *per se*. An example of such transposition is shown in Example 4.

### Methods of cultivation

The present invention also provides a method for producing a gene product of plasmid DNA which comprises cultivating microorganisms carrying a plasmid in which a regulatable promoter has been inserted in such a way that transcription regulates replication, especially in such a way that increased transcription from this promoter leads to a substantially increased plasmid copy number, the method comprising at least a period of cultivation under conditions securing increased transcription from the regulatable promoter resulting in a substantially increased plasmid copy number, and harvesting, from the microbial culture, a gene product of the plasmid. The cultivation *per se* is suitably performed using conventional techniques, including conventional nutrient media which are known to be optimal to the microbial species in question. Also, the harvesting of the gene product is performed in accordance with well-known methods adapted to the identity and properties of the particular gene product prepared, the properties of the host microorganism, etc. The present invention further provides a method of growing the host microorganism to which the plasmid according to the invention has been transformed under conditions resulting in a constant, low plasmid copy number during the seeding and multiplication stages until the desired number of cells has been obtained and then cultivating the host microorganism under conditions leading to a substantially increased plasmid copy number.

Thus, the cultivation may initially be performed under conditions where any transcription from the promoter does not significantly influence the copy number of the plasmid, after which a substance which induces promoter activity is added to the culture medium in an amount leading to substantially increased replication. If desired, these cultivation conditions may be reversed after a period of time sufficient to obtain a substantial amplification of the plasmid, but sufficiently brief to avoid any substantial impairment of the microbial culture, the substance is removed from the culture medium leading to a slow decrease in plasmid copy number until it eventually reaches the pre-induction level. In this context, the term "removed" need not imply a complete absence of the substance in question from the culture medium, but only a reduction in the concentration of the substance to an extent where it no longer has any effect.

In an alternative method, employing a promoter which is inhibited, rather than induced, by a certain substance, the microbial culture may initially be cultivated in the presence of the substance inhibiting the activity of the particular promoter used, after which the concentration of the substance is reduced, e.g. by diluting the culture medium or by being gradually consumed by the cells, to an extent leading to activation of the promoter, thus causing a substantially increased plasmid copy number. If desired, the cultivation conditions may be reversed after a period of time sufficient to obtain a substantial amplification of the plasmid, but sufficiently brief to avoid any substantial impairment of the microbial culture, by adding the substance inhibiting promoter activity in an amount leading to a slow decrease in plasmid copy number until it reaches the pre-induction level.

The conditions under which the microorganism is grown may also comprise at least a period of cultivation at or near a temperature at which the plasmid copy number is substantially increased.

If the regulatable promoter is temperature-dependent or regulated by a temperature-sensitive factor, it is preferred that the propagation of the microorganism transformed with a plasmid of the invention up to a production size culture is performed at or near the temperature at which the plasmid shows a constant, low copy number in order avoid any inhibition of microbial growth by an increasing plasmid and gene product concentration. Then, the temperature may be shifted to a temperature at which the plasmid shows a substantially increased copy number. After a suitable production period, often until the growth of the microorganism is inhibited by the production of plasmid DNA and/or gene products from the plasmid, the harvesting of the gene product is performed. Depending on the particular conditions, it may be desired to perform the production cultivation continuously at a temperature which only approaches the temperature at which the plasmid copy number is substantially increased so as to permit the survival and continued growth of the host microorganism concomitantly with the formation of increased amounts of a gene product of the plasmid (which may then be continuously or intermittently harvested from the culture in a manner known *per se*).

Alternatively, the microorganism may be propagated up to a production size culture at the temperature at which the plasmid copy number is constant, whereupon the temperature is shifted to a temperature at or near the temperature at which the plasmid copy number is substantially increased, and this temperature is maintained for a period of time sufficient to obtain a substantial amplification of the plasmid, but sufficiently brief to avoid any substantial impairment of the microbial culture, after which the temperature is once more shifted to a temperature securing a low, constant plasmid copy number, and production cultivation is continued at this latter temperature causing a slow decrease in plasmid copy number until it reaches its initial level. This cultivation method is particularly important when the foreign gene inserted in the plasmid is derived from an organism which is not viable at temperatures above about 30°C so that gene products of such a gene may be denatured at higher temperatures Moreover, the yield of gene product is usually higher when this method of cultivation is employed (cf. Example 16; compare Uhlin et al., Gene *6*, 1979, 91-106, Table II).

The temperature at which the plasmid copy number is substantially increased may be higher than the temperature at which the plasmid has a constant, low copy number. This higher temperature may for instance be a temperature in the range of about 36-42°C.

DESCRIPTION OF THE DRAWING

Reference is made to the drawing in which
Fig. 1 is a schematic view of the replication region of the wild-type plasmid R1,
Fig. 2 is a key to the symbols used in the following figures, and
Figs. 3-14 show restriction maps of the plasmids described in the examples.

In Fig. 1, a schematic view of the basic replicon of plasmid R1 is shown in which the shaded areas denote the translated regions, the blank areas denote the transcripts and the dotted lines denote possible transcripts. Within the circle, the enlarged view of the hairpin loops is meant to illustrate the high degree of secondary structure of the CopA-RNA. *Ori* stands for origin of replication. The horizontally placed arrows denote the direction of transcription.

In Fig. 2, a key to the symbols used in the following figures is shown in which A denotes DNA from R1-type plasmids; B derives DNA derived from the bacteriophage λ (EDλ4); C denotes DNA from the Tn3 transposon (from EDλTn3); D denotes DNA from plasmid pBR322; E denotes DNA from plasmid pMC871; F denotes DNA from plasmid pVH1424; G denotes DNA from plasmid pSKS104; H denotes structural genes and important sites; I denotes a promoter with an indication of the direction of transcription; and J denotes a scale of the dimensions of the plasmids. The blank area denotes DNA from plasmid pBEU14.

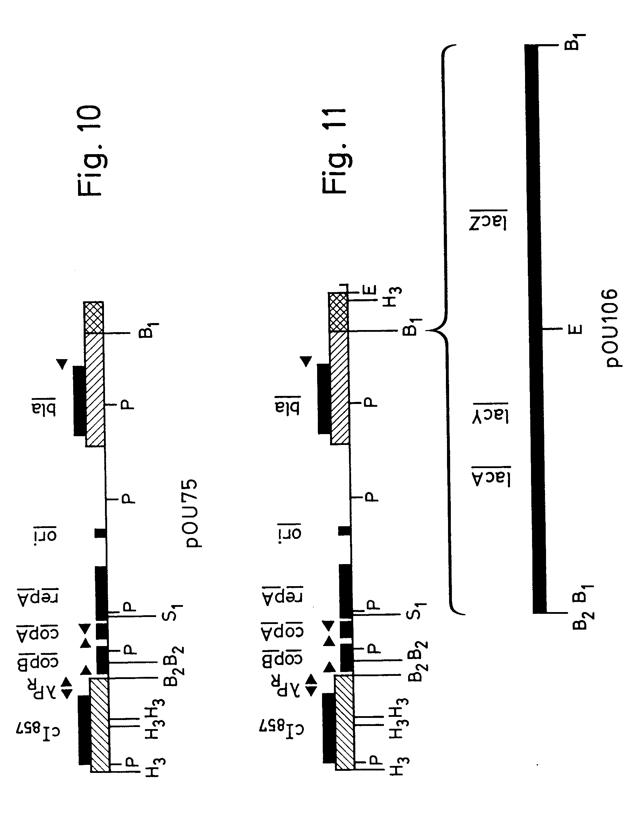

In Figs. 3-14, linear restriction maps of the plasmids described in the examples are shown in which the genotypes of the R1-type plasmids depicted are indicated above the horisontal line. Thus, *copB* represents a gene coding for a polypeptide which represses transcription from the *repA* promoter between the *copB* and *copA* genes; *copA* represents a gene coding for an RNA molecule that inhibits translation of RepA-mRNA; *repA* represents a gene coding for a protein required for plasmid R1 replication; *ori* denotes the vegetative origin of replication; *aphA* represents a gene encoding resistance to kanamycin; *bla* represents a gene encoding resistance to ampicillin; *lacA*, *lacY* and *lacZ* represent the inserted *lac* operon, of which *lacA* codes for transacetylase, *lacY* codes for permease and *lacZ* codes for β-galactosidase; $cl_{857}$ represents a gene coding for a temperature-sensitive repressor of the $\lambda P_R$ promoter; *tnpR* and *tnpA* represent genes coding for Tn3 transposition functions; *par* represents the region responsible for plasmid partitioning; *recA* represents a gene coding for a recombination protein; and PrecA represents the *recA* promoter. Below the horisontal line, the sites for restriction enzymes are shown in which P denotes *Pst*I; $B_2$ denotes *Bgl*II; $S_1$ denotes *Sal*I; E denotes *Eco*RI; $H_3$ denotes *Hind*III; B1 denotes *Bam*HI, S denotes *Sma*I; $B_2 B_1$ denotes a fusion of a *Bgl*II site and a *Bam*HI site; and C denotes *Cla*I. In Fig. 11, the plasmid depicted is drawn on the same scale as the plasmids in the other figures, and the bracketed insertion represents the inserted *lac* genes.

MATERIALS AND METHODS

The strain of *Escherichia coli* K-12 used was CSH50 (Δ *pro-lac*, *rpsL*; cf. J. Miller: *Experiments in Molecular Genetics*, Cold Spring Harbor Laboratory, Cold Spring Harbor, New York, 1972). Several plasmids (Table 1) and bacteriophages (Table 2) were used.

The experimental techniques used were standard techniques employed in the fields of microbial genetics (J. Miller: *Experiments in Molecular Genetics*, Cold Spring Harbor, New York, 1972) and genetic manipulation (Davis, Botstein and Roth: *A Manual for Genetic Engineering*; *Advanced Bacterial Genetics*, Cold Spring Harbor, New York, 1980).

All cells were grown in LB medium (Bertani, J. Bact. *62*, 1951, 293) or in $A^+B$ minimal medium (Clark and Maaløe, J. Mol. Biol. 23, 1967, 99), and the plates used were LA plates containing LB medium and 1.5% of agar. Preparation and analysis of plasmid DNA was performed using dye boyant density gradient centrifugation according to the method described by Stougaard & Molin, Anal. Biochem. *118*, 1981, 191. Labeling of DNA and preparation of lysates for the determination of plasmid content was performed according to Molin et. al., J. Bact. *138*, 1979, 70. The relative content of plasmid DNA was measured as amounts of labelled thymidine present in the plasmid band relative to that in the chromosomal band in the gradients. The restriction endonucleases employed in the examples were used in accordance with the prescriptions provided by the manufacturer. The partial restrictions were performed with a ten-fold dilution of the enzymes.

In addition, the following screening methods were employed:

*1. Screening for λ immunity (immλ⁺)*

In all the examples described, the $cI_{857}$ mutant allele is used. Plasmids carrying this gene make the host *E. coli* cell resistant to infection with lytic λ phages at 30°C. To select resistant cells more than $10^8$ λb2 phage particles are added to plates before spreading the bacteria. Surviving colonies are further tested for their plasmid content.

*2. Screening for copB⁺*

Genetic fusions between the *repA* promoter and the *lac* operon from which the *lac* promoter has been deleted are constructed and inserted into a p15 plasmid (pGA46). One resulting hybrid plasmid, pJL217, is responsible for a Lac⁺ phenotype when harboured by a Δ *lac* host *E. coli* strain.

The Lac⁺ phenotype is easily detected on McConkey lactose indicator plates (red colonies). The presence of a *copB*⁺ gene in cells harbouring pJL217 results in a Lac⁻ phenotype which appears as white colonies on the indicator plates. Thus, *copB*⁺ hybrids are easily scored as Lac⁻ colonies when plasmid DNA is transformed to *E. coli* Δ *lac* cells that harbour plasmid pJL217.

TABLE 1

Plasmids used

| Plasmid | Source |
| --- | --- |
| pKN1562 | S. Molin et al., J. Bact., *138*, 1979, pp. 70-79. |
| pBR322 | F. Bolivar et al., Gene *2*, 1977, p. 95. |
| pJL217 | Constructed by inserting a *Sau*3A fragment from pKN1562 carrying the *repA* promoter in the *Bgl*II site of pJL207, a hybrid plasmid carrying *lac* genes without the *lac* promoter. |
| pOU16 | Light & Molin, Mol. Gen. Genet. *184*, 1981, p. 57. |
| pGA46 | An & Friesen, J. Bact. *140*, 1979, pp. 400-407. |
| pMC903 | Casadaban et al., J. Bact. *143*, 1980, p. 971. |
| pMC871 | Casadaban et al., *op. cit.*, 971. |
| pVH1424 | Constructed by P. Valentin-Hansen by inserting a *Sau*3A fragment carrying the *deo* promoter from plasmid pVH17 (Valentin-Hansen et al., EMBO J., 1982, 317) in the *Bam*HI site of plasmid pMC1403 (Casadaban et al., *op. cit.*, 971). |
| pSKS104 | Constructed by M. Casadaban by inserting a *Pvu*II fragment containing the *lac* promoter and translation initiation region from pM13mp7 (Messing et al., Nucleic Acids Res. *9*, 1981, 309) in the *Sma*I site of pMC1403, followed by homologous recombination between the *lacZ* segments. |

## Table 1 (cont.)

| pKN184 | Nordström et al., Plasmid *4*, 1980, 322. |
| pJL3 | Molin et al., Mol. gen. Genet. *181*, 1981, 123-130. |
| pVH1451 | Valentin-Hansen et al., *op.cit.* |
| pBEU14 | Uhlin & Clark, J. Bact. *148*, 1981, 386-90 |
| pMC1403 | Casadaban et al., J. Bact. *143*, 1980, 971 |

TABLE 2

| Bacteriophages Used | |
| --- | --- |
| Phage | Source |
| EDλ4 | Dempsey & Willetts, J. Bact. *126*, 1976, p. 166. |
| EDλTn3 | Nordström, Molin, Aagaard-Hansen, Plasmid, 4, 1980, pp. 215-27 |

*Parent plasmids*

Plasmid pKN1562 is a derivative of plasmid R1, in all essentials constructed as described in Molin et al.: "Clustering of Genes Involved in Replication, Copy Number Control, Incompatibility, and Stable Maintenance of the Resistance Plasmid *R1drd-19*", J. Bact. *138*, 1979, pp. 70-79, except for a few modifications added by later research. pKN1562 has a molecular weight of $6.9 \times 10^6$ daltons and the following genotype: $copA^+$, $copB^+$, $repA^+$, $aphA^+$, $\Delta$ *par* (see Fig. 3). It carries resistance to kanamycin. The plasmid possesses the replication properties of the wild-type plasmid, has a copy number of 3-5 per fast growing cell and is lost with a frequency of 1% per generation due to the lack of the *par* gene (the gene for partitioning).

By restricting pKN1562 with *Bgl*II to delete part of the *copB* gene and ligating the sticky ends, another plasmid, pJL20, is constructed. The molecular weight of this plasmid is $6.7 \times 10^6$ daltons and its geno-type is as follows: $copA^+$, $\Delta$ *copB*, $repA^+$, $aphA^+$, $\Delta$ *par*. The plasmid has a copy number of 25-40 per fast growing cell, and there is no loss of the plasmid. This plasmid, too, mediates resistance to kanamycin.

*Plasmid designation*

It should be noted that the plasmids designated pJEL... in the examples and drawing of the basic application have been renamed pOU... in the present application.

EXAMPLE 1

*Construction and characterization of pOU51*

DNA from plasmid pJL20 and phage EDλ4 was prepared according to standard methods. Plasmid and phage DNA was mixed at a final concentration of 20μg/ml of each and in a final volume of 100μl, restricted with *Bgl*II for 30 minutes, heat inactivated at 70°C for 10 minutes, and ligated with T4 DNA ligase overnight at 15°C. Aliquots of the ligation mixture were transformed to *E. coli* strain CSH50. Transformants were selected on plates containing 50 μg/ml kanamycin onto which a suspension with about $10^9$ λb2 particles

had been spread. The plates were incubated for 20 hours at 30°C.

Surviving colonies were reisolated and tested for resistance to λb2 by cross-streaking at 30°C, for growth at 42°C by streaking on Km plates at 42°C, and for the molecular weight of the plasmid by preparing plasmid DNA and analysing on agarose gels.

In this way a plasmid, pOU51, was found which mediates resistance to Km and to λ infection, renders the host bacterium temperature-sensitive for growth, and has an insertion of phage DNA in pJL20 corresponding to about $1.6 \times 10^6$ daltons. The total molecular weight of the plasmid was $8.4 \times 10^6$ daltons. These properties are all carried by the plasmid since new *E. coli* recipient strains acquire all the plasmid's phenotypic properties when transformed with pOU51 DNA.

DNA was prepared from pOU51 and the plasmid mapped with restriction enzymes by purifying the plasmid DNA, cleaving it with restriction enzyme(s) and analysing the resulting fragments by means of agarose gel electrophoresis (cf.Fig. 4). In this way the inserted $1.6 \times 10^6$ dalton fragment was identified as a *Bgl*II fragment from phage λ carrying the cI repressor gene (responsible for λ immunity at 30°C) and the λ$P_R$ promoter, and the orientation of the inserted fragment was determined to be as shown in Fig. 4. From the restriction map it further appears that pOU51 has a unique site for the restriction endonuclease *Eco*RI which makes it possible to use the plasmid as a cloning vector.

The effect of the plasmid on the growth of the cells was investigated by growing a culture in medium at 30°C. At a time when growth was exponential the temperature was shifted to 42°C and cell growth followed (determined spectrophotometrically). Within 1.5-2 hours of growth at 42°C the culture ceased to grow.

The plasmid DNA content was measured as described in MATERIALS AND METHODS from 10 ml cultures growing in $A^+B$ minimal medium supplemented with 0.2% of glucose and 1% of casamino acids. One 10 ml culture was labelled with 50 μCi of $^3$H-thymidine at 30°C, the other received the isotope just after a shift to 42°C until the cells ceased to grow. The relative content of plasmid DNA was 2% at 30°C corresponding to 25-40 plasmid copies per cell. At 42°C the relative content was more than 50% corresponding to more than 1000 plasmid copies per cell.

The properties of this plasmid are shown in Table 5.

The strain of *E. coli* CSH50/pOU51 is deposited in the German Collection of Microorganisms (Deutsche Sammlung von Mikroorganismen, Grisebachstrasse 8, D-3400 Göttingen), in the following abbreviated to DSM, under the Accession No. 2467.

EXAMPLE 2

*Construction and characterization of pOU53*

By using the plasmid described in Example 1 as a starting material and by partially restricting pOU51 with *Hind*III, the title plasmid was constructed from which the gene for encoding kanamycin resistance had been deleted, but which had retained the gene for encoding λ immunity. The plasmid was transformed to *E. coli* strain CHS50. pJEL53 had a molecular weight of $3.2 \times 10^6$ daltons and the following genotype: $copA^+$, Δ *copB*, $repA^+$, Δ *par*, $imm\lambda^+$, Δ *aphA*.

The plasmid was mapped with restriction enzymes as described in Example 1 (cf. Fig. 5). From the restriction map it appears that pOU53 has a unique site for the restriction endonuclease *Eco*RI which makes it possible to use the plasmid as a cloning vector.

To demonstrate the presence of the desired plasmid in the host cells, cell cultures were grown on plates containing λb2 phage particles as described above to test for resistance to λ infection. The cells were further tested for sensitivity to kanamycin by replica plating colonies on plates containing 50 μg/ml Km. To test for temperature-sensitive growth, the cells were streaked on plates at 42°C and grown in the manner described in Example 1.

When the plasmid DNA content was measured in the manner described in Example 1, the cells were found to contain 20-40 plasmid copies at 30°C and more than 1000 plasmid copies at 42°C. There was no loss of the plasmid.

The properties of this plasmid are shown in Table 5.

The strain of *E. coli* CSH50/pOU53 is deposited in the DSM under the Accession No. 2468.

EXAMPLE 3

*Construction and characterization of pOU56*

By *in vivo* insertion of the Tn3 transposon from a λ::Tn3 phage including the genes for transposition

and ampicillin resistance ($\beta$-lactamase) (Nordström, Molin and Aagaard-Hansen, Plasmid *4*, 1980, 215-27) in pOU53, the title plasmid was constructed. Plasmid DNA was isolated from a strain carrying the $\lambda$ Tn3 phage in the chromosomes and plasmid pOU53. The plasmid DNA was transformed to *E. coli* strain CSH50 selecting for ampicillin resistance and $\lambda$ immunity. pOU56 had a molecular weight of $6.5 \times 10^6$ daltons and the following genotype: *copA*⁺, $\Delta$ *copB*, *repA*⁺, $\Delta$ *aphA*, *imm* $\lambda$⁺, *bla*⁺ (::Tn3).

The plasmid was mapped with restriction enzymes as described in Example 1 (cf. Fig. 6). The restriction map shows where the Tn3 transposon has been inserted in pOU56, and further shows that the plasmid has a unique site for each of the restriction endonucleases *Eco*RI and *Bam*HI. While the plasmid is useful as a starting material for preparing the plasmid pOU57 (cf. Example 4 below), it is not preferred as a cloning vector as, due to the presence of the transposition gene, antibiotic resistance may be transferred to other bacteria.

To demonstrate the presence of the desired plasmid in the host cell, cell cultures were grown on plates containing $\lambda$b2 phage particles as described above to test for resistance to $\lambda$ infection. The cells were further tested for ampicillin resistance on plates containing 50 $\mu$g/ml ampicillin. The test for temperature-sensitive growth was performed in the manner described above.

pOU56 was found to have the same replication properties as pOU53.

The properties of this plasmid are shown in Table 5.

The strain of *E. coli* CSH50/pOU56 is deposited in the DSM under the Accession No. 2469.

EXAMPLE 4

*Construction and Characterization of pOU57 (a type A plasmid)*

In order to eliminate the possibility of transposition of Tn3 from plasmid pOU56 due to its carrying the entire transposon, the plasmid was cleaved at the *Bam*HI and *Eco*RI sites to delete the transposition gene whereas the gene coding for $\beta$-lactamase was retained. In order to effect annealing of the sticky ends, a small *Bam*HI - *Eco*RI fragment from plasmid pBR322 was inserted, thus producing plasmid pOUL57. The plasmid was transformed to *E. coli* strain CSH50. pOU57 had a molecular weight of $4.2 \times 10^6$ daltons, and the following geno-type: *copA*⁺, $\Delta$ *copB*, *repA*⁺, $\Delta$ *par*, $\Delta$ *aphA*, *imm* $\lambda$⁺, *bla*⁺ ($\Delta$Tn3).

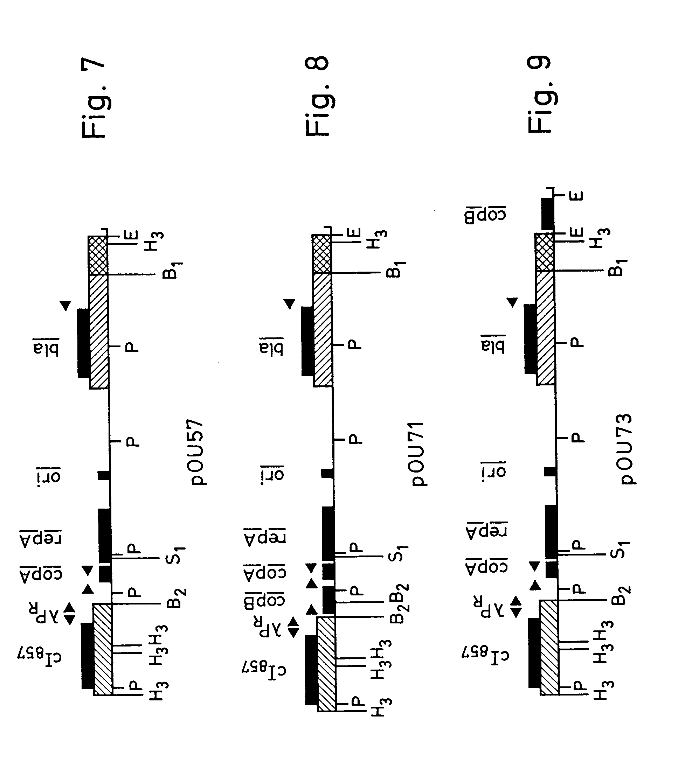

The plasmid was mapped with restriction enzymes as described in Example 1 (cf. Fig. 7). From the restriction map it appears that pOU57 has unique sites for each of the restriction endonucleases *Bam*HI and *Eco*RI which makes it useful as a cloning vector.

To demonstrate the presence of the desired plasmid in the host cell, cell cultures were grown on plates containing $\lambda$b2 phage particles as described above to test for resistance to phage infection. The cells were further tested for ampicillin resistance in the manner described above. The molecular weight of the plasmid was determined by means of agarose gel electrophoresis. The test for temperature-sensitive growth was performed in the manner described above.

pOU57 was found to have the same replication properties as pOU53.

The properties of this plasmid are shown in Table 5.

The strain of *E. coli* CSH50/pOU57 is deposited in the DSM under the Accession No. 2470.

EXAMPLE 5

*Construction and Characterization of pOU71 (a type B plasmid)*

By using the plasmid described in Example 4 as a starting material and by inserting a *Bgl*II fragment from pKN1562 including part of the *copB* gene in the *Bgl*II site of pOU57, the title plasmid was constructed which carried both the cI-$\lambda$P$_R$ repressor-promoter system and the wild-type gene for the *copB* inhibitor of R1-type plasmids. The plasmid was transformed to E. coli strain CSH50. The molecular weight of pOU71 was $4.3 \times 10^6$ daltons and the genotype was as follows: *copA*⁺, *copB*⁺, *repA*⁺, $\Delta$ *par*, $\Delta$ *aphA*, *imm* $\lambda$⁺, *bla*⁺.

The plasmid was mapped with restriction enzymes as described in Example 1 (cf. Fig. 8). From the restriction map it appears that pOU71 has the same unique restriction sites as pOU57.

To demonstrate the presence of the desired plasmid in the host cell, cell cultures were tested for resistance to $\lambda$ infection and ampicillin resistance in the manner described above. The plasmids were then screened for the presence of the *copB* gene by means of the method described in the section entitled "Screening for *copB*⁺" (p. 25 of the present application). The test for temperature-sensitive growth was carried out in the manner described above. Finally, the number of plasmid copies at 30°C was determined by means of radiolabelling and gradient centrifugation.

When the content of plasmid DNA was measured in the manner described in Example 1, the cells were found to contain 3-5 plasmid copies at 30°C and more than 1000 plasmid copies at 42°C. At 30°C, the plasmid was lost with a frequency of 1% per generation.

The properties of this plasmid are shown in Table 5.

The strain of *E. coli* CSH50/pOU71 is deposited in the DSM under the Accession No. 2471.

EXAMPLE 6

*Construction and Characterization of pOU73 (a type C plasmid)*

By using the plasmid described in Example 4 as a starting material and by inserting an *Eco*RI fragment from pOU16 carrying the *copB* gene in the *Eco*RI site of pOU57, the title plasmid was constructed having the gene for the *copB* inhibitor as well as the cI-$\lambda P_R$ repressor-promoter system. The plasmid was transformed to *E. coli* strain CSH50. pOU73 had a molecular weight of $4.4 \times 10^6$ daltons and the following genotype: $copA^+$, $copB^+$, $repA^+$, $\Delta\ par$, $\Delta\ aphA$, $imm\ \lambda^+$, $bla^+$.

The plasmid was mapped with restriction enzymes as described in Example 1 (cf. Fig. 9). From the restriction map it appears that the *copB* gene has been inserted in a region of the plasmid where it is not naturally located. It further appears that the plasmid has a unique site for the restriction endonuclease *Bam*HI so that it may be used as a cloning vector.

The same screening methods as for pOU71 were employed.

When the content of plasmid DNA was measured in the manner described in Example 1, the cells were found to contain 0.5-1 plasmid copy at 30°C and more than 1000 plasmid copies at 42°C. At 30°C, the plasmid was lost with a frequency of more than 5% per generation.

The properties of this plasmid are shown in Table 5.

The strain of E. coli CSH50/pOU73 is deposited in the DSM under the Accession No. 2472.

EXAMPLE 7

*Construction and characterization of pOU75 (a type B plasmid)*

By using the plasmid described in Example 5 as a starting material and by partially restricting pOU71 with *Hind*III, the title plasmid was constructed from which the *Eco*RI site had been deleted. The plasmid was transformed to *E. coli* strain CSH50. pOU75 had a molecular weight of $4.2 \times 10^6$ daltons and the following genotype: $copA^+$, $copB^+$, $repA^+$, $\Delta\ par$, $\Delta\ aphA$, $imm\lambda^+$, $bla^+$.

The plasmid was mapped with restriction enzymes as described in Example 1 (cf. Fig. 10). From the restriction map it appears that the deletion of the *Hind*III fragment results in the removal of the *Eco*RI site of pOU71.

To demonstrate the presence of the desired plasmid in the host cell, cell cultures were tested for resistance to $\lambda$ infection and ampicillin resistance, as well as temperature-sensitive growth in the manner described above. The plasmid was also tested for loss of the *Eco*RI site by restricting with the enzyme.

pOU75 was found to have the same replication properties as pOU71.

The properties of this plasmid are shown in Table 5.

The strain of *E. coli* CSH50/pOU75 is deposited in the DSM under the Accession No. 2473.

EXAMPLE 8

*Gene product amplification*

Plasmid pMC903 (Casadaban et al., J. Bact. *143*, 1980, 971) was cleaved with the restriction enzymes *Bam*HI and *Bgl*II producing a *Bam*HI - *Bgl*II fragment carrying *lac* genes. Plasmid pOU71 was cleaved with *Bam*HI, and the *Bam*HI - *Bgl*II fragment obtained above was inserted at this site as shown in Fig. 11 followed by ligation with T4 DNA ligase, thus producing the plasmid pOU106 which was transformed to *E. coli* strain CSH50. The cells were streaked onto McConkey lactose indicator plates (see J. Miller, *Experiments in Molecular Genetics,* Cold Spring Harbor, 1972) containing 50 $\mu$g/ml ampicillin and incubated at 30°C to select for red colonies ($Lac^+$). The temperature was then shifted to 42°C, and the amplification of gene product ($\beta$-galactosidase) was measured as shown in Table 3. The measuring was performed according to the method described by J. Miller, *op. cit.*

19

TABLE 3

| Amplification of gene product | | | |
|---|---|---|---|
| Plasmid of the Invention | | Prior Art Plasmid (pKN410) | |
| Minutes after shift to 42°C | Accumulated relative specific activity[1] of gene product[2] | Minutes after shift to 42°C | Accumulated relative specific activity of gene product[3] |
| 0 | 10 | 0 | 10 |
| 10 | 21 | | |
| 25 | 34 | | |
| 55 | 62 | 60 | 20 |
| 83 | 107 | 90 | 47 |

[1] Enzyme activity per total amount of protein, i.e. the increase of $\beta$-galactosidase per cell.
[2] $\beta$-galactosidase mediated by plasmid pOU106.
[3] $\beta$-lactamase mediated by plasmid pKN410 (Uhlin et al., Gene *6*, 1979, Table II, p. 100).

From the table it appears that the level of gene product amplification is comparable to that obtained with other runaway vectors (e.g. Uhlin et al., Gene *6*, 1979, 91-106).

The promoter from which the $\beta$-galactosidase gene is expressed is the cl promoter and/or the tetracycline promoter located on the DNA fragment from pBR322 which, although it is relatively weak, cannot be dismissed as an influencing factor and which transcribes in the same direction as the cl promoter.

pOU106 was mapped with restriction enzymes as described in Example 1 (cf. Fig. 11). From the restiction map it appears that the plasmid has a unique site for the restriction endonuclease *Bam*HI immediately downstream of the inserted *lac* genes, which makes the plasmid useful as a cloning vector.

The properties of this plasmid are shown in Table 5.

The strain of *E. coli* CSH50/pOU106 is deposited in the DSM under the Accession No. 2474.

EXAMPLE 9

*Construction and characterization of pOU79 (a type B plasmid)*

Plasmid pOU71 was cleaved completely with *Bam*HI and partially with *Pst*I and ligated with a DNA fragment (carrying the *lac* operon) from plasmid pMC871 (Casadaban et al., J. Bact. *143*, 1980, 971) which had been cleaved completely with *Bam*HI and *Pst*I.

The ligation mixture was transformed to *E. coli* strain CSH50 selecting for resistance to ampicillin on McConkey lactose indicator plates containing 50 $\mu$g/ml ampicillin which were incubated at 30°C. After 20 hours' incubation at 30°C, the plates were incubated for a short period (1-2 hours) at 42°C, and colonies that shifted from a Lac⁻ (white) to a Lac⁺ (red) phenotype were further analysed. The title plasmid was identified from one such colony. pOU79 had a molecular weight of $8.8 \times 10^6$ daltons and the following genotype: $copA^+$, $copB^+$, $repA^+$, $\Delta\, par$, $imm\lambda^+$, $bla^+$, $lac^+$.

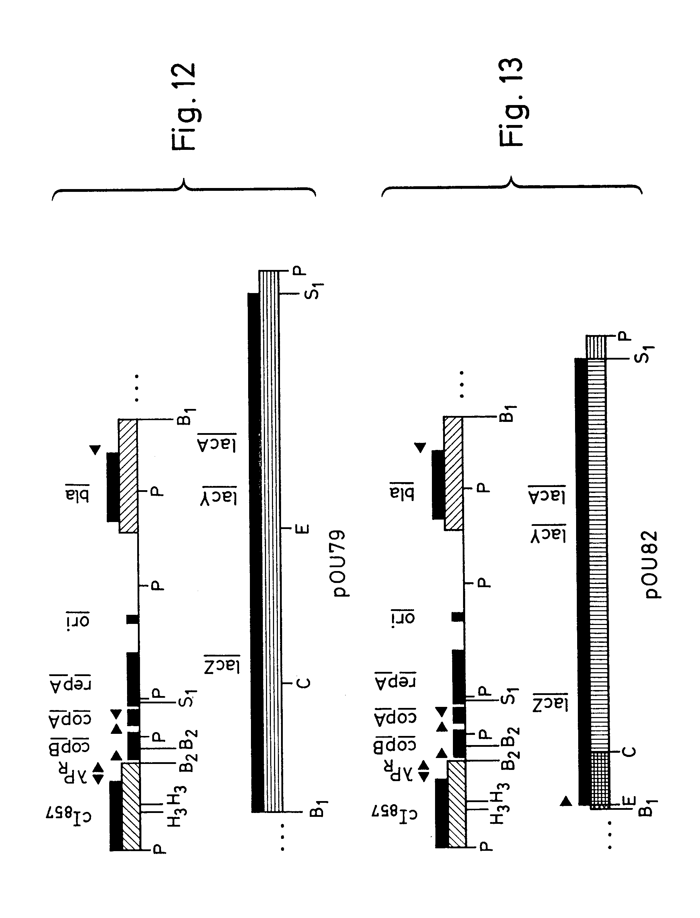

The plasmid was mapped with restriction enzymes as described in Example 1 (cf. Fig. 12). From the restriction map it appears that the plasmid has a unique site for the restriction endonuclease *Bam*HI just upstream of the *lac* genes. Insertion of promoter-carrying DNA fragments in the proper orientation at this site results in transcription of the *lac* genes thus giving rise to a Lac⁺ phenotype of cells carrying such hybrid plasmids at 30°C. Lac⁺ cells are easily identified on McConkey lactose indicator plates.

The plasmid also has a unique site for the restriction endonuclease *Eco*RI located at one end of the *lacZ* gene. Insertion of *Eco*RI fragments at this site eliminates the activity of the *lacZ* gene product, $\beta$-galactosidase, resulting in a Lac⁻ phenotype which is easily scored as the formation of colourless colonies on McConkey lactose indicator plates after a temperature shift from 30°C to 42°C (cf. the description above). The plasmid is thus useful as a cloning vector.

pOU79 was found to have the same replication properties as pOU71.

The properties of this plasmid are shown in Table 5.

The strain of *E. coli* CSH50/pOU79 is deposited in the DSM under the Accession No. 2481.

EXAMPLE 10

20

*Construction and characterization of pOU82 (a type B plasmid)*

Plasmid pOU79 was cleaved completely with *Bam*HI and partially with *Sal*I, and a *Bam*HI - *Sal*I fragment from pSKS104 carrying the *lac* operon from which the *lac* promoter had been deleted and the first four codons of the *lacZ* gene was inserted to construct pOU80 (not shown).

pOU80 is Lac⁻ and has a *Bam*HI site and an *Eco*RI site just upstream of the *lac* genes. Moreover, the *Eco*RI site normally found in the *lacZ* gene is missing since the *lac* genes in pSKS104 are derived from pMC1403 (Casadaban et al., J. Bact. *143*, 1980, 971).

The title plasmid was constructed by replacing an *Eco*RI - *Cla*I fragment in the *lacZ* gene (cf. the map of pOU79, Fig. 12) of pOU80 with an *Eco*RI - *Cla*I fragment from pVH1424 carrying the *deo* promoter and the amino terminal end of the *lacZ* gene. The replacement results in a reconstruction of the *lacZ* gene and, due to the presence of the *deo* promoter, the resulting hybrid plasmid, pOU82, mediates a Lac⁺ phenotype when transformed to *E. coli* strain CSH50. pOU82 had a molecular weight of $8.1 \times 10^6$ daltons and the following genotype: *copA*⁺, *copB*⁺, *repA*⁺, Δ *par*, *imm*λ⁺, *bla*⁺, *lac*⁺.

The plasmid is eventually lost from the cells at 30°C due to the lack of the *par* region, which is easily observed on non-selective McConkey lactose indicator plates (cf. Example 11).

The plasmid was mapped with restriction enzymes as described in Example 1 (cf. Fig. 13). From the restriction map it appears that the plasmid has a unique site for each of the restriction endonucleases *Eco*RI and *Bam*HI, which makes it possible to use the plasmid as a cloning vector.

pOU82 was found to have the same replication properties as pOU71.

The properties of this plasmid are shown in Table 5.

The strain of *E. coli* CSH50/pOU82 is deposited in the DSM under the Accession No. 2482.

EXAMPLE 11

*Construction and characterization of pOU91 (a type B plasmid)*

To construct a plasmid which is stable with respect to partitioning, pOU82 was cleaved with *Eco*RI, and the *Eco*RI-A fragment including the wild-type *par* region of a plasmid R1 derivative, pKN184 (Nordström et al., Plasmid *4*, 1980, 322), was inserted at this site followed by ligation. The resulting plasmid (pOU90, not shown) was transformed to *E. coli* strain CSH50, and the cells were grown selectively on plates containing 50 μg/ml ampicillin at 30°C. As a control, cells containing pOU82 were grown in a similar manner to ensure the presence of the plasmids in both starting colonies. Next, samples of both colonies were streaked on plates not containing any antibiotic, and the cells were left to grow for 25 cell generations. To screen for the stable inheritance of the Lac⁺ phenotype, cells from both the resulting colonies were streaked onto McConkey lactose indicator plates at 30°C where the cells to which pOU90 had been transformed generated red colonies which showed that this plasmid had been retained, while the control cells to which pOU82 had been transformed generated both red and colourless colonies which means that, during the selection-free period, pOU82 had been lost from some of the cells.

The intermediate plasmid, pOU90, was then cleaved completely with *Bam*HI and partially with *Sau*3A to reduce the size of the plasmid, thus producing the title plasmid. After transformation to *E. coli* strain CSH50, plasmid stability was determined in the manner described above. pOU91 had a molecular weight of $12.5 \times 10^6$ daltons and the following genotype: *copA*⁺, *copB*⁺, *repA*⁺, *par*⁺, *imm*λ⁺, *bla*⁺, *lac*⁺.

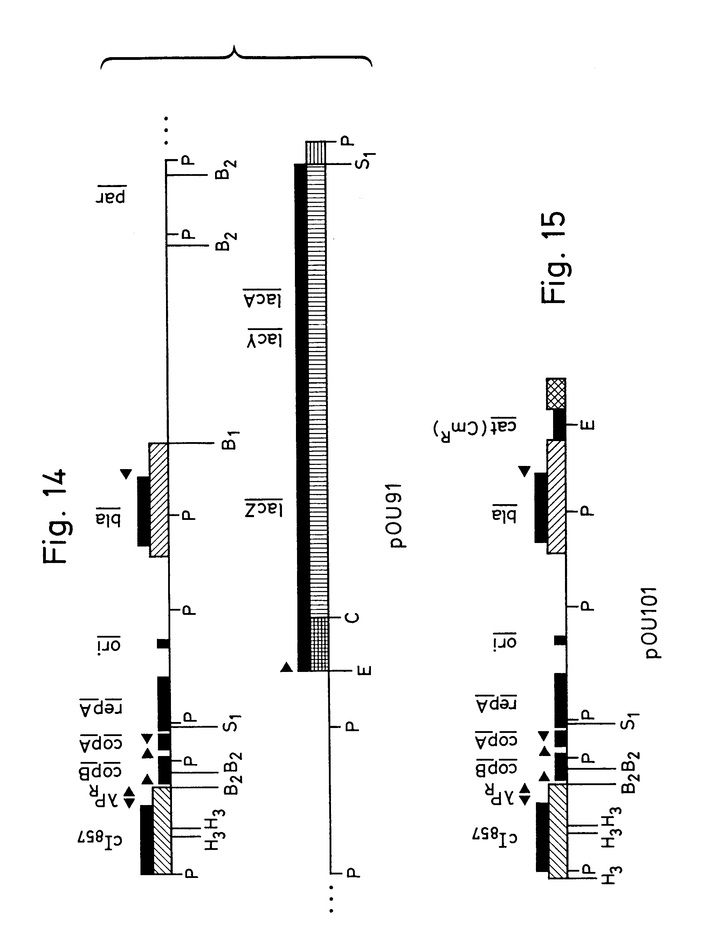

The plasmid was mapped with restriction enzymes as described in Example 1 (cf. Fig. 14). From the restriction map it appears that pOU91 has unique sites for each of the restriction endonucleases *Eco*RI and *Bam*HI, which makes the plasmid useful as a cloning vector.

pOUL91 was found to have the same replication properties as pOU71 but, in contradistinction to pOU71, the plasmid is completely stable at 30°C.

The properties of this plasmid are shown in Table 5.

The strain of *E. coli* CSH50/pOU91 is deposited in the DSM under the Accession No. 2483.

EXAMPLE 12

*Construction and characterization of pOU101 (a type B plasmid)*

By using the plasmid described in Example 7 as a starting material and by inserting a *Sau*3A fragment carrying the gene coding for chloramphenicol acetyl transferase (*cat*⁺) in the *Bam*HI site of pOU75, the title plasmid was constructed which conferred chloramphenicol resistance to the host cell. The plasmid was transformed to *E. coli* strain CSH50. pOU101 had a molecular weight of $4.7 \times 10^6$ daltons and the following genotype: $copA^+$, $copB^+$, $repA^+$, $\Delta\ par$, $\Delta\ aphA$, $imm\lambda^+$, $bla^+$, $cat^+$.

The plasmid was mapped with restriction enzymes as described in Example 1 (cf. Fig 15). From the restriction map it appears that pOU101 has a unique site for the restriction endonuclease *Eco*RI which makes it possible to use the plasmid as a cloning vector. Moreover, insertion of *Eco*RI fragments inactivates the $cat^+$ gene allowing screening for *Eco*RI hybrids.

To demonstrate the presence of the desired plasmid in the host cell, cell cultures were tested for resistance to λ infection and ampicillin resistance in the manner described above. Further, the cells were tested for resistance to chloramphenicol by growing cultures on plates containing 20 $\mu$g/ml chloramphenicol. The cells were also tested for temperature-sensitive growth in the manner described above. pOU101 was found to have the same replication properties as pOU71.

The properties of this plasmid are shown in Table 5.

The strain of *E. coli* CSH50/pOU101 is deposited in the DSM under the Accession No. 2757.

EXAMPLE 13

*Construction and Characterization of pOU110*

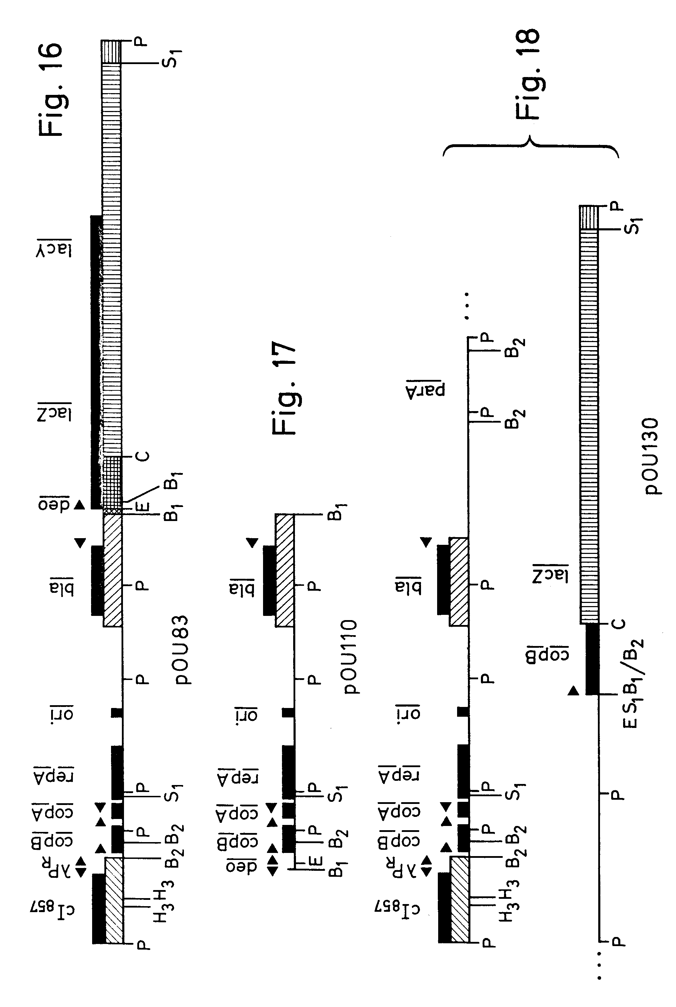

The *Eco*RI - *Cla*I fragment containing part of the *lac*Z gene was deleted from plasmid pOU80 (described in Example 10; cf. the map of pOU79, Fig. 12) and replaced with an *Eco*RI - *Cla*I fragment from plasmid pVH1451 (Valentin-Hansen et al., the EMBO Journal 1, 1982) carrying the *deo* promoter and the amino terminal end of the *lac*Z gene. The resulting plasmid was denoted pOU83 (cf. Fig. 16).

Plasmid pOU83 was partially restricted with *Bam*HI and *Bgl*II in order to delete the *lac* genes and the DNA fragment carrying the cI repressor gene and the $\lambda P_R$ promoter. After ligation and transformation to CSH50, a plasmid with the desired properties was isolated, resulting in a close fusion of the *deo* promoter and the *copB* gene. The plasmid, denoted pOU110, had a molecular weight of $3,2 \times 10^6$ daltons and the following genotype: $copA^+$, $copB^+$, $repA^+$, $\Delta\ par$, $bla^+$.

The plasmid was mapped with restriction enzymes as described in Example 1 (cf. Fig. 17). From the restriction map it appears that the plasmid has a unique site for each of the restriction endonucleases *Eco*RI and *Bam*HI which makes it possible to use the plasmid as a cloning vector.

When cultures of *E.coli* strain SØ929 (*cytR, lac, deo; recA*), to which pOU110 had been transformed, were grown overnight in LB medium without glucose, uncontrolled plasmid replication occurred at a cell density of above $2 \times 10^8$ cells/ml.

The strain of E.coli CSH50/pOU110 is deposited in the DSM under the Accession No. 2758. EXAMPLE 14

*Construction and Characterization of pOU130*

Plasmid pOU91 was cleaved with *Bam*HI and the *Bam*HI site was deleted by means of the exonuclease *Bal*31 to produce plasmid pOU92 (not shown). The plasmid was restricted with *Eco*RI and *Cla*I and a corresponding *Eco*RI - *Cla*I fragment from plasmid pMC1403 (Casadaban et al., J. Bact *143*, 1980, 971) was inserted to produce plasmid pOU93 which carried the *lac* operon without the *deo* promoter. At the *Bam*HI site thus produced a *Bgl*II fragment carrying a *copB* gene was inserted to produce plasmid pOU130 (cf. Fig. 18) followed by ligation and transformation to *E.coli* strain CSH50. The cells were streaked onto McConkey lactose indicator plates containing 50 $\mu$m/ml ampicillin and incubated at 30°C to select for red colonies (Lac$^+$). The temperature was then shifted to 42°C, and the amplification of $\beta$-galactosidase is shown in Table 5. After 45 minutes of incubation at 42°C, the temperature was lowered to below 38°C. The $\beta$-galactosidase amplification measured after the temperature shift is shown in Table 5.

TABLE 4

| Expression of $\beta$-galactosidase from pOU130 after amplification of plasmid DNA | | |
|---|---|---|
| Growth conditions | [d]Spec. act. of $\beta$-galactosidase | Amplification factor |
| [a] 30°C; steady state | 0.03 | 1 |
| [b] 30°C 42°C | 0.7 | 23 |
| [c] 30°C 42°C 37°C | 2.5 | 83 |

[a] Cells of CSH50 harbouring pOU130 grown exponentially in LB medium at 30°C.

[b] After exponential growth at 30°C the culture was shifted to 42°C, at which temperature growth was continued for 2 hrs.

[c] After exponential growth at 30°C the culture was shifted to 42°C for 45 minutes, after which it was shifted to 37°C for 90 minutes.

[d] See Materials and Methods. Specific activities of $\beta$-galactosidase are expressed as described by Light and Molin, Mol. Gen. Genet. 1981, (pp. 56-61).

From the table it appears that gene product amplification is considerably improved when employing a shift back to a lower temperature, thus demonstrating that this method is particularly useful to obtain a maximum expression of the gene products in question.

As $\beta$-galactosidase is constitutively expressed from such a gene fusion (Light & Molin, J. Bact. *151*, 1982, 1129-1135), the levels of enzyme activity accurately reflect the gene product amplification capacity of the plasmids of the invention.

The strain of *E.coli* CHS50/pOU130 is deposited in the DSM under the Accession No. 2759.

EXAMPLE 15

*Construction and characterization of pLC31*

pOU75 was cleaved with *Bam*HI, and a *Bam*HI fragment from plasmid pBEU14 (Uhlin and Clark, J. Bact. *148*, 1981, 386-90) carrying the *recA* promoter and gene was inserted followed by transformation to *E.coli* strain CSH50. The title plasmid had a molecular weight of $6.3 \times 10^6$ daltons and the following genotype: *recA, bla*[+], *copA*[+], *copB*[+], *repA*[+], *immm*λ[+].

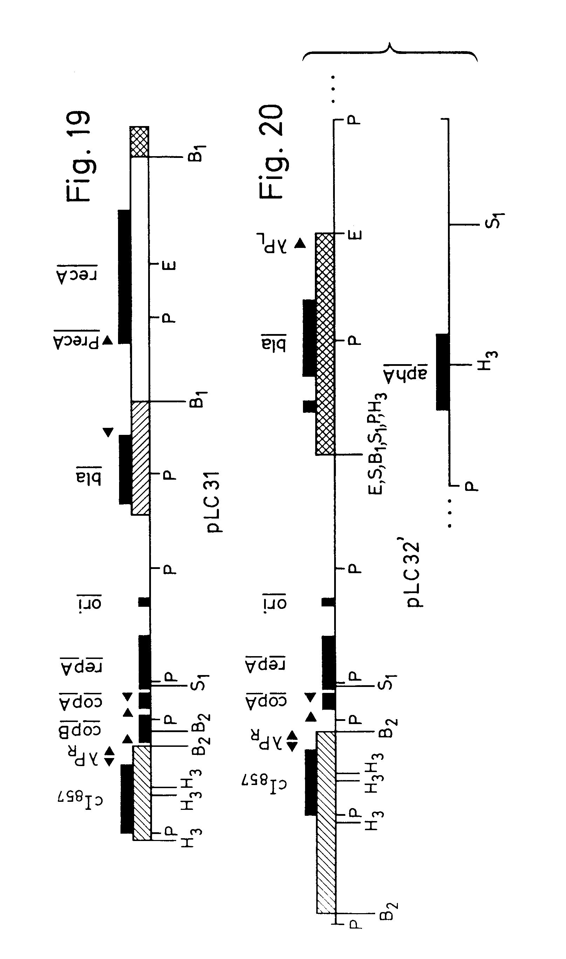

The plasmid was mapped with restriction enzymes as described in Example 1 (cf. Fig. 19). From the restriction map it appears that pLC31 contains a unique site for the restriction enzyme *Eco*RI downstream of the *recA* promoter suitable for the insertion of foreign genes.

pLC31 was found to have the same replication properties as pOU71.

When cells containing pLC31 were grown at 30°C, the promoter remained fully repressed, while after a temperature shift to 42°C, the *lexA* repressor was gradually titrated and transcription from *recA* was initiated leading to an amplified production of *recA* gene product which could be detected by polyacrylamide gel electrophoresis.

The properties of this plasmid are shown in Table 5.

The strain of *E.coli* CSH50/pLC31 is deposited in the DSM under the Accession No. 2718.

TABLE 5

| Properties of miniR1-λP$_R$ plasmids as cloning vectors | | | | | | |
|---|---|---|---|---|---|---|
| Plasmid | Relevant pheno-type | Unique restriction site(s) | Screening (insertional inactivation) | Copy no. per cell | | Stable inheritance |
| | | | | 30°C | 42°C | |
| pOU51 | Km,λ | EcoRI | None | 25 | >1000 | Yes |
| pOU53 | λ | EcoRI | None | 25 | >1000 | Yes |
| pOU56 | Ap,λ | EcoRI, BamHI | None | 25 | >1000 | Yes |
| pOU57 | Ap,λ | EcoRI, BamHI | None | 25 | >1000 | Yes |
| pOU71 | Ap,λ | EcoRI, BamHI | None | 3 | >1000 | No |
| pOU73 | Ap,λ | BamHI | None | 1 | >1000 | No |
| pOU75 | Ap,λ | BamHI | None | 3 | >1000 | No |
| pOU106 | Ap,λ | BamHI | None | 3 | >1000 | No |
| pOU79 | Ap,λ,Lac($^+$) | EcoRI, BamHI | Lac$^-$ (EcoRI) | 3 | >1000 | No |
| pOU82 | Ap,λ,Lac$^+$ | EcoRI, BamHI | None | 3 | >1000 | No |
| pOU91 | Ap,λ,Lac$^+$, Par$^+$ | EcoRI, BamHI | None | 3 | >1000 | Yes |
| pOU101 | Ap, Cm, λ | EcoRI | Cm$^s$ (EcoRI) | 3 | >1000 | No |
| pOU130 | Ap,λ,Lac$^+$ | EcoRI | None | 3 | >1000 | Yes |
| pLC31 | Ap,RecA$^+$,λ | EcoRI | RecA$^-$ | 3 | >1000 | No |
| pOU110 | Ap | EcoRI, BamHI | None | a) | | No |
| a) copy number not temperature-dependent | | | | | | |

BIBLIOGRAPHY

1. Published European Patent Application No. 78101877.5

2. Molin et al., J. Bact. *138*, 1979, pp. 70-79.

3. Molin et al., Microbiology, 1981, pp. 408-11.

4. Rosen et al., Mol. Gen. Genet. *179*, 1980, pp. 527-37.

5. Light and Molin, Mol. Gen. Genet. *184*, 1981, pp. 56-61.

6. Stougaard et al., Proc. Natl. Acad. Sci. USA *78*, 1981, pp. 6008-12.

7. Molin et al., Mol. Gen. Genet. *181*, 1981, pp. 123-30.

8. Stougaard et al., the EMBO Journal *1*, 1982, pp. 323-28.

9. Sussman and Jacob, Compt. Rend. Acad. Sci. *254*, 1962, p. 1517.

10. Sninsky et al., Gene *16*, 1981, p. 275.

11. Uhlin et al., Gene *6*, 1979, pp. 91-106.

12. J. Miller: *Experiments in Molecular Genetics*, Cold Spring Harbor Laboratory, Cold Spring Harbor, New York, 1972.

13. Davis, Botstein and Roth: *A Manual for Genetic Engineering*; *Advanced Bacterial Genetics*, Cold Spring Harbor, New York, 1980.

14. Bertani, J. Bact. 62, 1954, p. 293.

15. Bolivar et al., Gene 2, 1977, p. 95.

16. An & Friesen, J. Bact. *140*, 1979, pp. 400-407.

17. Casadaban et al., J. Bact. *143*, 1980, p. 971.

18. Dempsey & Willetts, J. Bact. *126*, 1976, p. 166.

19. Nordström, Molin, Aagaard-Hansen, Plasmid *4*, 1980, pp. 215-17.

20. Stougaard & Molin, Anal. Biochem. *118*, 1981, p. 191.

21. Nordström et al., Plasmid *4*, 1980, p.322.

22. Valentin-Hansen et al., the EMBO Journal *1*, 1982, p. 317.

23. Messing et al., Nucleic Acids Res. *9*, 1981, p. 309.

24. Uhlin & Clark, J. Bact. *148*, 1981, pp. 386-90.

**Claims**
**Claims for the following Contracting States : BE, CH, DE, FR, GB, IT, LI, LU, NL, SE**

EP 0 109 150 B1

1. A plasmid useful as a cloning vector having a size in the range of about $2.5\text{-}15.0 \times 10^6$ daltons which plasmid carries:

(a) an inserted regulatable promoter at a position and in an orientation permitting transcription from said promoter into the replication region;

(b) said replication region comprising a gene coding for a gene product that is positively needed for replication, increased transcription from the regulatable promoter leading to an increased level of the gene product which is positively needed for replication;

(c) said increased level resulting in a plasmid replication rate which is higher than the host cell growth rate leading to an increased plasmid copy number which, usually after 4-5 cell doublings, will have increased by a factor of 25-1000.

2. A plasmid according to claim 1 in which the promoter is one which is inherently regulatable due to a particular DNA structure within the region of the promoter.

3. A plasmid according to claim 1 in which the promoter is controllable by means of a regulating factor.

4. A plasmid according to claim 3 in which the regulating factor is one that exerts positive control.

5. A plasmid according to claim 3 in which the regulating factor is one that exerts negative control.

6. A plasmid according to claim 1 in which increased transcription from the regulatable promoter leading to an increased level of the gene product which is positively needed for replication is caused by derepression of said promoter.

7. A plasmid according to any of the preceding claims in which the promoter is one which is chemically regulatable.

8. A plasmid according to claim 7 in which the promoter is a lac promoter, trp promoter or deo promoter.

9. A plasmid according to any of claims 1-6 in which the activity of the promoter is temperature-dependent or controlled by a temperature-sensitive regulating factor.

10. A plasmid according to claim 9 in which the promoter is a λ promoter, and in which the gene for a temperature-sensitive λ cI repressor controlling transcription from said promoter is also present.

11. A plasmid according to claim 10 in which the λ promoter is either $\lambda P_R$ or $\lambda P_L$.

12. A plasmid according to claim 11 in which the λ promoter is $\lambda P_R$.

13. A plasmid according to any of the preceding claims in which transcription from said regulatable promoter causes the plasmid to show a constant, low copy number at low temperatures and a substantially increased copy number at higher temperatures.

14. A plasmid according to claim 13 in which transcription from said regulatable promoter causes the plasmid to show a constant, low copy number at a temperature of about 30°C and a substantially increased copy number at a temperature in the range of about 36-42°C.

15. A plasmid according to claim 14 showing an increased plasmid copy number at temperatures above about 39°C.

16. A plasmid according to any of the preceding claims from which part of one native replication regulatory gene has been deleted and replaced by the regulatable promoter.

17. A plasmid according to claim 16 which, when host microorganisms containing the plasmid are grown under conditions securing a constant, low plasmid copy number, has a copy number in the range of 20-40, preferably 20-30 and particularly 20-25 copies per cell, and which, when the host microorganisms are grown under different conditions leading to a substantially increased plasmid copy number, has a copy number in the range of at least about 500-1000 copies per cell.

25

**18.** A plasmid according to claim 17 which has a plasmid copy number in the range of about 20-40, preferably 20-30 and particularly 20-25 copies per cell at one temperature, such as a temperature of about 30°C, and an increased plasmid copy number in the range of at least about 500-1000 copies per cell at a higher temperature, such as a temperature of about 42°C.

**19.** A plasmid according to claim 16-18 which is an R1-type plasmid from which the copB gene has been deleted and replaced by the regulatable promoter.

**20.** A plasmid according to any of claims 16-19 which, when host microorganisms containing the plasmid are grown under conditions securing a constant, low plasmid copy number, has a copy number in the range of about 3-5 copies per cell, and which, when the host microorganisms are grown under different conditions leading to a substantially increased plasmid copy number, has a copy number in the range of at least about 500-1000 copies per cell.

**21.** A plasmid according to claim 20 which has a plasmid copy number in the range of about 3-5 copies per cell at one temperature, such as a temperature of about 30°C, and an increased plasmid copy number in the range of at least about 500-1000 copies per cell at a higher temperature, such as a temperature of about 42°C.

**22.** A plasmid according to any of claims 1-15 in which a regulatable promoter has been inserted upstream of the native replication control gene(s) of the plasmid.

**23.** A plasmid according to claim 22 which, when host microorganisms containing the plasmid are grown under conditions securing a constant, low plasmid copy number, has a copy number in the range of about 3-5 copies per cell, and which, when the host microorganisms are grown under different conditions leading to a substantially increased plasmid copy number, has a copy number in the range of at least about 500-1000 copies per cell.

**24.** A plasmid according to claim 23 which has a plasmid copy number in the range of about 3-5 copies per cell at one temperature, such as a temperature of about 30°C, and an increased plasmid copy number in the range of at least about 500-1000 copies per cell at a higher temperature, such as a temperature of about 42°C.

**25.** A plasmid according to any of claims 22-24 which is an R1-type plasmid in which the regulatable promoter has been inserted upstream of the copB and copA genes.

**26.** A plasmid according to any of claims 22-25 which, when host microorganisms containing the plasmid are grown under conditions securing a constant, low plasmid copy number, has a copy number in the range of about 0.5-1 copy per cell, and which, when the host microorganisms are grown under different conditions leading to a substantially increased plasmid copy number, has a copy number in the range of at least about 500-1000 copies per cell.

**27.** A plasmid according to claim 26 which has a plasmid copy number in the range of about 0.5-1 copy per cell at one temperature, such as a temperature of about 30°C, and an increased plasmid copy number in the range of at least about 500-1000 copies per cell at a higher temperature, such as a temperature of about 42°C.

**28.** A plasmid according to any of claims 1-15 which carries a regulatable promoter in the replication region from which part of one native replication regulatory gene has been deleted, and in which the native replication regulatory gene has been inserted into a region where it is not naturally located.

**29.** A plasmid according to claim 28 which, when host microorganisms containing the plasmid are grown under conditions securing a constant, low plasmid copy number, has a copy number in the range of about 0.5-1 copy per cell, and which, when the host microorganisms are grown under different conditions leading to a substantially increased plasmid copy number, has a copy number in the range of at least about 500-1000 copies per cell.

**30.** A plasmid according to claim 29 which has a plasmid copy number in the range of about 0.5-1 copy per cell at one temperature, such as a temperature of about 30°C, and an increased plasmid copy number in the range of at least about 500-1000 copies per cell at a higher temperature, such as a temperature of about 42°C.

**31.** A plasmid according to any of claims 28-30 which is an R1-type plasmid in which the copB gene has been inserted at the EcoR1 site outside the replication region of the plasmid.

**32.** A plasmid according to any of the preceding claims in which, in addition to the first regulatable promoter regulating transcription from the replication region, a second promoter has been inserted in a manner permitting the insertion of a structural gene the expression of which is controlled from said second promoter.

**33.** A plasmid according to claim 32 in which the second promoter regulating the expression of said gene is controllable.

**34.** A plasmid according to claim 32 or 33 in which said second inserted promoter controlling the expression of the structural gene is the $\lambda P_L$ promoter.

**35.** A plasmid according to any of claims 32-34 having a constant, low plasmid copy number and very little or no gene expression at about 30°C and having a substantially increased plasmid copy number, and gene expression at a temperature in the range of about 36-42°C.

**36.** A plasmid according to claim 35 having an increased plasmid copy number and a very high gene expression at temperatures above 39°C.

**37.** A plasmid according to any of the preceding claims which carries a marker useful for the identification and/or selection of cells containing the plasmid.

**38.** A plasmid according to claim 37 in which the marker is a gene mediating antibiotic resistance to the host microorganism.

**39.** A plasmid according to any of the preceding claims which carries a gene allowing gene fusion upon insertion of another gene from which fusion it is possible to detect expression.

**40.** A plasmid according to any of the preceding claims, characterized in that it is a miniplasmid.

**41.** A plasmid according to any of the preceding claims which additionally carries a gene or genes not naturally related to the plasmid.

**42.** A microorganism in which a plasmid according to claims 1-41 has been inserted.

**43.** A microorganism according to claim 42 which is a bacterium.

**44.** A microorganism according to claim 43 which is a gram-negative and mesophilic bacterium.

**45.** A microorganism according to claim 44 which is an Escherichia coli.

**46.** A method of constructing a plasmid according to claims 1-41 comprising inserting a regulatable promoter into a plasmid in such a way that transcription from said promoter regulates replication and identifying the plasmid so treated by screening for those plasmids which show a substantially increased plasmid copy number when transcription from said promoter is increased.

**47.** A method according to claim 46 comprising deleting part of one native replication regulatory gene and inserting a DNA fragment including the regulatable promoter at the site of the partially deleted native replication regulatory gene.

**48.** A method according to claim 47 comprising deleting the copB gene from the plasmid by cleaving with the restriction endonuclease BglII and inserting a BglII fragment including the regulated promoter at this site.

**49.** A method according to claim 46 comprising inserting the regulatable promoter into the plasmid upstream of the native replication control gene(s).

**50.** A method according to claim 49 comprising inserting the regulatable promoter at the BglII site upstream of the copB and copA genes.

**51.** A method according to claim 46 comprising, by using the plasmid with the partially deleted native replication regulatory gene as a starting material, reinserting the native replication regulatory gene at its original site to construct a plasmid in which all native replication control genes are present.

**52.** A method according to claim 51 comprising, by using the plasmid with the partially deleted copB gene as a starting material, reinserting the copB gene at the BglII site to construct a plasmid in which both the copB and the copA genes are present.

**53.** A method according to claim 46 comprising, by using the plasmid with the partially deleted native replication regulatory gene as a starting material, inserting one native replication regulatory gene in a region of the plasmid where it is not naturally located.

**54.** A method according to claim 53 comprising, by using the plasmid with the partially deleted copB gene as a starting material, inserting the copB gene at the EcoRI site outside the replication region of the plasmid.

**55.** A method of constructing a plasmid according to claim 32 comprising, in addition to the insertion of the regulatable promoter, the insertion of said second promoter at an appropriate restriction site permitting the insertion of a structural gene the expression of which is regulatable from said second promoter.

**56.** A method of producing a gene product of plasmid DNA which comprises cultivating microorganisms carrying a plasmid according to claims 1-41 comprising at least a period of cultivation under conditions securing increased transcription from said regulatable promoter resulting in a substantially increased plasmid copy number and harvesting, from the microbial culture, a gene product of the plasmid.

**57.** A method according to claim 56 comprising cultivating the host microorganism, to which a plasmid according to claims 1-41 has been transformed, under conditions resulting in a constant, low plasmid copy number during the seeding and multiplication stages until the desired number of cells has been obtained and then cultivating the host microorganism under conditions leading to a substantially increased plasmid copy number.

**58.** A method according to claim 56 or 57 in which, after a period of cultivation under conditions where any transcription from the promoter does not significantly influence the copy number of the plasmid, a substance inducing promoter activity is added to the culture medium in an amount leading to a substantially increased plasmid copy number.

**59.** A method according to claim 58 in which, after maintaining the conditions leading to a substantially increased or uncontrolled plasmid copy number for a period of time sufficient to obtain a substantial amplification of the plasmid, but sufficiently brief to avoid any substantial impairment of the microbial culture, the substance inducing promoter activity is removed from the culture medium leading to a slow decrease in plasmid copy number to the pre-induction level.

**60.** A method according to claim 56 or 57 in which, after a period of cultivation in the presence of a substance substantially inhibiting promoter activity, the concentration of the substance is reduced to an extent leading to activation of the promoter and a substantially increased plasmid copy number.

**61.** A method according to claim 60 in which, after maintaining the conditions leading to a substantially increased or uncontrolled copy number for a period sufficient to obtain a substantial amplification of the plasmid, but sufficiently brief to avoid any substantial impairment of the microbial culture, the substance inhibiting promoter activity is added in an amount leading to a slow decrease in plasmid copy number

28

EP 0 109 150 B1

to pre-induction level.

62. A method according to claim 56 or 57 wherein the conditions under which the host microorganism is cultivated comprise at least a period of cultivation at or near a temperature at which the plasmid copy number is substantially increased.

63. A method according to claim 62 wherein the cultivation at the temperature at which the plasmid copy number is substantially increased is continued until the growth of the host microorganism is inhibited by the formation of plasmid DNA or gene products from the plasmids.

64. A method according to claim 62 wherein production scale cultivation is performed continuously at a temperature approaching the temperature at which the plasmid copy number is substantially increased so as to permit the survival and continual growth of the host microorganism concomitantly with formation of increased amounts of a gene product of the plasmid, and the gene product is then continuously or intermittently harvested from the culture.

65. A method according to claim 62 wherein, after the propagation of the microbial culture to reach production size, the temperature is shifted to a temperature at or near the temperature at which the plasmid copy number is substantially increased, and this temperature is maintained for a period of time sufficient to obtain a substantial amplification of the plasmid, but sufficiently brief to avoid any substantial impairment of the microbial culture, after which the temperature is once more shifted to a temperature securing a low, constant plasmid copy number causing a slow decrease in the copy number of the plasmid until it reaches the initial level.

66. A method according to any of claims 62-65 wherein the temperature at which the plasmid copy number is substantially increased is higher than the temperature at which the plasmid has a constant, low copy number.

67. A method according to claim 66 wherein the temperature at which the plasmid has a constant, low copy number is a temperature of about 30°C, and the temperature at which the plasmid has a substantially increased copy number is in the range of about 36-42°C.

68. A method for preparing a plasmid according to any of claims 1-41 comprising inserting a DNA fragment carrying a marker useful for the identification and/or selection of cells containing the plasmid.

69. A method according to claim 68 in which the marker is a gene mediating antibiotic resistance to the host microorganism.

**Claims for the following Contracting State : AT**

1. A method of preparing a plasmid useful as a cloning vector and having a size in the range of about 2.5-15.0 x $10^6$ daltons by inserting into a plasmid:
   (a) a regulatable promoter at a position and in an orientation permitting transcription from said promoter into the replication region;
   (b) said replication region comprising a gene coding for a gene product that is positively needed for replication, increased transcription from the regulatable promoter leading to an increased level of the gene product which is positively needed for replication;
   (c) said increased level resulting in a plasmid replication rate which is higher than the host cell growth rate leading to an increased plasmid copy number which, usually after 4-5 cell doublings, will have increased by a factor of 25-1000.

2. A method according to claim 1 in which the promoter is one which is inherently regulatable due to a particular DNA structure within the region of the promoter.

3. A method according to claim 1 in which the promoter is controllable by means of a regulating factor.

4. A method according to claim 3 in which the regulating factor is one that exerts positive control.

29

**5.** A method according to claim 3 in which the regulating factor is one that exerts negative control.

**6.** A method according to claim 1 in which increased transcription from the regulatable promoter leading to an increased level of the gene product which is positively needed for replication is caused by derepression of said promoter.

**7.** A method according to any of the preceding claims in which the promoter is one which is chemically regulatable.

**8.** A method according to claim 7 in which the promoter is a lac promoter, trp promoter or deo promoter.

**9.** A method according to any of claims 1-6 in which the activity of the promoter is temperature-dependent or controlled by a temperature-sensitive regulating factor.

**10.** A method according to claim 9 in which the promoter is a $\lambda$ promoter, and in which the gene for a temperature-sensitive $\lambda$ cI repressor controlling transcription from said promoter is also present.

**11.** A method according to claim 10 in which the $\lambda$ promoter is either $\lambda P_R$ or $\lambda P_L$.

**12.** A method according to claim 11 in which the $\lambda$ promoter is $\lambda P_R$.

**13.** A method according to any of the preceding claims in which transcription from said regulatable promoter causes the plasmid to show a constant, low copy number at low temperatures and a substantially increased copy number at higher temperatures.

**14.** A method according to claim 13 in which transcription from said regulatable promoter causes the plasmid to show a constant, low copy number at a temperature of about 30°C and a substantially increased copy number at a temperature in the range of about 36-42°C.

**15.** A method according to claim 14 showing an increased plasmid copy number at temperatures above about 39°C.

**16.** A method according to any of the preceding claims in which part of one native replication regulatory gene has been deleted and replaced by the regulatable promoter.

**17.** A method according to claim 16 in which, when host microorganisms containing the plasmid are grown under conditions securing a constant, low plasmid copy number, the plasmid has a copy number in the range of 20-40, preferably 20-30 and particularly 20-25 copies per cell, and in which, when the host microorganisms are grown under different conditions leading to a substantially increased plasmid copy number, the plasmid has a copy number in the range of at least about 500-1000 copies per cell.

**18.** A method according to claim 17 which has a plasmid copy number in the range of about 20-40, preferably 20-30 and particularly 20-25 copies per cell at one temperature, such as a temperature of about 30°C, and an increased plasmid copy number in the range of at least about 500-1000 copies per cell at a higher temperature, such as a temperature of about 42°C.

**19.** A method according to claim 16-18 in which the plasmid is an R1-type plasmid from which the copB gene has been deleted and replaced by the regulatable promoter.

**20.** A method according to any of claims 16-19 in which, when host microorganisms containing the plasmid are grown under conditions securing a constant, low plasmid copy number, the plasmid has a copy number in the range of about 3-5 copies per cell, and in which, when the host microorganisms are grown under different conditions leading to a substantially increased plasmid copy number, the plasmid has a copy number in the range of at least about 500-1000 copies per cell.

**21.** A method according to claim 20 which has a plasmid copy number in the range of about 3-5 copies per cell at one temperature, such as a temperature of about 30°C, and an increased plasmid copy number in the range of at least about 500-1000 copies per cell at a higher temperature, such as a temperature of about 42°C.

22. A method according to any of claims 1-15 in which a regulatable promoter has been inserted upstream of the native replication control gene(s) of the plasmid.

23. A method according to claim 22 in which, when host microorganisms containing the plasmid are grown under conditions securing a constant, low plasmid copy number, the plasmid has a copy number in the range of about 3-5 copies per cell, and in which, when the host microorganisms are grown under different conditions leading to a substantially increased plasmid copy number, the plasmid has a copy number in the range of at least about 500-1000 copies per cell.

24. A method according to claim 23 which has a plasmid copy number in the range of about 3-5 copies per cell at one temperature, such as a temperature of about 30°C, and an increased plasmid copy number in the range of at least about 500-1000 copies per cell at a higher temperature, such as a temperature of about 42°C.

25. A method according to any of claims 22-24 in which the plasmid is an R1-type plasmid in which the regulatable promoter has been inserted upstream of the copB and copA genes.

26. A method according to any of claims 22-25 in which, when host microorganisms containing the plasmid are grown under conditions securing a constant, low plasmid copy number, the plasmid has a copy number in the range of about 0.5-1 copy per cell, and in which, when the host microorganisms are grown under different conditions leading to a substantially increased plasmid copy number, the plasmid has a copy number in the range of at least about 500-1000 copies per cell.

27. A method according to claim 26 which has a plasmid copy number in the range of about 0.5-1 copy per cell at one temperature, such as a temperature of about 30°C, and an increased plasmid copy number in the range of at least about 500-1000 copies per cell at a higher temperature, such as a temperature of about 42°C.

28. A method according to any of claims 1-15 in which the plasmid carries a regulatable promoter in the replication region from which part of one native replication regulatory gene has been deleted, and in which the native replication regulatory gene has been inserted into a region where it is not naturally located.

29. A method according to claim 28 in which, when host microorganisms containing the plasmid are grown under conditions securing a constant, low plasmid copy number, the plasmid has a copy number in the range of about 0.5-1 copy per cell, and in which, when the host microorganisms are grown under different conditions leading to a substantially increased plasmid copy number, the plasmid has a copy number in the range of at least about 500-1000 copies per cell.

30. A method according to claim 29 which has a plasmid copy number in the range of about 0.5-1 copy per cell at one temperature, such as a temperature of about 30°C, and an increased plasmid copy number in the range of at least about 500-1000 copies per cell at a higher temperature, such as a temperature of about 42°C.

31. A method according to any of claims 28-30 in which the plasmid is an R1-type plasmid in which the copB gene has been inserted at the EcoR1 site outside the replication region of the plasmid.

32. A method according to any of the preceding claims in which, in addition to the first regulatable promoter regulating transcription from the replication region, a second promoter has been inserted in a manner permitting the insertion of a structural gene the expression of which is controlled from said second promoter.

33. A method according to claim 32 in which the second promoter regulating the expression of said gene is controllable.

34. A method according to claim 32 or 33 in which said second inserted promoter controlling the

31

expression of the structural gene is the $\lambda P_L$ promoter.

35. A method according to any of claims 32-34 having a constant, low plasmid copy number and very little or no gene expression at about 30°C and having a substantially increased plasmid copy number, and gene expression at a temperature in the range of about 36-42°C.

36. A method according to claim 35 having an increased plasmid copy number and a very high gene expression at temperatures above 39°C.

37. A method according to any of the preceding claims in which the plasmid carries a marker useful for the identification and/or selection of cells containing the plasmid.

38. A method according to claim 37 in which the marker is a gene mediating antibiotic resistance to the host microorganism.

39. A method according to any of the preceding claims in which the plasmid carries a gene allowing gene fusion upon insertion of another gene from which fusion it is possible to detect expression.

40. A method according to any of the preceding claims, characterized in that the plasmid is a miniplasmid.

41. A method according to any of the preceding claims in which the plasmid additionally carries a gene or genes not naturally related to the plasmid.

42. A method of preparing a microorganism by inserting a plasmid according to claims 1-41.

43. A method according to claim 42 in which the microorganism is a bacterium.

44. A method according to claim 43 in which the microorganism is a gram-negative and mesophilic bacterium.

45. A method according to claim 44 in which the microorganism is an Escherichia coli.

46. A method of constructing a plasmid comprising inserting a regulatable promoter into a plasmid in such a way that transcription from said promoter regulates replication and identifying the plasmid so treated by screening for those plasmids which show a substantially increased plasmid copy number when transcription from said promoter is increased.

47. A method according to claim 46 comprising deleting part of one native replication regulatory gene and inserting a DNA fragment including the regulatable promoter at the site of the partially deleted native replication regulatory gene.

48. A method according to claim 47 comprising deleting the copB gene from the plasmid by cleaving with the restriction endonuclease BglII and inserting a BglII fragment including the regulated promoter at this site.

49. A method according to claim 46 comprising inserting the regulatable promoter into the plasmid upstream of the native replication control gene(s).

50. A method according to claim 49 comprising inserting the regulatable promoter at the BglII site upstream of the copB and copA genes.

51. A method according to claim 46 comprising, by using the plasmid with the partially deleted native replication regulatory gene as a starting material, reinserting the native replication regulatory gene at its original site to construct a plasmid in which all native replication control genes are present.

52. A method according to claim 51 comprising, by using the plasmid with the partially deleted copB gene as a starting material, reinserting the copB gene at the BglII site to construct a plasmid in which both the copB and the copA genes are present.

**53.** A method according to claim 46 comprising, by using the plasmid with the partially deleted native replication regulatory gene as a starting material, inserting one native replication regulatory gene in a region of the plasmid where it is not naturally located.

**54.** A method according to claim 53 comprising, by using the plasmid with the partially deleted copB gene as a starting material, inserting the copB gene at the EcoRI site outside the replication region of the plasmid.

**55.** A method of constructing a plasmid according to claim 32 comprising, in addition to the insertion of the regulatable promoter, the insertion of said second promoter at an appropriate restriction site permitting the insertion of a structural gene the expression of which is regulatable from said second promoter.

**56.** A method of producing a gene product of plasmid DNA which comprises cultivating microorganisms carrying a plasmid according to claims 1-41 comprising at least a period of cultivation under conditions securing increased transcription from said regulatable promoter resulting in a substantially increased plasmid copy number and harvesting, from the microbial culture, a gene product of the plasmid.

**57.** A method according to claim 56 comprising cultivating the host microorganism, to which a plasmid according to claims 1-41 has been transformed, under conditions resulting in a constant, low plasmid copy number during the seeding and multiplication stages until the desired number of cells has been obtained and then cultivating the host microorganism under conditions leading to a substantially increased plasmid copy number.

**58.** A method according to claim 56 or 57 in which, after a period of cultivation under conditions where any transcription from the promoter does not significantly influence the copy number of the plasmid, a substance inducing promoter activity is added to the culture medium in an amount leading to a substantially increased plasmid copy number.

**59.** A method according to claim 58 in which, after maintaining the conditions leading to a substantially increased or uncontrolled plasmid copy number for a period of time sufficient to obtain a substantial amplification of the plasmid, but sufficiently brief to avoid any substantial impairment of the microbial culture, the substance inducing promoter activity is removed from the culture medium leading to a slow decrease in plasmid copy number to the pre-induction level.

**60.** A method according to claim 56 or 57 in which, after a period of cultivation in the presence of a substance substantially inhibiting promoter activity, the concentration of the substance is reduced to an extent leading to activation of the promoter and a substantially increased plasmid copy number.

**61.** A method according to claim 60 in which, after maintaining the conditions leading to a substantially increased or uncontrolled copy number for a period sufficient to obtain a substantial amplification of the plasmid, but sufficiently brief to avoid any substantial impairment of the microbial culture, the substance inhibiting promoter activity is added in an amount leading to a slow decrease in plasmid copy number to pre-induction level.

**62.** A method according to claim 56 or 57 wherein the conditions under which the host microorganism is cultivated comprise at least a period of cultivation at or near a temperature at which the plasmid copy number is substantially increased.

**63.** A method according to claim 62 wherein the cultivation at the temperature at which the plasmid copy number is substantially increased is continued until the growth of the host microorganism is inhibited by the formation of plasmid DNA or gene products from the plasmids.

**64.** A method according to claim 62 wherein production scale cultivation is performed continuously at a temperature approaching the temperature at which the plasmid copy number is substantially increased so as to permit the survival and continual growth of the host microorganism concomitantly with formation of increased amounts of a gene product of the plasmid, and the gene product is then continuously or intermittently harvested from the culture.

**65.** A method according to claim 62 wherein, after the propagation of the microbial culture to reach production size, the temperature is shifted to a temperature at or near the temperature at which the plasmid copy number is substantially increased, and this temperature is maintained for a period of time sufficient to obtain a substantial amplification of the plasmid, but sufficiently brief to avoid any substantial impairment of the microbial culture, after which the temperature is once more shifted to a temperature securing a low, constant plasmid copy number causing a slow decrease in the copy number of the plasmid until it reaches the initial level.

**66.** A method according to any of claims 62-65 wherein the temperature at which the plasmid copy number is substantially increased is higher than the temperature at which the plasmid has a constant, low copy number.

**67.** A method according to claim 66 wherein the temperature at which the plasmid has a constant, low copy number is a temperature of about 30°C, and the temperature at which the plasmid has a substantially increased copy number is in the range of about 36-42°C.

**68.** A method for preparing a plasmid according to any of claims 1-41 comprising inserting a DNA fragment carrying a marker useful for the identification and/or selection of cells containing the plasmid.

**69.** A method according to claim 68 in which the marker is a gene mediating antibiotic resistance to the host microorganism.

**Revendications**
**Revendications pour les Etats contractants suivants : BE, CH, DE, FR, GB, IT, LI, LU, NL, SE**

**1.** Plasmide utile comme vecteur de clonage et ayant une taille située dans un intervalle d'environ 2,5-15,0 x 10$^6$ dalton, lequel plasmide porte:
   (a) un promoteur régulable inséré en une position et en une orientation permettant la transcription à partir dudit promoteur dans la région de réplication;
   (b) ladite région de réplication comprenant un gène codant pour un produit de gène qui est positivement nécessaire pour la réplication, l'augmentation de transcription à partir du promoteur régulable aboutissant à une augmentation du niveau du produit de gène qui est positivement nécessaire pour la réplication ;
   (c) ledit niveau augmenté aboutissant à une vitesse de réplication du plasmide qui est supérieure à la vitesse de croissance de la cellule-hôte, aboutissant à un nombre accru de copies de plasmide qui, généralement au bout de quatre à cinq doublages cellulaires, aura augmenté d'un facteur de 25-1000.

**2.** Plasmide selon la revendication 1, dans lequel le promoteur est un promoteur qui est régulable de façon inhérente du fait d'une structure d'ADN particulière dans la région du promoteur.

**3.** Plasmide selon la revendication 1, dans lequel le promoteur est contrôlable au moyen d'un facteur de régulation.

**4.** Plasmide selon la revendication 3, dans lequel le facteur de régulation est un facteur qui exerce un contrôle positif.

**5.** Plasmide selon la revendication 3, dans lequel le facteur de régulation est un facteur qui exerce un contrôle négatif.

**6.** Plasmide selon la revendication 1, dans lequel la transcription accrue à partir du promoteur régulable aboutissant à un niveau accru de produit de gène qui est positivement nécessaire pour la réplication est provoquée par la dérépression dudit promoteur.

**7.** Plasmide selon l'une quelconque des revendications précédentes, dans lequel le promoteur est un promoteur qui est chimiquement régulable.

**8.** Plasmide selon la revendication 7, dans lequel le promoteur est un promoteur lac, un promoteur trp ou un promoteur deo.

9. Plasmide selon l'une quelconque des revendications 1-6, dans lequel l'activité du promoteur dépend de la température ou est contrôlée par un facteur de régulation sensible à la température.

10. Plasmide selon la revendication 9, dans lequel le promoteur est un promoteur $\lambda$, et dans lequel le gène d'un répresseur $\lambda$cI sensible à la température contrôlant la transcription à partir dudit promoteur est également présent.

11. Plasmide selon la revendication 10, dans lequel le promoteur $\lambda$ est soit $\lambda P_R$, soit $\lambda P_L$.

12. Plasmide selon la revendication 11, dans lequel le promoteur $\lambda$ est $\lambda P_R$.

13. Plasmide selon l'une quelconque des revendications précédentes, dans lequel la transcription à partir dudit promoteur régulable aboutit à ce que le plasmide présente un nombre faible et constant de copies à faible température et un nombre de copies sensiblement accru à des températures plus élevées.

14. Plasmide selon la revendication 13, dans lequel la transcription à partir dudit promoteur régulable fait présenter au plasmide un nombre faible et constant de copies à une température d'environ 30°C et un nombre sensiblement accru de copies à une température située dans un intervalle d'environ 36 - 42°C.

15. Plasmide selon la revendication 14, présentant un nombre accru de copies de plasmides à des températures supérieures à environ 39°C.

16. Plasmide selon l'une quelconque des revendications précédentes, dans lequel une partie d'un gène régulateur de réplication natif a été délétée et remplacée par le promoteur régulable.

17. Plasmide selon la revendication 16, dans lequel, lorsqu'on cultive des microorganismes hôtes contenant le plasmide dans des conditions assurant un nombre faible et constant de copies de plasmide, le plasmide a un nombre de copies situé dans un intervalle de 20 - 40, de préférence de 20 - 30, et en particulier de 20 - 25 copies par cellule, et où lorsqu'on cultive les microorganismes hôtes dans des conditions différentes aboutissant à un nombre sensiblement accru de copies de plasmide, le plasmide a un nombre de copies situé dans un intervalle d'au moins environ 500 - 1000 copies par cellule.

18. Plasmide selon la revendication 17, qui a un nombre de copies de plasmide dans un intervalle d'environ 20 - 40, de préférence 20 - 30 et en particulier 20 - 25 copies par cellule, à une température, comme une température d'environ 30°C, et un nombre accru de copies de plasmide dans un intervalle d'au moins environ 500 - 1000 copies par cellule à une température supérieure, comme une température d'environ 42°C.

19. Plasmide selon les revendications 16 - 18, qui est un plasmide de type R1 dont le gène copB a été délété et remplacé par le promoteur régulable.

20. Plasmide selon l'une quelconque des revendications 16 - 19, dans lequel, lorsqu'on cultive des microorganismes hôtes contenant le plasmide dans des conditions assurant un nombre faible et constant de copies de plasmide, le plasmide a un nombre de copies situé dans un intervalle d'environ 3 - 5 copies par cellule, et où, lorsqu'on cultive les microorganismes hôtes dans des conditions différentes aboutissant à un nombre de copies de plasmide sensiblement accru, le plasmide a un nombre de copies situé dans un intervalle d'au moins environ 500 - 1000 copies par cellule.

21. Plasmide selon la revendication 20 qui a un nombre de copies de plasmide dans un intervalle d'environ 3 - 5 copies par cellule à une température, comme une température d'environ 30°C, et un nombre accru de copies de plasmide dans un intervalle d'au moins environ 500 - 1000 copies par cellule à une température plus élevée, comme une température d'environ 42°C.

22. Plasmide selon l'une quelconque des revendications 1 - 15, dans lequel un promoteur régulable a été inséré en amont du(des) gène(s) de contrôle de réplication natif(s) du plasmide.

**23.** Plasmide selon la revendication 22, dans lequel, lorsqu'on cultive des microorganismes hôtes contenant le plasmide dans des conditions assurant un nombre faible et constant de copies de plasmide, le plasmide a un nombre de copies situé dans un intervalle d'environ 3 - 5 copies par cellule, et où, lorsqu'on cultive les microorganismes hôtes dans des conditions différentes aboutissant à un nombre sensiblement accru de copies de plasmide, le plasmide a un nombre de copies situé dans un intervalle d'au moins environ 500 - 1000 copies par cellule.

**24.** Plasmide selon la revendication 23, qui a un nombre de copies de plasmide situé dans un intervalle d'environ 3 - 5 copies par cellule, à une température, comme une température d'environ 30°C, et un nombre accru de copies de plasmide dans l'intervalle d'au moins environ 500 - 1000 copies par cellule à une température plus élevée, comme une température d'environ 42°C.

**25.** Plasmide selon l'une quelconque des revendications 22 - 24, dans lequel le plasmide est un plasmide de type R1 dans lequel le promoteur régulable a été inséré en amont des gènes copB et copA.

**26.** Plasmide selon l'une quelconque des revendications 22 - 25, dans lequel, lorsqu'on cultive des microorganismes hôtes contenant le plasmide dans des conditions assurant un nombre faible et constant de copies de plasmide, le plasmide a un nombre de copies situé dans un intervalle d'environ 0,5 - 1 copie par cellule, et où, lorsqu'on cultive les microorganismes hôtes dans des conditions différentes aboutissant à un nombre sensiblement accru de copies de plasmide, le plasmide a un nombre de copies situé dans un intervalle d'au moins environ 500 - 1000 copies par cellule.

**27.** Plasmide selon la revendication 26, qui a un nombre de copies de plasmide situé dans un intervalle d'environ 0,5 - 1 copie par cellule à une température, comme une température d'environ 30°C, et un nombre accru de copies de plasmide dans un intervalle d'au moins environ 500 - 1000 copies par cellule à une température plus élevée, comme une température d'environ 42°C.

**28.** Plasmide selon l'une quelconque des revendications 1 - 15, dans lequel le plasmide porte un promoteur régulable dans la région de réplication à partir de laquelle une partie d'un gêne réplicateur de réplication natif a été délétée et où le gène réplicateur de réplication natif a été inséré dans une région où il n'est pas naturellement situé.

**29.** Plasmide selon la revendication 28, dans lequel, lorsqu'on cultive des microorganismes hôtes contenant le plasmide dans des conditions assurant un nombre faible et constant de copies de plasmide, le plasmide a un nombre de copies situé dans un intervalle d'environ 0,5 - 1 copie par cellule et où, lorsqu'on cultive les microorganismes hôtes dans des conditions différentes aboutissant à un nombre sensiblement accru de copies de plasmide, le plasmide a un nombre de copies situé dans un intervalle d'au moins 500 - 1000 copies par cellule.

**30.** Plasmide selon la revendication 29, qui a un nombre de copies de plasmide situé dans un intervalle d'environ 0,5 - 1 copie par cellule à une température, comme une température d'environ 30°C et un nombre accru de copies de plasmide dans un intervalle d'au moins environ 500 - 1000 copies par cellule à une température plus élevée, comme une température d'environ 42°C.

**31.** Plasmide selon l'une quelconque des revendications 28 - 30, dans lequel le plasmide est un plasmide de type R1 dans lequel le gène copB a été inséré au site EcoRI à l'extérieur de la région de réplication du plasmide.

**32.** Plasmide selon l'une quelconque des revendications précédentes dans lequel, outre le premier promoteur régulable régulant la transcription à partir de la région de réplication, un second promoteur a été inséré d'une manière permettant l'insertion d'un gène de structure dont l'expression est contrôlée à partir dudit second promoteur.

**33.** Plasmide selon la revendication 32, dans lequel le second promoteur régulant l'expression dudit gène est contrôlable.

**34.** Plasmide selon la revendication 32 ou 33, dans lequel ledit second promoteur inséré contrôlant l'expression du gène de structure est le promoteur $\lambda P_L$.

**35.** Plasmide selon l'une quelconque des revendications 32 - 34 ayant un nombre faible et constant de copies de plasmide et très peu ou pas d'expression de gène à environ 30°C et ayant un nombre de copies de plasmide et une expression de gène sensiblement accrus à une température située dans un intervalle d'environ 36 - 42°C.

**36.** Plasmide selon la revendication 35, ayant un nombre accru de copies de plasmide et une expression de gène très élevée à des températures supérieures à 39°C.

**37.** Plasmide selon l'une quelconque des revendications précédentes, dans lequel le plasmide porte un marqueur utile pour l'identification et/ou la sélection de cellules contenant le plasmide.

**38.** Plasmide selon la revendication 37, dans lequel le marqueur est un gène jouant un rôle de médiation la résistance antibiotique au microorganisme hôte.

**39.** Plasmide selon l'une quelconque des revendications précédentes dans lequel le plasmide porte un gène permettant la fusion de gènes lors de l'insertion d'un autre gène, à partir de laquelle fusion il est possible de détecter l'expression.

**40.** Plasmide selon l'une quelconque des revendications précédentes, caractérisé en ce qu'il s'agit d'un mini-plasmide.

**41.** Plasmide selon l'une quelconque des revendications précédentes, qui porte en outre un ou plusieurs gènes non naturellement apparentés au plasmide.

**42.** Microorganisme dans lequel un plasmide selon les revendications 1 - 41 a été inséré.

**43.** Microorganisme selon la revendication 42, qui est une bactérie.

**44.** Microorganisme selon la revendication 43, qui est une bactérie gram-négative et mésophile.

**45.** Microorganisme selon la revendication 44, qui est un Escherichia coli.

**46.** Procédé de construction d'un plasmide selon les revendications 1-41, dans lequel on inséré un promoteur régulable dans un plasmide de manière que la transcription à partir dudit promoteur régule la réplication, on identifie le plasmide ainsi traité par criblage par rapport aux plasmides qui présentent un nombre de copies de plasmide sensiblement accru lorsque la transcription à partir dudit promoteur est accrue.

**47.** Procédé selon la revendication 46, dans lequel on délète une partie d'un régulateur de réplication natif et on insère un fragment d'ADN contenant le promoteur régulable au site du gène régulateur de réplication natif partiellement délété.

**48.** Procédé selon la revendication 47, dans lequel on délète le gène copB du plasmide par clivage avec l'endonucléase de restriction BglII et on insère un fragment de BglII incluant le promoteur régulé en ce site.

**49.** Procédé selon la revendication 46, dans lequel on insère le promoteur régulable dans le plasmide en amont du(des) gène(s) de contrôle de réplication natif(s).

**50.** Procédé selon la revendication 49, dans lequel on insère le promoteur régulable au site BglII en amont des gènes copB et copA.

**51.** Procédé selon la revendication 46, dans lequel, en utilisant le plasmide avec le gène régulateur de réplication natif partiellement délété comme produit de départ, on réinsère le gène régulateur de réplication natif en son site d'origine pour construire un plasmide dans lequel tous les gènes de contrôle de réplication natifs sont présents.

**52.** Procédé selon la revendication 51, dans lequel, en utilisant le plasmide avec le gène copB partiellement délété comme produit de départ, on réinsère le gène copB au site BglII pour construire un plasmide dans lequel les deux gènes copB et copA sont présents.

**53.** Procédé selon la revendication 46 dans lequel, en utilisant le plasmide avec le gène régulateur de réplication natif partiellement délété comme produit de départ, on insère un gène régulateur de réplication natif dans une région du plasmide où il n'est pas naturellement situé.

**54.** Procédé selon la revendication 53 dans lequel, en utilisant le plasmide avec le gène copB partiellement délété comme produit de départ, on insère le gène copB au site EcoRI à l'extérieur de la région de réplication du plasmide.

**55.** Procédé de construction d'un plasmide selon la revendication 32 dans lequel, outre l'insertion du promoteur régulable, on insère ledit second promoteur en un site de restriction approprié permettant l'insertion d'un gène de structure dont l'expression est régulable à partir dudit second promoteur.

**56.** Procédé de production d'un produit de gène d'ADN de plasmide dans lequel on cultive des microorganismes portant un plasmide selon les revendications 1 - 41, comprenant au moins une période de culture dans des conditions assurant une transcription accrue à partir dudit promoteur régulable, aboutissant à un nombre de copies de plasmide sensiblement accru et on récolte, à partir de la culture microbienne un produit de gène du plasmide.

**57.** Procédé selon la revendication 56, dans lequel on cultive le microorganisme hôte, auquel on a transformé un plasmide selon les revendications 1 - 41, dans des conditions aboutissant à un nombre faible et constant de copies de plasmide pendant les étapes d'ensemencement et de multiplication, jusqu'à ce qu'on ait obtenu le nombre désiré de cellules, puis on cultive le microorganisme hôte dans des conditions aboutissant à un nombre de copies de plasmide sensiblement accru.

**58.** Procédé selon la revendication 56 ou 57 dans lequel, après une période de culture dans des conditions dans lesquelles aucune transcription à partir du promoteur n'influence de manière significative le nombre de copies de plasmide, on ajoute une substance induisant une activité de promoteur au milieu de culture en une quantité aboutissant à un nombre de copies de plasmide sensiblement accru.

**59.** Procédé selon la revendication 58, dans lequel, après avoir maintenu des conditions aboutissant à un nombre sensiblement accru ou non contrôlé de copies de plasmide pendant une durée suffisante pour obtenir une amplification substantielle du plasmide, mais suffisamment brève pour éviter toute détérioration substantielle de la culture microbienne, on retire du milieu de culture la substance induisant l'activité de promoteur, aboutissant à une lente diminution du nombre de copies de plasmide jusqu'au niveau de pré-induction.

**60.** Procédé selon la revendication 56 ou 57 dans lequel, après une période de culture en présence d'une substance inhibant sensiblement l'activité de promoteur, la concentration de la substance est réduite dans une mesure aboutissant à l'activation du promoteur et à un nombre de copies de plasmide sensiblement accru.

**61.** Procédé selon la revendication 60 dans lequel, après maintien des conditions aboutissant à un nombre de copies sensiblement accru ou non contrôlé pendant une période suffisante pour obtenir une amplification substantielle du plasmide, mais suffisamment brève pour éviter toute détérioration substantielle de la culture microbienne, on ajoute la substance inhibant l'activité de promoteur en une quantité aboutissant à une lente diminution du nombre de copies de plasmide au niveau de pré-induction.

**62.** Procédé selon la revendication 56 ou 57, dans lequel les conditions dans lesquelles le microorganisme hôte est cultivé comprennent au moins une période de culture à ou au voisinage d'une température à laquelle le nombre de copies de plasmide est sensiblement accru.

**63.** Procédé selon la revendication 62, dans lequel la culture à la température à laquelle le nombre de copies de plasmide est sensiblement accru est poursuivie jusqu'à ce que la croissance du microorganisme hôte soit inhibée par la formation d'ADN de plasmide ou de produits de gène provenant des plasmides.

**64.** Procédé selon la revendication 62, dans lequel la culture à l'échelle de la production est effectuée de façon continue à une température approchant la température à laquelle le nombre de copies de plasmide est sensiblement accru de manière à permettre la survie et la croissance continue du microorganisme hôte de façon concomitante avec la formation de quantités accrues d'un produit de gène du plasmide, puis le produit de gène est alors recueilli de façon continue ou intermittente à partir de la culture.

**65.** Procédé selon la revendication 62, dans lequel, après propagation de la culture microbienne pour atteindre la taille de production, on fait passer la température à une température égale à ou voisine de la température à laquelle le nombre de copies de plasmide est sensiblement accru, et on maintient cette température pendant une durée suffisante pour obtenir une amplification substantielle du plasmide, mais suffisamment brève pour éviter toute détérioration substantielle de la culture microbienne, après quoi on fait encore une fois passer la température à un niveau assurant un nombre faible et constant de copies de plasmide, provoquant une lente diminution du nombre de copies du plasmide jusqu'à ce qu'il atteigne le niveau initial.

**66.** Procédé selon l'une quelconque des revendications 62 - 65, dans lequel la température à laquelle le nombre de copies de plasmide est sensiblement augmenté est supérieure à la température à laquelle le plasmide a un nombre faible et constant de copies.

**67.** Procédé selon la revendication 66, dans lequel la température à laquelle le plasmide a un nombre faible et constant de copies est une température d'environ 30°C, et la température à laquelle le plasmide a un nombre de copies sensiblement accru est dans un intervalle d'environ 36 - 42°C.

**68.** Procédé de préparation d'un plasmide selon l'une quelconque des revendications 1 - 41, dans lequel on insère un fragment d'ADN portant un marqueur utile pour l'identification et/ou la sélection de cellules contenant le plasmide.

**69.** Procédé selon la revendication 68, dans lequel le marqueur est un gène jouant un rôle de médiateur la résistance aux antibiotiques au microorganisme hôte.

**Revendications pour l'Etat contractant suivant : AT**

**1.** Procédé de préparation d'un plasmide utile comme vecteur de clonage et ayant une taille située dans un intervalle d'environ 2,5-15,0 x $10^6$ dalton, lequel plasmide porte:
(a) un promoteur régulable inséré en une position et en une orientation permettant la transcription à partir dudit promoteur dans la région de réplication;
(b) ladite région de réplication comprenant un gène codant pour un produit de gène qui est positivement nécessaire pour la réplication, l'augmentation de transcription à partir du promoteur régulable aboutissant à une augmentation du niveau du produit de gène qui est positivement nécessaire pour la réplication ;
(c) ledit niveau augmenté aboutissant à une vitesse de réplication du plasmide qui est supérieure à la vitesse de croissance de la cellule-hôte, aboutissant à un nombre accru de copies de plasmide qui, généralement au bout de quatre à cinq doublages cellulaires, aura augmenté d'un facteur de 25-1000.

**2.** Procédé selon la revendication 1, dans lequel le promoteur est un promoteur qui est régulable de façon inhérente du fait d'une structure d'ADN particulière dans la région du promoteur.

**3.** Procédé selon la revendication 1, dans lequel le promoteur est contrôlable au moyen d'un facteur de régulation.

**4.** Procédé selon la revendication 3, dans lequel le facteur de régulation est un facteur qui exerce un contrôle positif.

**5.** Procédé selon la revendication 3, dans lequel le facteur de régulation est un facteur qui exerce un

EP 0 109 150 B1

contrôle négatif.

**6.** Procédé selon la revendication 1, dans lequel la transcription accrue à partir du promoteur régulable aboutissant à un niveau accru de produit de gène qui est positivement nécessaire pour la réplication est provoquée par la dérépression dudit promoteur.

**7.** Procédé selon l'une quelconque des revendications précédentes, dans lequel le promoteur est un promoteur qui est chimiquement régulable.

**8.** Procédé selon la revendication 7, dans lequel le promoteur est un promoteur lac, un promoteur trp ou un promoteur deo.

**9.** Procédé selon l'une quelconque des revendications 1-6, dans lequel l'activité du promoteur dépend de la température ou est contrôlée par un facteur de régulation sensible à la température.

**10.** Procédé selon la revendication 9, dans lequel le promoteur est un promoteur $\lambda$ , et dans lequel le gène d'un répresseur $\lambda$cI sensible à la température contrôlant la transcription à partir dudit promoteur est également présent.

**11.** Procédé selon la revendication 10, dans lequel le promoteur $\lambda$ est soit $\lambda$ $P_R$, soit $\lambda$ $P_L$.

**12.** Procédé selon la revendication 11, dans lequel le promoteur $\lambda$ est $\lambda P_R$.

**13.** Procédé selon l'une quelconque des revendications précédentes, dans lequel la transcription à partir dudit promoteur régulable aboutit à ce que le plasmide présente un nombre faible et constant de copies à faible température et un nombre de copies sensiblement accru à des températures plus élevées.

**14.** Procédé selon la revendication 13, dans lequel la transcription à partir dudit promoteur régulable fait présenter au plasmide un nombre faible et constant de copies à une température d'environ 30°C et un nombre sensiblement accru de copies à une température située dans un intervalle d'environ 36 - 42°C.

**15.** Procédé selon la revendication 14, présentant un nombre accru de copies de plasmides à des températures supérieures à environ 39°C.

**16.** Procédé selon l'une quelconque des revendications précédentes, dans lequel une partie d'un gène régulateur de réplication natif a été délétée et remplacée par le promoteur régulable.

**17.** Procédé selon la revendication 16, dans lequel, lorsqu'on cultive des microorganismes hôtes contenant le plasmide dans des conditions assurant un nombre faible et constant de copies de plasmide, le plasmide a un nombre de copies situé dans un intervalle de 20 - 40, de préférence de 20 - 30, et en particulier de 20 - 25 copies par cellule, et où lorsqu'on cultive les microorganismes hôtes dans des conditions différentes aboutissant à un nombre sensiblement accru de copies de plasmide, le plasmide a un nombre de copies situé dans un intervalle d'au moins environ 500 - 1000 copies par cellule.

**18.** Procédé selon la revendication 17, qui a un nombre de copies de plasmide dans un intervalle d'environ 20 - 40, de préférence 20 - 30 et en particulier 20 - 25 copies par cellule, à une température, comme une température d'environ 30°C, et un nombre accru de copies de plasmide dans un intervalle d'au moins environ 500 - 1000 copies par cellule à une température supérieure, comme une température d'environ 42°C.

**19.** Procédé selon les revendications 16 - 18, dans lequel le plasmide est un plasmide de type R1 dont le gène copB a été délété et remplacé par le promoteur régulable.

**20.** Procédé selon l'une quelconque des revendications 16 - 19, dans lequel, lorsqu'on cultive des microorganismes hôtes contenant le plasmide dans des conditions assurant un nombre faible et constant de copies de plasmide, le plasmide a un nombre de copies situé dans un intervalle d'environ 3 - 5 copies par cellule, et où, lorsqu'on cultive les microorganismes hôtes dans des conditions

40

différentes aboutissant à un nombre de copies de plasmide sensiblement accru, le plasmide a un nombre de copies situé dans un intervalle d'au moins environ 500 - 1000 copies par cellule.

21. Procédé selon la revendication 20 qui a un nombre de copies de plasmide dans un intervalle d'environ 3 - 5 copies par cellule à une température, comme une température d'environ 30°C, et un nombre accru de copies de plasmide dans un intervalle d'au moins environ 500 - 1000 copies par cellule à une température plus élevée, comme une température d'environ 42°C.

22. Procédé selon l'une quelconque des revendications 1 - 15, dans lequel un promoteur régulable a été inséré en amont du(des) gène(s) de contrôle de réplication natif(s) du plasmide.

23. Procédé selon la revendication 22, dans lequel, lorsqu'on cultive des microorganismes hôtes contenant le plasmide dans des conditions assurant un nombre faible et constant de copies de plasmide, le plasmide a un nombre de copies situé dans un intervalle d'environ 3 - 5 copies par cellule, et où, lorsqu'on cultive les microorganismes hôtes dans des conditions différentes aboutissant à un nombre sensiblement accru de copies de plasmide, le plasmide a un nombre de copies situé dans un intervalle d'au moins environ 500 - 1000 copies par cellule.

24. Procédé selon la revendication 23, qui a un nombre de copies de plasmide situé dans un intervalle d'environ 3 - 5 copies par cellule, à une température, comme une température d'environ 30°C, et un nombre accru de copies de plasmide dans l'intervalle d'au moins environ 500 - 1000 copies par cellule à une température plus élevée, comme une température d'environ 42°C.

25. Procédé selon l'une quelconque des revendications 22 - 24, dans lequel le plasmide est un plasmide de type R1 dans lequel le promoteur régulable a été inséré en amont des gènes copB et copA.

26. Procédé selon l'une quelconque des revendications 22 - 25, dans lequel, lorsqu'on cultive des microorganismes hôtes contenant le plasmide dans des conditions assurant un nombre faible et constant de copies de plasmide, le plasmide a un nombre de copies situé dans un intervalle d'environ 0,5 - 1 copie par cellule, et où, lorsqu'on cultive les microorganismes hôtes dans des conditions différentes aboutissant à un nombre sensiblement accru de copies de plasmide, le plasmide a un nombre de copies situé dans un intervalle d'au moins environ 500 - 1000 copies par cellule.

27. Procédé selon la revendication 26, qui a un nombre de copies de plasmide situé dans un intervalle d'environ 0,5 - 1 copie par cellule à une température, comme une température d'environ 30°C, et un nombre accru de copies de plasmide dans un intervalle d'au moins environ 500 - 1000 copies par cellule à une température plus élevée, comme une température d'environ 42°C.

28. Procédé selon l'une quelconque des revendications 1 - 15, dans lequel le plasmide porte un promoteur régulable dans la région de réplication à partir de laquelle une partie d'un gène réplicateur de réplication natif a été délétée et où le gène réplicateur de réplication natif a été inséré dans une région où il n'est pas naturellement situé.

29. Procédé selon la revendication 28, dans lequel, lorsqu'on cultive des microorganismes hôtes contenant le plasmide dans des conditions assurant un nombre faible et constant de copies de plasmide, le plasmide a un nombre de copies situé dans un intervalle d'environ 0,5 - 1 copie par cellule et où, lorsqu'on cultive les microorganismes hôtes dans des conditions différentes aboutissant à un nombre sensiblement accru de copies de plasmide, le plasmide a un nombre de copies situé dans un intervalle d'au moins 500 - 1000 copies par cellule.

30. Procédé selon la revendication 29, qui a un nombre de copies de plasmide situé dans un intervalle d'environ 0,5 - 1 copie par cellule à une température, comme une température d'environ 30°C et un nombre accrue de copies de plasmide dans un intervalle d'au moins environ 500 - 1000 copies par cellule à une température plus élevée, comme une température d'environ 42°C.

31. Procédé selon l'une quelconque des revendications 28 - 30, dans lequel le plasmide est un plasmide de type R1 dans lequel le gène copB a été inséré au site EcoRI à l'extérieur de la région de réplication du plasmide.

**32.** Procédé selon l'une quelconque des revendications précédentes dans lequel, outre le premier promoteur régulable régulant la transcription à partir de la région de réplication, un second promoteur a été inséré d'une manière permettant l'insertion d'un gène de structure dont l'expression est contrôlée à partir dudit second promoteur.

**33.** Procédé selon la revendication 32, dans lequel le second promoteur régulant l'expression dudit gène est contrôlable.

**34.** Procédé selon la revendication 32 ou 33, dans lequel ledit second promoteur inséré contrôlant l'expression du gène de structure est le promoteur λ $P_L$.

**35.** Procédé selon l'une quelconque des revendications 32 - 34 ayant un nombre faible et constant de copies de plasmide et très peu ou pas d'expression de gène à environ 30°C et ayant un nombre de copies de plasmide et une expression de gène sensiblement accrus à une température située dans un intervalle d'environ 36 - 42°C.

**36.** Procédé selon la revendication 35, ayant un nombre accru de copies de plasmide et une expression de gène très élevée à des températures supérieures à 39°C.

**37.** Procédé selon l'une quelconque des revendications précédentes, dans lequel le plasmide porte un marqueur utile pour l'identification et/ou la sélection de cellules contenant le plasmide.

**38.** Procédé selon la revendication 37, dans lequel le marqueur est un gène jouant un rôle de médiation la résistance antibiotique au microorganisme hôte.

**39.** Procédé selon l'une quelconque des revendications précédentes dans lequel le plasmide porte un gène permettant la fusion de gènes lors de l'insertion d'un autre gène, à partir de laquelle fusion il est possible de détecter l'expression.

**40.** Procédé selon l'une quelconque des revendications précédentes, caractérisé en ce que le plasmide est un mini-plasmide.

**41.** Procédé selon l'une quelconque des revendications précédentes, dans lequel le plasmide porte en outre un ou plusieurs gènes non naturellement apparentés au plasmide.

**42.** Procédé de préparation d'un microorganisme par insertion d'un plasmide selon les revendications 1-41.

**43.** Procédé selon la revendication 42, dans lequel le microorganisme est une bactérie.

**44.** Procédé selon la revendication 43, dans lequel le microorganisme est une bactérie gram-positive et mésophile.

**45.** Procédé selon la revendication 44, dans lequel le microorganisme est un Escherichia coli.

**46.** Procédé de construction d'un plasmide selon les revendications 1-41, dans lequel on inséré un promoteur régulable dans un plasmide de manière que la transcription à partir dudit promoteur régule la réplication, on identifie le plasmide ainsi traité par criblage par rapport aux plasmides qui présentent un nombre de copies de plasmide sensiblement accru lorsque la transcription à partir dudit promoteur est accrue.

**47.** Procédé selon la revendication 46, dans lequel on délète une partie d'un régulateur de réplication natif et on insère un fragment d'ADN contenant le promoteur régulable au site du gène régulateur de réplication natif partiellement délété.

**48.** Procédé selon la revendication 47, dans lequel on délète le gène copB du plasmide par clivage avec l'endonucléase de restriction BglII et on insère un fragment de BglII incluant le promoteur régulé en ce site.

**49.** Procédé selon la revendication 46, dans lequel on insère le promoteur régulable dans le plasmide en amont du(des) gène(s) de contrôle de réplication natif(s).

**50.** Procédé selon la revendication 49, dans lequel on insère le promoteur régulable au site BglII en amont des gènes copB et copA.

**51.** Procédé selon la revendication 46, dans lequel, en utilisant le plasmide avec le gène régulateur de réplication natif partiellement délété comme produit de départ, on réinsère le gène régulateur de réplication natif en son site d'origine pour construire un plasmide dans lequel tous les gènes de contrôle de réplication natifs sont présents.

**52.** Procédé selon la revendication 51, dans lequel, en utilisant le plasmide avec le gène copB partiellement délété comme produit de départ, on réinsère le gène copB au site BglII pour construire un plasmide dans lequel les deux gènes copB et copA sont présents.

**53.** Procédé selon la revendication 46 dans lequel, en utilisant le plasmide avec le gène régulateur de réplication natif partiellement délété comme produit de départ, on insère un gène régulateur de réplication natif dans une région du plasmide où il n'est pas naturellement situé.

**54.** Procédé selon la revendication 53 dans lequel, en utilisant le plasmide avec le gène copB partiellement délété comme produit de départ, on insère le gène copB au site EcoRI à l'extérieur de la région de réplication du plasmide.

**55.** Procédé de construction d'un plasmide selon la revendication 32 dans lequel, outre l'insertion du promoteur régulable, on insère ledit second promoteur en un site de restriction approprié permettant l'insertion d'un gène de structure dont l'expression est régulable à partir dudit second promoteur.

**56.** Procédé de production d'un produit de gène d'ADN de plasmide dans lequel on cultive des microorganismes portant un plasmide selon les revendications 1 - 41, comprenant au moins une période de culture dans des conditions assurant une transcription accrue à partir dudit promoteur régulable, aboutissant à un nombre de copies de plasmide sensiblement accru et on récolte, à partir de la culture microbienne un produit de gène du plasmide.

**57.** Procédé selon la revendication 56, dans lequel on cultive le microorganisme hôte, auquel on a transformé un plasmide selon les revendications 1 - 41, dans des conditions aboutissant à un nombre faible et constant de copies de plasmide pendant les étapes d'ensemencement et de multiplication, jusqu'à ce qu'on ait obtenu le nombre désiré de cellules, puis on cultive le microorganisme hôte dans des conditions aboutissant à un nombre de copies de plasmide sensiblement accru.

**58.** Procédé selon la revendication 56 ou 57 dans lequel, après une période de culture dans des conditions dans lesquelles aucune transcription à partir du promoteur n'influence de manière significative le nombre de copies de plasmide, on ajoute une substance induisant une activité de promoteur au milieu de culture en une quantité aboutissant à un nombre de copies de plasmide sensiblement accru.

**59.** Procédé selon la revendication 58, dans lequel, après avoir maintenu des conditions aboutissant à un nombre sensiblement accru ou non contrôlé de copies de plasmide pendant une durée suffisante pour obtenir une amplification substantielle du plasmide, mais suffisamment brève pour éviter toute détérioration substantielle de la culture microbienne, on retire du milieu de culture la substance induisant l'activité de promoteur, aboutissant à une lente diminution du nombre de copies de plasmide jusqu'au niveau de pré-induction.

**60.** Procédé selon la revendication 56 ou 57 dans lequel, après une période de culture en présence d'une substance inhibant sensiblement l'activité de promoteur, la concentration de la substance est réduite dans une mesure aboutissant à l'activation du promoteur et à un nombre de copies de plasmide sensiblement accru.

**61.** Procédé selon la revendication 60 dans lequel, après maintien des conditions aboutissant à un nombre de copies sensiblement accru ou non contrôlé pendant une période suffisante pour obtenir une

EP 0 109 150 B1

amplification substantielle du plasmide, mais suffisamment brève pour éviter toute détérioration substantielle de la culture microbienne, on ajoute la substance inhibant l'activité de promoteur en une quantité aboutissant à une lente diminution du nombre de copies de plasmide au niveau de préinduction.

62. Procédé selon la revendication 56 ou 57, dans lequel les conditions dans lesquelles le microorganisme hôte est cultivé comprennent au moins une période de culture à ou au voisinage d'une température à laquelle le nombre de copies de plasmide est sensiblement accru.

63. Procédé selon la revendication 62, dans lequel la culture à la température à laquelle le nombre de copies de plasmide est sensiblement accru est poursuivie jusqu'à ce que la croissance du microorganisme hôte soit inhibée par la formation d'ADN de plasmide ou de produits de gène provenant des plasmides.

64. Procédé selon la revendication 62, dans lequel la culture à l'échelle de la production est effectuée de façon continue à une température approchant la température à laquelle le nombre de copies de plasmide est sensiblement accru de manière à permettre la survie et la croissance continue du microorganisme hôte de façon concomitente avec la formation de quantités accrues d'un produit de gène du plasmide, puis le produit de gène est alors recueilli de façon continue ou intermittente à partir de la culture.

65. Procédé selon la revendication 62, dans lequel, après propagation de la culture microbienne pour atteindre la taille de production, on fait passer la température à une température égale à ou voisine de la température à laquelle le nombre de copies de plasmide est sensiblement accru, et on maintient cette température pendant une durée suffisante pour obtenir une amplification substantielle du plasmide, mais suffisamment brève pour éviter toute détérioration substantielle de la culture microbienne, après quoi on fait encore une fois passer la température à un niveau assurant un nombre faible et constant de copies de plasmide, provoquant une lente diminution du nombre de copies du plasmide jusqu'à ce qu'il atteigne le niveau initial.

66. Procédé selon l'une quelconque des revendications 62 - 65, dans lequel la température à laquelle le nombre de copies de plasmide est sensiblement augmenté est supérieure à la température à laquelle le plasmide a un nombre faible et constant de copies.

67. Procédé selon la revendication 66, dans lequel la température à laquelle le plasmide a un nombre faible et constant de copies est une température d'environ 30°C, et la température à laquelle le plasmide a un nombre de copies sensiblement accru est dans un intervalle d'environ 36 - 42°C.

68. Procédé de préparation d'un plasmide selon l'une quelconque des revendications 1 - 41, dans lequel on insère un fragment d'ADN portant un marqueur utile pour l'identification et/ou la sélection de cellules contenant le plasmide.

69. Procédé selon la revendication 68, dans lequel le marqueur est un gène jouant un rôle de médiateur la résistance aux antibiotiques au microorganisme hôte.

**Patentansprüche**
**Patentansprüche für folgende Vertragsstaaten : BE, CH, DE, FR, GB, IT, LI, LU, NL, SE**

1. Plasmid, das als Klonierungsvektor verwendbar ist und eine Größe im Bereich von etwa 2,5-15,0 x $10^6$ dalton hat, welches trägt:
   (a) einen eingebauten regulierbaren Promotor in einer Position und in einer Ausrichtung, welche die Transkription von diesem Promotor aus in die Replikationsregion ermöglicht;
   (b) wobei diese Replikationsregion ein Gen umfaßt, das für ein Genprodukt codiert, das zur Replikation nötig ist, wobei erhöhte Transkription von dem regulierbaren Promotor zu einer erhöhten Menge des Genprodukts führt, welches zur Replikation nötig ist;
   (c) wobei diese erhöhte Menge eine Replikationsrate des Plasmids ergibt, die höher ist als die Wachstumsrate der Wirtszelle, was zu einer erhöhten Zahl an Kopien des Plasmids führt, welche üblicherweise nach 4-5 Zellverdopplungen um einen Faktor von 25-1000 angestiegen ist.

44

**2.** Plasmid nach Anspruch 1, in dem der Promotor ein aufgrund einer besonderen DNA-Struktur innerhalb der Region des Promotors inhärent regulierbarer Promotor ist.

**3.** Plasmid nach Anspruch 1, in dem der Promotor durch einen regulierenden Faktor kontrollierbar ist.

**4.** Plasmid nach Anspruch 3, in dem der regulierende Faktor ein Faktor ist, der positive Kontrolle ausübt.

**5.** Plasmid nach Anspruch 3, in dem der regulierende Faktor ein Faktor ist, der negative Kontrolle ausübt.

**6.** Plasmid nach Anspruch 1, in dem die erhöhte Transkription von dem regulierbaren Promotor, die zu einer erhöhten Menge des Genprodukts führt, welches für die Replikation nötig ist, durch Derepression des Promotors verursacht wird.

**7.** Plasmid nach einem der vorhergehenden Ansprüche, in dem der Promotor ein chemisch regulierbarer Promotor ist.

**8.** Plasmid nach Anspruch 7, in dem der Promotor ein lac-Promotor, ein trp-Promotor oder ein deo-Promotor ist.

**9.** Plasmid nach einem der Ansprüche 1-6, in dem die Aktivität des Promotors temperaturabhängig ist oder durch einen temperaturempfindlichen regulierenden Faktor kontrolliert wird.

**10.** Plasmid nach Anspruch 9, in dem der Promotor ein λ-Promotor ist, und in dem das Gen für einen temperaturempfindlichen λ cI-Repressor, der die Transkription von diesem Promotor kontrolliert, ebenfalls vorhanden ist.

**11.** Plasmid nach Anspruch 10, in dem der λ -Promotor entweder $\lambda P_R$ oder $\lambda P_L$ ist.

**12.** Plasmid nach Anspruch 11, in dem der λ -Promotor λ $P_R$ ist.

**13.** Plasmid nach einem der vorhergehenden Ansprüche, in dem die Transkription von dem regulierbaren Promotor das Plasmid dazu bringt, eine konstante niedrige Kopienzahl bei niedrigen Temperaturen und eine wesentlich erhöhte Kopienzahl bei höheren Temperaturen aufzuweisen.

**14.** Plasmid nach Anspruch 13, in dem die Transkription von dem regulierbaren Promotor das Plasmid dazu bringt, eine konstante, niedrige Kopienzahl bei einer Temperatur von etwa 30°C und eine wesentlich erhöhte Kopienzahl bei einer Temperatur im Bereich von etwa 36-42°C aufzuweisen.

**15.** Plasmid nach Anspruch 14, das eine erhöhte Zahl an Kopien des Plasmids bei Temperaturen oberhalb von etwa 39°C aufweist.

**16.** Plasmid nach einem der vorhergehenden Ansprüche, in dem ein Teil eines ursprünglichen, die Replikation regulierenden Gens entfernt und durch den regulierbaren Promotor ersetzt worden ist.

**17.** Plasmid nach Anspruch 16, welches, wenn Wirts-Mikroorganismen, die das Plasmid enthalten, unter Bedingungen kultiviert werden, die eine konstante niedrige Zahl an Kopien des Plasmids gewährleisten, eine Kopienzahl in Bereich von 20-40, vorzugsweise 20-30 und insbesondere 20-25 Kopien pro Zelle hat, und welches, wenn die Wirts-Mikroorganismen unter anderen Bedingungen kultiviert werden, die zu einer wesentlich erhöhten Zahl an Kopien des Plasmids führen, eine Kopienzahl im Bereich von mindestens etwa 500-1000 Kopien pro Zelle hat.

**18.** Plasmid nach Anspruch 17, welches eine Zahl an Kopien des Plasmids im Bereich von etwa 20-40, vorzugsweise 20-30 und insbesondere 20-25 Kopien pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

**19.** Plasmid nach den Ansprüchen 16-18, welches ein R1-Typ-Plasmid ist, aus dem das copB-Gen entfernt und durch den regulierbaren Promotor ersetzt worden ist.

20. Plasmid nach einem der Ansprüche 16-19, welches, wenn Wirts-Mikroorganismen, die das Plasmid enthalten, unter Bedingungen kultiviert werden, die eine konstante, niedrige Zahl an Kopien des Plasmids gewährleisten, eine Kopienzahl im Bereich von etwa 3-5 Kopien pro Zelle hat, und welches, wenn die Wirts-Mikroorganismen unter anderen Bedingungen kultiviert werden, die zu einer wesentlich erhöhten Zahl an Kopien des Plasmids führen, eine Kopienzahl im Bereich von mindestens etwa 500-1000 Kopien pro Zelle hat.

21. Plasmid nach Anspruch 20, welches eine Zahl an Kopien des Plasmids im Bereich von etwa 3-5 Kopien pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

22. Plasmid nach einem der Ansprüche 1-15, in dem ein regulierbarer Promotor stromaufwärts von dem (den) ursprünglichen Replikationskontrollgen(en) des Plasmids eingebaut worden ist.

23. Plasmid nach Anspruch 22, welches, wenn Wirts-Mikroorganismen, die das Plasmid enthalten, unter Bedingungen kultiviert werden, die eine konstante, niedrige Zahl an Kopien des Plasmids gewährleisten, eine Kopienzahl im Bereich von etwa 3-5 Kopien pro Zelle hat, und welches, wenn die Wirts-Mikroorganismen unter anderen Bedingungen kultiviert werden, die zu einer wesentlich erhöhten Zahl an Kopien des Plasmids führen, eine Kopienzahl im Bereich von mindestens etwa 500-1000 Kopien pro Zelle hat.

24. Plasmid nach Anspruch 23, welches eine Zahl an Kopien des Plasmids im Bereich von etwa 3-5 Kopien pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

25. Plasmid nach einem der Ansprüche 22-24, welches ein R1-Typ-Plasmid ist, in dem der regulierbare Promotor stromaufwärts von den copB- und copA-Genen eingebaut worden ist.

26. Plasmid nach einem der Ansprüche 22-25, welches, wenn Wirts-Mikroorganismen, die das Plasmid enthalten, unter Bedingungen kultiviert werden, die eine konstante, niedrige Zahl an Kopien des Plasmids gewährleisten, eine Kopienzahl im Bereich von etwa 0,5-1 Kopie pro Zelle hat, und welches, wenn die Wirts-Mikroorganismen unter anderen Bedingungen kultiviert werden, die zu einer wesentlich erhöhten Zahl an Kopien des Plasmids führen, eine Kopienzahl im Bereich von mindestens etwa 500-1000 Kopien pro Zelle hat.

27. Plasmid nach Anspruch 26, welches eine Zahl an Kopien des Plasmids im Bereich von etwa 0,5-1 Kopie pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

28. Plasmid nach einem der Ansprüche 1-15, welches einen regulierbaren Promotor in der Replikationsregion trägt, aus dem ein Teil eines ursprünglichen, die Replikation regulierenden Gens entfernt worden ist, und in dem das ursprüngliche, die Replikation regulierende Gen in eine Region eingebaut worden ist, in der es nicht von Natur aus liegt.

29. Plasmid nach Anspruch 28, welches, wenn Wirts-Mikroorganismen, die das Plasmid enthalten, unter Bedingungen kultiviert werden, die eine konstante, niedrige Zahl an Kopien des Plasmids gewährleisten, eine Kopienzahl im Bereich von etwa 0,5-1 Kopie pro Zelle hat, und welches, wenn die Wirts-Mikroorganismen unter anderen Bedingungen kultiviert werden, die zu einer wesentlich erhöhten Zahl an Kopien des Plasmids führen, eine Kopienzahl im Bereich von mindestens etwa 500-1000 Kopien pro Zelle hat.

30. Plasmid nach Anspruch 29, welches eine Zahl an Kopien des Plasmids im Bereich von etwa 0,5-1 Kopie pro Zelle bei einer Temperatur, wie etwa einer Temperatur von etwa 30°C, und eine erhöhte

46

Zahl an Kopien des Plasmids im Bereich von mindestens etwa 500-1000 Kopien pro Zelle bei einer höheren Temperatur, wie etwa einer Temperatur von etwa 42°C, hat.

31. Plasmid nach einem der Ansprüche 28-30, welches ein R1-Typ-Plasmid ist, in dem das copB-Gen an der EcoR1-Stelle außerhalb der Replikationsregion des Plasmids eingebaut worden ist.

32. Plasmid nach einem der vorhergehenden Ansprüche, in dem, zusätzlich zu dem ersten regulierbaren Promotor, der die Transkription von der Replikationsregion reguliert, ein zweiter Promotor so eingebaut worden ist, daß es möglich ist, ein Strukturgen einzubauen, dessen Expression von dem zweiten Promotor kontrolliert wird.

33. Plasmid nach Anspruch 32, in dem der zweite Promotor, der die Expression des Gens reguliert, kontrollierbar ist.

34. Plasmid nach Anspruch 32 oder 33, in dem der zweite eingebaute Promotor, der die Expression des Strukturgens kontrolliert, der λ $P_L$-Promotor ist.

35. Plasmid nach einem der Ansprüche 32-34, das eine konstante, geringe Zahl an Kopien des Plasmids und sehr wenig oder keine Genexpression bei etwa 30°C und eine wesentlich erhöhte Zahl an Kopien des Plasmids und Genexpression bei einer Temperatur im Bereich von etwa 36-42°C hat.

36. Plasmid nach Anspruch 35, das eine erhöhte Zahl an Kopien des Plasmids und eine sehr starke Genexpression bei Temperaturen oberhalb von 39°C hat.

37. Plasmid nach einem der vorhergehenden Ansprüche, das einen Marker trägt, der zur Identifizierung und/oder Selektion von Zellen, die das Plasmid enthalten, verwendbar ist.

38. Plasmid nach Anspruch 37, in dem der Marker ein Gen ist, das dem Wirts-Mikroorganismus Antibiotika-Resistenz vermittelt.

39. Plasmid nach einem der vorhergehenden Ansprüche, das ein Gen trägt, das eine Genfusion nach dem Einbau eines anderen Gens ermöglicht, durch die es möglich ist, die Expression nachzuweisen.

40. Plasmid nach einem der vorhergehenden Ansprüche, dadurch gekennzeichnet, daß es ein Miniplasmid ist.

41. Plasmid nach einem der vorhergehenden Ansprüche, welches zusätzlich ein Gen oder Gene trägt, die nicht von Natur aus mit dem Plasmid verwandt sind.

42. Mikroorganismus, in den ein Plasmid nach den Ansprüchen 1-41 eingebaut worden ist.

43. Mikroorganismus nach Anspruch 42, welcher ein Bakterium ist.

44. Mikroorganismus nach Anspruch 43, welcher ein gramnegatives und mesophiles Bakterium ist.

45. Mikroorganismus nach Anspruch 44, welcher ein Escherichia coli ist.

46. Verfahren zum Konstruieren eines Plasmids nach den Ansprüchen 1-41, welches das Einbauen eines regulierbaren Promotors in ein Plasmid derart, daß die Transkription von diesem Promotor die Replikation reguliert, und das Identifizieren des so behandelten Plasmids durch Durchsuchen nach denjenigen Plasmiden, die eine wesentlich erhöhte Zahl an Kopien des Plasmids aufweisen, wenn die Transkription von diesem Promotor erhöht wird, umfaßt.

47. Verfahren nach Anspruch 46, welches das Entfernen eines Teils eines ursprünglichen, die Replikation regulierenden Gens und das Einbauen eines DNA-Fragments, das den regulierbaren Promotor einschließt, an der Stelle des teilweise entfernten ursprünglichen, die Replikation regulierenden Gens umfaßt.

**48.** Verfahren nach Anspruch 47, welches das Entfernen des copB-Gens aus dem Plasmid durch Schneiden mit der Restriktionsendonuklease BglII und das Einbauen eines BglII-Fragments, welches den regulierten Promotor enthält, an dieser Stelle umfaßt.

**49.** Verfahren nach Anspruch 46, welches das Einbauen eines regulierbaren Promotors in das Plasmid stromaufwärts von dem (den) ursprünglichen Replikationskontrollgen(en) umfaßt.

**50.** Verfahren nach Anspruch 49, welches das Einbauen des regulierbaren Promotors an der BglII-Stelle stromaufwärts von den copB- und copA-Genen umfaßt.

**51.** Verfahren nach Anspruch 46, welches, unter Verwendung des Plasmids mit dem teilweise entfernten ursprünglichen, die Replikation regulierenden Gen als Ausgangsmaterial, das Wiedereinbauen des ursprünglichen, die Replikation regulierenden Gens an seiner ursprünglichen Stelle zum Konstruieren eines Plasmids, in dem alle ursprünglichen Replikationskontrollgene vorhanden sind, umfaßt.

**52.** Verfahren nach Anspruch 51, welches, unter Verwendung des Plasmids mit dem teilweise entfernten copB-Gen als Ausgangsmaterial, das Wiedereinbauen des copB-Gens an der BglII-Stelle zum Konstruieren eines Plasmids, in dem sowohl die copB- als auch die copA-Gene vorhanden sind, umfaßt.

**53.** Verfahren nach Anspruch 46, welches, unter Verwendung des Plasmids mit dem teilweise entfernten ursprünglichen, die Replikation regulierenden Gen als Ausgangsmaterial, das Einbauen eines ursprünglichen, die Replikation regulierenden Gens in einer Region des Plasmids, wo es nicht von Natur aus liegt, umfaßt.

**54.** Verfahren nach Anspruch 53, welches, unter Verwendung des Plasmids mit dem teilweise entfernten copB-Gen als Ausgangsmaterial, das Einbauen des copB-Gens an der EcoRI-Stelle außerhalb der Replikationsregion des Plasmids, umfaßt.

**55.** Verfahren zum Konstruieren eines Plasmids nach Anspruch 32, das, zusätzlich zu dem Einbau des regulierbaren Promotors, den Einbau des zweiten Promotors an einer geeigneten Restriktionsstelle umfaßt, was den Einbau eines Strukturgens ermöglicht, dessen Expression von dem zweiten Promotor regulierbar ist.

**56.** Verfahren zum Herstellen eines Genprodukts der Plasmid-DNA, welches das Kultivieren von Mikroorganismen, die ein Plasmid nach den Ansprüchen 1-41 tragen, welches mindestens eine Periode der Kultivierung unter Bedingungen umfaßt, die erhöhte Transkription von dem regulierbaren Promotor gewährleisten, was zu einer wesentlich erhöhten Zahl der Kopien des Plasmids führt, und das Ernten eines Genprodukts des Plasmids aus der mikrobiellen Kultur umfaßt.

**57.** Verfahren nach Anspruch 56, welches das Kultivieren des Wirts-Mikroorganismus, in den ein Plasmid nach den Ansprüchen 1-41 transformiert worden ist, unter Bedingungen, die zu einer konstanten, niedrigen Kopienzahl während der Animpf- und Vermehrungsphasen führen, bis die gewünschte Zahl an Zellen erhalten wurde, und dann das Kultivieren des Wirts-Mikroorganismus unter Bedingungen, die zu einer wesentlich erhöhten Zahl an Kopien des Plasmids führen, umfaßt.

**58.** Verfahren nach Anspruch 56 oder 57, in dem nach einer Periode der Kultivierung unter Bedingungen, wo die Transkription von dem Promotor die Zahl der Kopien des Plasmids nicht signifikant beeinflußt, eine Substanz, die die Promotoraktivität induziert, dem Kulturmedium in einer Menge zugegeben wird, die zu einer wesentlich erhöhten Zahl an Kopien des Plasmids führt.

**59.** Verfahren nach Anspruch 58, in dem, nach dem Aufrechterhalten der Bedingungen, die zu einer wesentlich erhöhten oder unkontrollierten Zahl der Kopien des Plasmids führen, über einen Zeitraum, der ausreichend ist, um eine wesentliche Vermehrung des Plasmids zu erhalten, aber ausreichend kurz ist, um jede wesentliche Beeinträchtigung der mikrobiellen Kultur zu vermeiden, die Substanz, welche die Promotoraktivität induziert, aus dem Kulturmedium entfernt wird, was zu einem langsamen Rückgang der Zahl der Kopien des Plasmids auf das vor der Induktion bestehende Niveau führt.

**60.** Verfahren nach Anspruch 56 oder 57, in dem, nach einer Periode der Kultivierung in Gegenwart einer Substanz, die wesentlich die Promotoraktivität inhibiert, die Konzentration dieser Substanz in einem Maß reduziert wird, daß der Promotor aktiviert und die Zahl der Kopien des Plasmids wesentlich erhöht wird.

61. Verfahren nach Anspruch 60, in dem nach dem Aufrechterhalten der Bedingungen, die zu einer wesentlich erhöhten oder unkontrollierten Kopienzahl führen, über einen Zeitraum, der ausreichend ist, um eine wesentliche Vermehrung des Plasmids zu erhalten, aber ausreichend kurz ist, um jede wesentliche Beeinträchtigung der mikrobiellen Kultur zu vermeiden, die Substanz, welche die Promotoraktivität inhibiert, in einer Menge zugegeben wird, die zu einem langsamen Rückgang der Zahl der Kopien des Plasmids auf das vor der Induktion bestehende Niveau führt.

62. Verfahren nach Anspruch 56 oder 57 worin die Bedingungen, unter denen der Wirts-Mikroorganismus kultiviert wird, mindestens eine Periode der Kultivierung bei oder nahe bei einer Temperatur umfassen, bei der die Zahl der Kopien des Plasmids wesentlich erhöht ist.

63. Verfahren nach Anspruch 62, worin die Kultivierung bei der Temperatur, bei der die Zahl der Kopien des Plasmids wesentlich erhöht ist, fortgesetzt wird bis das Wachstum des Wirts-Mikroorganismus durch die Bildung von Plasmid-DNA oder von Genprodukten von den Plasmiden inhibiert wird.

64. Verfahren nach Anspruch 62, worin eine Kultivierung im Produktionsmaßstab kontinuierlich durchgeführt wird bei einer Temperatur, die der Temperatur nahe kommt, bei der die Zahl der Kopien des Plasmids wesentlich erhöht ist, so daß das Überleben und kontiniuierliche Wachstum des Wirts-Mikroorganismus zusammen mit der Bildung erhöhter Mengen eines Genprodukts des Plasmids ermöglicht wird und worin das Genprodukt dann kontinuierlich oder in Abständen aus der Kultur geerntet wird.

65. Verfahren nach Anspruch 62, worin, nachdem die mikrobielle Kultur bis zum Erreichen des Produktionsmaßstabes vermehrt wurde, die Temperatur auf einen Wert von oder nahe bei der Temperatur gebracht wird, bei der die Zahl der Kopien des Plasmids wesentlich erhöht ist, und diese Temperatur für einen Zeitraum beibehalten wird, der ausreichend ist, um eine wesentliche Vermehrung des Plasmids zu erhalten, aber ausreichend kurz ist, um jede wesentliche Beeinträchtigung der mikrobiellen Kultur zu vermeiden, woran anschließend die Temperatur erneut geändert wird auf einen Wert, der eine niedrige konstante Zahl der Kopien des Plasmids gewährleistet, was einen langsamen Rückgang der Zahl der Kopien des Plasmids bis zum Erreichen des Ausgangsniveaus zur Folge hat.

66. Verfahren nach einem der Ansprüche 62-65, worin die Temperatur, bei der die Zahl der Kopien des Plasmids wesentlich erhöht ist, höher ist als die Temperatur, bei der das Plasmid eine konstante, niedrige Kopienzahl hat.

67. Verfahren nach Anspruch 66, worin die Temperatur, bei der das Plasmid eine konstante, niedrige Kopienzahl hat, eine Temperatur von etwa 30°C ist, und die Temperatur, bei der das Plasmid eine wesentlich erhöhte Kopienzahl hat, im Bereich von etwa 36-42°C liegt.

68. Verfahren zum Herstellen eines Plasmids nach einem der Ansprüche 1-41, welches das Einbauen eines DNA-Fragments umfaßt, das einen Marker trägt, der zur Identifizierung und/oder Selektion von Zellen, die das Plasmid enthalten, verwendbar ist.

69. Verfahren nach Anspruch 68, in dem der Marker ein Gen ist, das dem Wirts-Mikroorganismus Antibiotika-Resistenz vermittelt.

**Patentansprüche für folgenden Vertragsstaat : AT**

1. Verfahren zum Herstellen eines Plasmids, das als Klonierungsvektor verwendbar ist und das eine Größe im Bereich von etwa 2,5-15,0 x $10^6$ dalton hat, durch Einbauen in ein Plasmid von:
   (a) einem regulierbaren Promotor in einer Position und in einer Ausrichtung, welche die Transkription von diesem Promotor aus in die Replikationsregion ermöglicht;
   (b) wobei diese Replikationsregion ein Gen umfaßt, das für ein Genprodukt codiert, das zur Replikation nötig ist, wobei erhöhte Transkription von dem regulierbaren Promotor zu einer erhöhten Menge des Genprodukts führt, welches zur Replikation nötig ist;

EP 0 109 150 B1

(c) wobei diese erhöhte Menge eine Replikationsrate des Plasmids ergibt, die höher ist als die Wachstumsrate der Wirtszelle, was zu einer erhöhten Zahl an Kopien des Plasmids führt, welche üblicherweise nach 4-5 Zellverdopplungen um einen Faktor von 25-1000 angestiegen ist.

2. Verfahren nach Anspruch 1, in dem der Promotor ein aufgrund einer besonderen DNA-Struktur innerhalb der Region des Promotors inhärent regulierbarer Promotor ist.

3. Verfahren nach Anspruch 1, in dem der Promotor durch einen regulierenden Faktor kontrollierbar ist.

4. Verfahren nach Anspruch 3, in dem der regulierende Faktor ein Faktor ist, der positive Kontrolle ausübt.

5. Verfahren nach Anspruch 3, in dem der regulierende Faktor ein Faktor ist, der negative Kontrolle ausübt.

6. Verfahren nach Anspruch 1, in dem die erhöhte Transkription von dem regulierbaren Promotor, die zu einer erhöhten Menge des Genprodukts führt, welches für die Replikation nötig ist, durch Derepression des Promotors verursacht wird.

7. Verfahren nach einem der vorhergehenden Ansprüche, in dem der Promotor ein chemisch regulierbarer Promotor ist.

8. Verfahren nach Anspruch 7, in dem der Promotor ein lac-Promotor, ein trp-Promotor oder ein deo-Promotor ist.

9. Verfahren nach einem der Ansprüche 1-6, in dem die Aktivität des Promotors temperaturabhängig ist oder durch einen temperaturempfindlichen regulierenden Faktor kontrolliert wird.

10. Verfahren nach Anspruch 9, in dem der Promotor ein $\lambda$-Promotor ist, und in dem das Gen für einen temperaturempfindlichen $\lambda$ cI-Repressor, der die Transkription von diesem Promotor kontrolliert, ebenfalls vorhanden ist.

11. Verfahren nach Anspruch 10, in dem der $\lambda$-Promotor entweder $\lambda P_R$ oder $\lambda P_L$ ist.

12. Verfahren nach Anspruch 11, in dem der $\lambda$-Promotor $\lambda P_R$ ist.

13. Verfahren nach einem der vorhergehenden Ansprüche, in dem die Transkription von dem regulierbaren Promotor das Plasmid dazu bringt, eine konstante niedrige Kopienzahl bei niedrigen Temperaturen und eine wesentlich erhöhte Kopienzahl bei höheren Temperaturen aufzuweisen.

14. Verfahren nach Anspruch 13, in dem die Transkription von dem regulierbaren Promotor das Plasmid dazu bringt, eine konstante, niedrige Kopienzahl bei einer Temperatur von etwa 30°C und eine wesentlich erhöhte Kopienzahl bei einer Temperatur im Bereich von etwa 36-42°C aufzuweisen.

15. Verfahren nach Anspruch 14, das eine erhöhte Zahl an Kopien des Plasmids bei Temperaturen oberhalb von etwa 39°C aufweist.

16. Verfahren nach einem der vorhergehenden Ansprüche, in dem ein Teil eines ursprünglichen, die Replikation regulierenden Gens entfernt und durch den regulierbaren Promotor ersetzt worden ist.

17. Verfahren nach Anspruch 16, in dem, wenn Wirts-Mikroorganismen, die das Plasmid enthalten, unter Bedingungen kultiviert werden, die eine konstante niedrige Zahl an Kopien des Plasmids gewährleisten, das Plasmid eine Kopienzahl in Bereich von 20-40, vorzugsweise 20-30 und insbesondere 20-25 Kopien pro Zelle hat, und in dem, wenn die Wirts-Mikroorganismen unter anderen Bedingungen kultiviert werden, die zu einer wesentlich erhöhten Zahl an Kopien des Plasmids führen, das Plasmid eine Kopienzahl im Bereich von mindestens etwa 500-1000 Kopien pro Zelle hat.

18. Verfahren nach Anspruch 17, welches eine Zahl an Kopien des Plasmids im Bereich von etwa 20-40, vorzugsweise 20-30 und insbesondere 20-25 Kopien pro Zelle bei einer Temperatur, wie etwa einer

50

Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

19. Verfahren nach den Ansprüchen 16-18, in dem das Plasmid ein R1-Typ-Plasmid ist, aus dem das copB-Gen entfernt und durch den regulierbaren Promotor ersetzt worden ist.

20. Verfahren nach einem der Ansprüche 16-19, in dem, wenn Wirts-Mikroorganismen, die das Plasmid enthalten, unter Bedingungen kultiviert werden, die eine konstante, niedrige Zahl an Kopien des Plasmids gewährleisten, das Plasmid eine Kopienzahl im Bereich von etwa 3-5 Kopien pro Zelle hat, und in dem, wenn die Wirts-Mikroorganismen unter anderen Bedingungen kultiviert werden, die zu einer wesentlich erhöhten Zahl an Kopien des Plasmids führen, das Plasmid eine Kopienzahl im Bereich von mindestens etwa 500-1000 Kopien pro Zelle hat.

21. Verfahren nach Anspruch 20, welches eine Zahl an Kopien des Plasmids im Bereich von etwa 3-5 Kopien pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

22. Verfahren nach einem der Ansprüche 1-15, in dem ein regulierbarer Promotor stromaufwärts von dem (den) ursprünglichen Replikationskontrollgen(en) des Plasmids eingebaut worden ist.

23. Verfahren nach Anspruch 22, in dem, wenn Wirts-Mikroorganismen, die das Plasmid enthalten, unter Bedingungen kultiviert werden, die eine konstante, niedrige Zahl an Kopien des Plasmids gewährleisten, das Plasmid eine Kopienzahl im Bereich von etwa 3-5 Kopien pro Zelle hat, und in dem, wenn die Wirts-Mikroorganismen unter anderen Bedingungen kultiviert werden, die zu einer wesentlich erhöhten Zahl an Kopien des Plasmids führen, das Plasmid eine Kopienzahl im Bereich von mindestens etwa 500-1000 Kopien pro Zelle hat.

24. Verfahren nach Anspruch 23, welches eine Zahl an Kopien des Plasmids im Bereich von etwa 3-5 Kopien pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

25. Verfahren nach einem der Ansprüche 22-24, in dem das Plasmid ein R1-Typ-Plasmid ist, in dem der regulierbare Promotor stromaufwärts von den copB- und copA-Genen eingebaut worden ist.

26. Verfahren nach einem der Ansprüche 22-25, in dem, wenn Wirts-Mikroorganismen, die das Plasmid enthalten, unter Bedingungen kultiviert werden, die eine konstante, niedrige Zahl an Kopien des Plasmids gewährleisten, das Plasmid eine Kopienzahl im Bereich von etwa 0,5-1 Kopie pro Zelle hat, und in dem, wenn die Wirts-Mikroorganismen unter anderen Bedingungen kultiviert werden, die zu einer wesentlich erhöhten Zahl an Kopien des Plasmids führen, das Plasmid eine Kopienzahl im Bereich von mindestens etwa 500-1000 Kopien pro Zelle hat.

27. Verfahren nach Anspruch 26, welches eine Zahl an Kopien des Plasmids im Bereich von etwa 0,5-1 Kopie pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

28. Verfahren nach einem der Ansprüche 1-15, in dem das Plasmid einen regulierbaren Promotor in der Replikationsregion trägt, aus dem ein Teil eines ursprünglichen, die Replikation regulierenden Gens entfernt worden ist, und in dem das ursprüngliche, die Replikation regulierende Gen in eine Region eingebaut worden ist, in der es nicht von Natur aus liegt.

29. Verfahren nach Anspruch 28, in dem, wenn Wirts-Mikroorganismen, die das Plasmid enthalten, unter Bedingungen kultiviert werden, die eine konstante, niedrige Zahl an Kopien des Plasmids gewährleisten, das Plasmid eine Kopienzahl im Bereich von etwa 0,5-1 Kopie pro Zelle hat, und in dem, wenn die Wirts-Mikroorganismen unter anderen Bedingungen kultiviert werden, die zu einer wesentlich

51

erhöhten Zahl an Kopien des Plasmids führen, das Plasmid eine Kopienzahl im Bereich von mindestens etwa 500-1000 Kopien pro Zelle hat.

30. Verfahren nach Anspruch 29, welches eine Zahl an Kopien des Plasmids im Bereich von etwa 0,5-1 Kopie pro Zelle bei einer Temperatur, wie etwa einer Temperatur von etwa 30°C, und eine erhöhte Zahl an Kopien des Plasmids im Bereich von mindestens etwa 500-1000 Kopien pro Zelle bei einer höheren Temperatur, wie etwa einer Temperatur von etwa 42°C, hat.

31. Verfahren nach einem der Ansprüche 28-30, in dem das Plasmid ein R1-Typ-Plasmid ist, in dem das copB-Gen an der EcoR1-Stelle außerhalb der Replikationsregion des Plasmids eingebaut worden ist.

32. Verfahren nach einem der vorhergehenden Ansprüche, in dem, zusätzlich zu dem ersten regulierbaren Promotor, der die Transkription von der Replikationsregion reguliert, ein zweiter Promotor so eingebaut worden ist, daß es möglich ist, ein Strukturgen einzubauen, dessen Expression von dem zweiten Promotor kontrolliert wird.

33. Verfahren nach Anspruch 32, in dem der zweite Promotor, der die Expression des Gens reguliert, kontrollierbar ist.

34. Verfahren nach Anspruch 32 oder 33, in dem der zweite eingebaute Promotor, der die Expression des Strukturgens kontrolliert, der λ $P_L$-Promotor ist.

35. Verfahren nach einem der Ansprüche 32-34, das eine konstante, geringe Zahl an Kopien des Plasmids und sehr wenig oder keine Genexpression bei etwa 30°C und eine wesentlich erhöhte Zahl an Kopien des Plasmids und Genexpression bei einer Temperatur im Bereich von etwa 36-42°C hat.

36. Verfahren nach Anspruch 35, das eine erhöhte Zahl an Kopien des Plasmids und eine sehr starke Genexpression bei Temperaturen oberhalb von 39°C hat.

37. Verfahren nach einem der vorhergehenden Ansprüche, in dem das Plasmid einen Marker trägt, der zur Identifizierung und/oder Selektion von Zellen, die das Plasmid enthalten, verwendbar ist.

38. Verfahren nach Anspruch 37, in dem der Marker ein Gen ist, das dem Wirts-Mikroorganismus Antibiotika-Resistenz vermittelt.

39. Verfahren nach einem der vorhergehenden Ansprüche, in dem das Plasmid ein Gen trägt, das eine Genfusion nach dem Einbau eines anderen Gens ermöglicht, durch die es möglich ist, die Expression nachzuweisen.

40. Verfahren nach einem der vorhergehenden Ansprüche, dadurch gekennzeichnet, daß das Plasmid ein Miniplasmid ist.

41. Verfahren nach einem der vorhergehenden Ansprüche, in dem das Plasmid zusätzlich ein Gen oder Gene trägt, die nicht von Natur aus mit dem Plasmid verwandt sind.

42. Verfahren zum Herstellen eines Mikroorganismus durch Einbauen eines Plasmids nach den Ansprüchen 1-41.

43. Verfahren nach Anspruch 42, in dem der Mikroorganismus ein Bakterium ist.

44. Verfahren nach Anspruch 43, in dem der Mikroorganismus ein gram-negatives und mesophiles Bakterium ist.

45. Verfahren nach Anspruch 44, in dem der Mikroorganismus ein Escherichia coli ist.

46. Verfahren zum Konstruieren eines Plasmids, welches das Einbauen eines regulierbaren Promotors in ein Plasmid derart, daß die Transkription von diesem Promotor die Replikation reguliert, und das Identifizieren des so behandelten Plasmids durch Durchsuchen nach denjenigen Plasmiden, die eine wesentlich erhöhte Zahl an Kopien des Plasmids aufweisen, wenn die Transkription von diesem Promotor erhöht wird, umfaßt.

47. Verfahren nach Anspruch 46, welches das Entfernen eines Teils eines ursprünglichen, die Replikation regulierenden Gens und das Einbauen eines DNA-Fragments, das den regulierbaren Promotor einschließt, an der Stelle des teilweise entfernten ursprünglichen , die Replikation regulierenden Gens umfaßt.

48. Verfahren nach Anspruch 47, welches das Entfernen des copB-Gens aus dem Plasmid durch Schneiden mit der Restriktionsendonuklease BglII und das Einbauen eines BglII-Fragments, welches den regulierten Promotor enthält, an dieser Stelle umfaßt.

49. Verfahren nach Anspruch 46, welches das Einbauen eines regulierbaren Promotors in das Plasmid stromaufwärts von dem (den) ursprünglichen Replikationskontrollgen(en) umfaßt.

50. Verfahren nach Anspruch 49, welches das Einbauen des regulierbaren Promotors an der BglII-Stelle stromaufwärts von den copB- und copA-Genen umfaßt.

51. Verfahren nach Anspruch 46, welches, unter Verwendung des Plasmids mit dem teilweise entfernten ursprünglichen, die Replikation regulierenden Gen als Ausgangsmaterial, das Wiedereinbauen des ursprünglichen, die Replikation regulierenden Gens an seiner ursprünglichen Stelle zum Konstruieren eines Plasmids, in dem alle ursprünglichen Replikationskontrollgene vorhanden sind, umfaßt.

52. Verfahren nach Anspruch 51, welches, unter Verwendung des Plasmids mit dem teilweise entfernten copB-Gen als Ausgangsmaterial, das Wiedereinbauen des copB-Gens an der BglII-Stelle zum Konstruieren eines Plasmids, in dem sowohl die copB- als auch die copA-Gene vorhanden sind, umfaßt.

53. Verfahren nach Anspruch 46, welches, unter Verwendung des Plasmids mit dem teilweise entfernten ursprünglichen, die Replikation regulierenden Gen als Ausgangsmaterial, das Einbauen eines ursprünglichen, die Replikation regulierenden Gens in einer Region des Plasmids, wo es nicht von Natur aus liegt, umfaßt.

54. Verfahren nach Anspruch 53, welches, unter Verwendung des Plasmids mit dem teilweise entfernten copB-Gen als Ausgangsmaterial, das Einbauen des copB-Gens an der EcoRI-Stelle außerhalb der Replikationsregion des Plasmids, umfaßt.

55. Verfahren zum Konstruieren eines Plasmids nach Anspruch 32, das, zusätzlich zu dem Einbau des regulierbaren Promotors, den Einbau des zweiten Promotors an einer geeigneten Restriktionsstelle umfaßt, was den Einbau eines Strukturgens ermöglicht, dessen Expression von dem zweiten Promotor regulierbar ist.

56. Verfahren zum Herstellen eines Genprodukts der Plasmid-DNA, welches das Kultivieren von Mikroorganismen, die ein Plasmid nach den Ansprüchen 1-41 tragen, welches mindestens eine Periode der Kultivierung unter Bedingungen umfaßt, die erhöhte Transkription von dem regulierbaren Promotor gewährleisten, was zu einer wesentlich erhöhten Zahl der Kopien des Plasmids führt, und das Ernten eines Genprodukts des Plasmids aus der mikrobiellen Kultur umfaßt.

57. Verfahren nach Anspruch 56, welches das Kultivieren des Wirts-Mikroorganismus, in den ein Plasmid nach den Ansprüchen 1-41 transformiert worden ist, unter Bedingungen: die zu einer konstanten, niedrigen Kopienzahl während der Animpf- und Vermehrungsphasen führen, bis die gewünschte Zahl an Zellen erhalten wurde, und dann das Kultivieren des Wirts-Mikroorganismus unter Bedingungen, die zu einer wesentlich erhöhten Zahl an Kopien des Plasmids führen, umfaßt.

58. Verfahren nach Anspruch 56 oder 57, in dem nach einer Periode der Kultivierung unter Bedingungen, wo die Transkription von dem Promotor die Zahl der Kopien des Plasmids nicht signifikant beeinflußt, eine Substanz, die die Promotoraktivität induziert, dem Kulturmedium in einer Menge zugegeben wird, die zu einer wesentlich erhöhten Zahl an Kopien des Plasmids führt.

**59.** Verfahren nach Anspruch 58, in dem, nach dem Aufrechterhalten der Bedingungen, die zu einer wesentlich erhöhten oder unkontrollierten Zahl der Kopien des Plasmids führen, über einen Zeitraum, der ausreichend ist, um eine wesentliche Vermehrung des Plasmids zu erhalten, aber ausreichend kurz ist, um jede wesentliche Beeinträchtigung der mikrobiellen Kultur zu vermeiden, die Substanz, welche die Promotoraktivität induziert, aus dem Kulturmedium entfernt wird, was zu einem langsamen Rückgang der Zahl der Kopien des Plasmids auf das vor der Induktion bestehende Niveau führt.

**60.** Verfahren nach Anspruch 56 oder 57, in dem, nach einer Periode der Kultivierung in Gegenwart einer Substanz, die wesentlich die Promotoraktivität inhibiert, die Konzentration dieser Substanz in einem Maß reduziert wird, daß der Promotor aktiviert und die Zahl der Kopien des Plasmids wesentlich erhöht wird.

**61.** Verfahren nach Anspruch 60, in dem nach dem Aufrechterhalten der Bedingungen, die zu einer wesentlich erhöhten oder unkontrollierten Kopienzahl führen, über einen Zeitraum, der ausreichend ist, um eine wesentliche Vermehrung des Plasmids zu erhalten, aber ausreichend kurz ist, um jede wesentliche Beeinträchtigung der mikrobiellen Kultur zu vermeiden, die Substanz, welche die Promotoraktivität inhibiert, in einer Menge zugegeben wird, die zu einem langsamen Rückgang der Zahl der Kopien des Plasmids auf das vor der Induktion bestehende Niveau führt.

**62.** Verfahren nach Anspruch 56 oder 57 worin die Bedingungen, unter denen der Wirts-Mikroorganismus kultiviert wird, mindestens eine Periode der Kultivierung bei oder nahe bei einer Temperatur umfassen, bei der die Zahl der Kopien des Plasmids wesentlich erhöht ist.

**63.** Verfahren nach Anspruch 62, worin die Kultivierung bei der Temperatur, bei der die Zahl der Kopien des Plasmids wesentlich erhöht ist, fortgesetzt wird bis das Wachstum des Wirts-Mikroorganismus durch die Bildung von Plasmid-DNA oder von Genprodukten von den Plasmiden inhibiert wird.

**64.** Verfahren nach Anspruch 62, worin eine Kultivierung im Produktionsmaßstab kontinuierlich durchgeführt wird bei einer Temperatur, die der Temperatur nahe kommt, bei der die Zahl der Kopien des Plasmids wesentlich erhöht ist, so daß das Überleben und kontiniuierliche Wachstum des Wirts-Mikroorganismus zusammen mit der Bildung erhöhter Mengen eines Genprodukts des Plasmids ermöglicht wird und worin das Genprodukt dann kontinuierlich oder in Abständen aus der Kultur geerntet wird.

**65.** Verfahren nach Anspruch 62, worin, nachdem die mikrobielle Kultur bis zum Erreichen des Produktionsmaßstabes vermehrt wurde, die Temperatur auf einen Wert von oder nahe bei der Temperatur gebracht wird, bei der die Zahl der Kopien des Plasmids wesentlich erhöht ist, und diese Temperatur für einen Zeitraum beibehalten wird, der ausreichend ist, um eine wesentliche Vermehrung des Plasmids zu erhalten, aber ausreichend kurz ist, um jede wesentliche Beeinträchtigung der mikrobiellen Kultur zu vermeiden, woran anschließend die Temperatur erneut geändert wird auf einen Wert, der eine niedrige konstante Zahl der Kopien des Plasmids gewährleistet, was einen langsamen Rückgang der Zahl der Kopien des Plasmids bis zum Erreichen des Ausgangsniveaus zur Folge hat.

**66.** Verfahren nach einem der Ansprüche 62-65, worin die Temperatur, bei der die Zahl der Kopien des Plasmids wesentlich erhöht ist, höher ist als die Temperatur, bei der das Plasmid eine konstante, niedrige Kopienzahl hat.

**67.** Verfahren nach Anspruch 66, worin die Temperatur, bei der das Plasmid eine konstante, niedrige Kopienzahl hat, eine Temperatur von etwa 30°C ist, und die Temperatur, bei der das Plasmid eine wesentlich erhöhte Kopienzahl hat, im Bereich von etwa 36-42°C liegt.

**68.** Verfahren zum Herstellen eines Plasmids nach einem der Ansprüche 1-41, welches das Einbauen eines DNA-Fragments umfaßt, das einen Marker trägt, der zur Identifizierung und/oder Selektion von Zellen, die das Plasmid enthalten, verwendbar ist.

**69.** Verfahren nach Anspruch 68, in dem der Marker ein Gen ist, das dem Wirts-Mikroorganismus Antibiotika-Resistenz vermittelt.

Fig. 1

Fig. 2

Fig. 3

pKN1562

Fig. 4

Fig. 5

Fig. 6

apha̅

repA̅    copA̅    ori̅

pOU51

pOU53

tnpA̅    tnpR̅    bla̅ (ApR)

pOU56

cI857    λPR    cI857    λPR    cI857    λPR

Fig. 7

Fig. 8

Fig. 9

pOU57

pOU71

pOU73

Fig. 10

Fig. 11

pOU75

pOU106

Fig. 12

Fig. 13

pOU79

pOU82

Fig. 14

Fig. 15

Fig. 16

Fig. 17

Fig. 18

pOU83

pOU110

pOU130

Fig. 19

pLC 31

Fig. 20

pLC 32'

EP 0 109 150 B1